# EUROPEAN PATENT APPLICATION

(11) **EP 4 714 388 A1**
(43) Date of publication of application: **25.03.2026**
(21) Application number: 25203962.3
(22) Date of filing: 23.09.2025
(51) Int. Cl.: A61B 34/20

(54) **SYSTEMS FOR GUIDING BONE REMOVAL**

(30) Priority: 24.09.2024 US 202463698199 P; 13.06.2025 US 202563823713 P
(71) Applicant: Stryker Corporation, Portage, MI 49002 (US)
(72) Inventor: ZEH, Christopher, Portage, 49002 (US); FOUTS, Brian, Portage, 49002 (US); WOOLFORD, Brady Lewis, Portage, 49002 (US); KAISER, William, Portage, 49002 (US)
(74) Representative: V.O.

(57) **Abstract**

A method for guiding bone removal includes receiving at least one two-dimensional image of bony anatomy captured by a two-dimensional imaging system and at least one endoscopic image of the bony anatomy captured by an endoscopic imager. A three-dimensional model of the bony anatomy is registered with the endoscopic imager in a common reference frame using the two-dimensional image and a tracking system. A position and orientation of the three-dimensional model relative to the endoscopic image is determined based on registration of the three-dimensional model with the endoscopic imager in the common reference frame. The endoscopic image is displayed simultaneously with at least a portion of the three-dimensional model based on the determined position and orientation of the three-dimensional model relative to the endoscopic image.

## Description

### CROSS-REFERENCE TO RELATED APPLICATIONS

This application claims priority to U.S. Provisional Application No. 63/698,199, filed September 24, 2024, and U.S. Provisional Application No. 63/823,713, filed June 13, 2025, the entire contents of which are hereby incorporated by reference.

### FIELD

The present disclosure relates generally to orthopedics, and more particularly to methods and systems for guiding bone removal.

### BACKGROUND

Orthopedics is a medical specialty that focuses on the diagnosis, correction, prevention, and treatment of patients with skeletal conditions, including for example conditions or disorders of the bones, joints, muscles, ligaments, tendons, nerves, and skin, which make up the musculoskeletal system. Joint injuries or conditions such as those of the hip joint, knee joint, shoulder joint, or other joints can occur from overuse or over-stretching or due to other factors, including genetic factors that may cause deviations from "normal" joint morphology.

Joints are susceptible to a number of different pathologies (e.g., conditions or disorders, which may cause deviation from the normal joint morphology). These pathologies can have both congenital and injury-related origins. In some cases, the pathology can be substantial at the outset. In other cases, the pathology may be minor at the outset but, if left untreated, may worsen over time. More particularly, in many cases an existing pathology may be exacerbated, for example, by the dynamic nature of the joint, the substantial forces imposed on the joint, or a combination thereof. The pathology may, either initially or thereafter, significantly interfere with patient comfort and lifestyle and may require surgical treatment.

Orthopedic surgery commonly involves treating joint pathologies using minimally invasive techniques, such as joint arthroscopy, in which an endoscope is inserted into the joint through a small incision. Procedures performed arthroscopically include debridement of bony pathologies in which portions of bone in a joint that deviate from a "normal" or target morphology are removed. During a debridement procedure, the surgeon uses an endoscopic camera to view the debridement area, but because the resulting endoscopic image has a limited field of view and is somewhat distorted, the surgeon cannot view the entire pathology all at once. As a result, it is challenging for the surgeon to determine exactly where and how much bone should be removed, and whether the shape of the remaining bone has the desired geometry.

Three-dimensional models generated from preoperative images can be used to help a surgeon visualize where and how much bone should be removed from a joint. However, determining which portion of a three-dimensional model corresponds to the limited field of view provided by an endoscopic camera during surgery can be challenging. Furthermore, preoperatively generated three-dimensional models show only the preoperative status of a joint, which does not reflect the changes in joint morphology realized over the course of a bone removal procedure. Accordingly, it can be difficult for a surgeon to gauge whether a sufficient amount of bone has been removed at a given point during surgery.

### SUMMARY

According to various aspects, systems and methods can display a three-dimensional model of bony anatomy of a joint based on a determined position and orientation of the bony anatomy relative to at least one endoscopic image (e.g., snapshot image or video frame) of the joint to guide a surgeon during a bone removal procedure. The three-dimensional model can be displayed in a position and orientation that aligns with the position and orientation of the bony anatomy in the endoscopic image. Displaying the three-dimensional model in alignment with the endoscopic image can help a surgeon associate the three-dimensional model with the limited view of the joint that the surgeon sees in the endoscopic image. The three-dimensional model may include a representation of planned bone removal that is displayed in association with the endoscopic image to guide the surgeon in bone removal according to the plan. The planned bone removal can be displayed, for example, as an overlay on the bony anatomy in the endoscopic image.

The position and orientation of the three-dimensional model relative to the endoscopic image can be determined by registering the three-dimensional model with the endoscopic imager used to capture the endoscopic image in a common reference frame. The three-dimensional model can be registered with the endoscopic imager in the common reference frame using a two-dimensional image of the joint (e.g., an intra-operatively generated X-ray image) and a tracking system, such as an electromagnetic tracking system or an optical tracking system. The position and orientation of the three-dimensional model relative to the endoscopic image captured by the endoscopic imager can be determined based on the registration of the three-dimensional model with the endoscopic imager and one or more attributes of the endoscopic imager and/or the endoscopic image. The determined position and orientation of the three-dimensional model with respect to the endoscopic image can be used for generating a visualization of the three-dimensional model that can be displayed to the user in association with the endoscopic image. For example, a portion of the three-dimensional model corresponding to the endoscopic image can be overlaid on the endoscopic image, displayed simultaneously with but separately from the endoscopic image, or used to simulate an endoscopic image. Displaying the three-dimensional model in any of these ways enables a surgeon to better associate the relatively limited field of view shown in the endoscopic image with the overall joint represented by the three-dimensional model.

The displayed three-dimensional model may be updated as a surgical procedure progresses in order to reflect the removal of bone. The three-dimensional model can be updated by tracking a bone removal instrument (e.g., a bur, shaver, drill, etc.) using the tracking system. When the bone removal instrument is determined to be in a location previously known to correspond to bone, the system determines that the bone in that location has been removed, and the three-dimensional model is updated accordingly. The three-dimensional model can be updated while bone is being removed and the updated model can be displayed to the user, such that changes to the bony anatomy can be visualized by a surgeon in real-time. Thus, the surgeon may be guided during bone removal. Hence, during the surgical procedure, the data obtained by the use of the method may immediately allow the surgeon to decide on the course of action to be taken during the surgical procedure. It will be appreciated that such guiding does not constitute a treatment of the human or animal body by surgery.

An exemplary method for guiding bone removal includes: receiving at least one two-dimensional image of bony anatomy captured by a two-dimensional imaging system and at least one endoscopic image of the bony anatomy captured by an endoscopic imager; registering a three-dimensional model of the bony anatomy with the endoscopic imager in a common reference frame using the at least one two-dimensional image and a tracking system; determining a position and orientation of the three-dimensional model relative to the at least one endoscopic image based on the registration of the three-dimensional model with the endoscopic imager in the common reference frame; and displaying at least a portion of the three-dimensional model based on the determined position and orientation of the three-dimensional model relative to the at least one endoscopic image. The endoscopic imager may have been inserted into a subject prior to start of the method for guiding.

A device tracked by the tracking system may be captured in the at least one two-dimensional image of the bony anatomy and registering the three-dimensional model of the bony anatomy with the endoscopic imager in the common reference frame may include analyzing the at least one two-dimensional image to determine a position and orientation of the tracked device with respect to the at least one two-dimensional image. Registering the three-dimensional model of the bony anatomy with the endoscopic imager in the common reference frame may include analyzing the at least one two-dimensional image to register the three-dimensional model relative to the at least one two-dimensional image, and transforming the registration of the three-dimensional model relative to the at least one two-dimensional image to the common reference frame based on the position and orientation of the tracked device in the at least one two-dimensional image and a position and orientation of the tracked device in the common reference frame. The device tracked by the tracking system may have been inserted into a subject prior to start of the method for guiding.

The device tracked by the tracking system may be secured to bone. The device tracked by the tracking system may have been secured to bone prior to start of the method for guiding. The device tracked by the tracking system may include a surgical instrument. The at least one two-dimensional image may capture a surgical instrument configured for securing a device tracked by the tracking system to bone and registering the three-dimensional model of the bony anatomy with the endoscopic imager in the common reference frame may include analyzing the at least one two-dimensional image to determine a position and orientation of the surgical instrument. The surgical instrument may have been inserted into a subject prior to start of the method for guiding. The tracking system may include an electromagnetic (EM) tracking system. A device secured to the bony anatomy may include an EM tracker. The method may further include tracking movement of the bony anatomy via the EM tracker and displaying the at least a portion of the three-dimensional model in an updated position and/or orientation based on the movement of the bony anatomy.

Registering the three-dimensional model of the bony anatomy with the endoscopic imager in the common reference frame may include: analyzing the at least one two-dimensional image to register the three-dimensional model relative to the at least one two-dimensional image; and transforming the registration of the three-dimensional model relative to at least one two-dimensional image to the common reference frame based on a position and orientation of the two-dimensional imaging system in the common reference frame. The tracking system may be an optical tracking system or an EM tracking system. A surgical instrument may be captured in the at least one two-dimensional image of the bony anatomy and registering the three-dimensional model of the bony anatomy with the endoscopic imager in the common reference frame may include analyzing the at least one two-dimensional image to determine a position and orientation of the surgical instrument in the at least one two-dimensional image. The surgical instrument may have been inserted into a subject prior to start of the method for guiding.

Determining the position and orientation of the three-dimensional model relative to the at least one endoscopic image may include analyzing the at least one endoscopic image to determine a rotational angle of an endoscope of the endoscopic imager. Determining the position and orientation of the three-dimensional model relative to the at least one endoscopic image may include determining a rotational angle of an endoscope of the endoscopic imager relative to a camera head of the endoscopic imager using the tracking system. Determining the position and orientation of the three-dimensional model relative to the at least one endoscopic image may include analyzing the at least one endoscopic image to determine at least one value associated with a depth of the bony anatomy relative to the endoscopic imager. Determining the position and orientation of the three-dimensional model relative to the at least one endoscopic image may include determining a depth of the bony anatomy relative to the endoscopic imager based on at least one of: (a) a position and orientation of at least a portion of the endoscopic imager captured in the at least one two-dimensional image, and (b) a position and orientation of a surgical instrument tracked by the tracking system.

Displaying the at least a portion of the three-dimensional model may include displaying an overlay of the at least a portion of the three-dimensional model on the at least one endoscopic image. The method may further include identifying a surgical instrument in the at least one endoscopic image, wherein the overlay is at least partially transparent and the surgical instrument in the at least one endoscopic image is at least partially visible through the overlay. The surgical instrument may have been inserted into a subject prior to start of the method for guiding. Displaying the at least a portion of the three-dimensional model may include displaying the at least a portion of the three-dimensional model separate from the at least one endoscopic image. Displaying the at least a portion of the three-dimensional model may include displaying a simulated endoscopic image that includes the at least a portion of the three-dimensional model. The at least a portion of the three-dimensional model that is displayed may include an indication of planned bone removal.

The method may further include tracking a bone removal instrument using the tracking system and updating the at least a portion of the three-dimensional model based on the tracking of the bone removal instrument. The method may further include tracking the bone removal instrument using a plurality of sensors, wherein a first sensor of the plurality of sensors is positioned at a portion of the bone removal instrument that is located externally of a body of a patient and a second sensor of the plurality of sensors is positioned at a portion of the bone removal instrument that is located within the body of the patient. The bone removal instrument may have been inserted into a subject prior to start of the method for guiding. Updating the at least a portion of the three-dimensional model may include updating the three-dimensional model to reflect removal of bone by the bone removal instrument. The at least a portion of the three-dimensional model may be updated to reflect removal of bone while the bone removal instrument is removing bone. The method may further include displaying the at least a portion of the updated three-dimensional model.

An exemplary system for guiding bone removal includes one or more processors and memory storing one or more programs for execution by the one or more processors, the one or more programs including instructions that, when executed by the one or more processors, cause the system to: receive at least one two-dimensional image of bony anatomy captured by a two-dimensional imaging system and at least one endoscopic image of the bony anatomy captured by an endoscopic imager; register a three-dimensional model of the bony anatomy with the endoscopic imager in a common reference frame using the at least one two-dimensional image and a tracking system; determine a position and orientation of the three-dimensional model relative to the at least one endoscopic image based on the registration of the three-dimensional model with the endoscopic imager in the common reference frame; and display at least a portion of the three-dimensional model based on the determined position and orientation of the three-dimensional model relative to the at least one endoscopic image.

A device tracked by the tracking system may be captured in the at least one two-dimensional image of the bony anatomy and registering the three-dimensional model of the bony anatomy with the endoscopic imager in the common reference frame may include analyzing the at least one two-dimensional image to determine a position and orientation of the tracked device with respect to the at least one two-dimensional image. Registering the three-dimensional model of the bony anatomy with the endoscopic imager in the common reference frame may include analyzing the at least one two-dimensional image to register the three-dimensional model relative to the at least one two-dimensional image, and transforming the registration of the three-dimensional model relative to the at least one two-dimensional image to the common reference frame based on the position and orientation of the tracked device in the at least one two-dimensional image and a position and orientation of the tracked device in the common reference frame.

The device tracked by the tracking system may be secured to bone. The device tracked by the tracking system may include a surgical instrument. The at least one two-dimensional image may capture a surgical instrument configured for securing a device tracked by the tracking system to bone and registering the three-dimensional model of the bony anatomy with the endoscopic imager in the common reference frame may include analyzing the at least one two-dimensional image to determine a position and orientation of the surgical instrument. The tracking system may include an electromagnetic (EM) tracking system. A device secured to the bony anatomy may include an EM tracking device. The one or more programs may further include instructions that, when executed by the one or more processors, cause the system to: track movement of the bony anatomy via the EM tracker and display the at least a portion of the three-dimensional model in an updated position and/or orientation based on the movement of the bony anatomy.

Registering the three-dimensional model of the bony anatomy with the endoscopic imager in the common reference frame may include: analyzing the at least one two-dimensional image to register the three-dimensional model relative to the at least one two-dimensional image; and transforming the registration of the three-dimensional model relative to the at least one two-dimensional image to the common reference frame based on a position and orientation of the two-dimensional imaging system in the common reference frame. The tracking system may be an optical tracking system or an EM tracking system. A surgical instrument may be captured in the at least one two-dimensional image of the bony anatomy and registering the three-dimensional model of the bony anatomy with the endoscopic imager in the common reference frame may include analyzing the at least one two-dimensional image to determine a position and orientation of the surgical instrument in the at least one two-dimensional image.

Determining the position and orientation of the three-dimensional model relative to the at least one endoscopic image may include analyzing the at least one endoscopic image to determine a rotational angle of an endoscope of the endoscopic imager. Determining the position and orientation of the three-dimensional model relative to the at least one endoscopic image may include determining a rotational angle of an endoscope of the endoscopic imager relative to a camera head of the endoscopic imager using the tracking system. Determining the position and orientation of the three-dimensional model relative to the at least one endoscopic image may include analyzing the at least one endoscopic image to determine at least one value associated with a depth of the bony anatomy relative to the endoscopic imager. Determining the position and orientation of the three-dimensional model relative to the at least one endoscopic image may include determining a depth of the bony anatomy relative to the endoscopic imager based on at least one of: (a) a position and orientation of at least a portion of the endoscopic imager captured in the at least one two-dimensional image, and (b) a position and orientation of a surgical instrument tracked by the tracking system.

Displaying the at least a portion of the three-dimensional model may include displaying an overlay of the at least a portion of the three-dimensional model on the at least one endoscopic image. The one or more programs may further include instructions that, when executed by the one or more processors, cause the system to: identify a surgical instrument in the at least one endoscopic image, wherein the overlay is partially transparent and the surgical instrument in the at least one endoscopic image is visible through the overlay. Displaying the at least a portion of the three-dimensional model may include displaying the at least a portion of the three-dimensional model separate from the at least one endoscopic image. Displaying the at least a portion of the three-dimensional model may include displaying a simulated endoscopic image that includes the at least a portion of the three-dimensional model. The at least a portion of the three-dimensional model that is displayed may include an indication of planned bone removal.

The one or more programs may further include instructions that, when executed by the one or more processors, cause the system to: track a bone removal instrument using the tracking system and update the at least a portion of the three-dimensional model based on the tracking of the bone removal instrument.

The one or more programs may further include instructions that, when executed by the one or more processors, cause the system to: track the bone removal instrument using a plurality of sensors, wherein a first sensor of the plurality of sensors is positioned at a portion of the bone removal instrument that is located externally of a body of a patient and a second sensor of the plurality of sensors is positioned at a portion of the bone removal instrument that is located within the body of the patient. Updating the at least a portion of the three-dimensional model may include updating the three-dimensional model to reflect removal of bone by the bone removal instrument. The at least a portion of the three-dimensional model may be updated to reflect removal of bone while the bone removal instrument is removing bone. The one or more programs may further include instructions that, when executed by the one or more processors, cause the system to display the updated three-dimensional model.

An exemplary method for guiding bone removal includes: receiving at least one two-dimensional image of bony anatomy captured by a two-dimensional imaging system and at least one endoscopic image of the bony anatomy captured by an endoscopic imager; registering a three-dimensional model of the bony anatomy with the endoscopic imager in a common reference frame using the at least one two-dimensional image and a tracking system; tracking a bone removal instrument in the common reference frame using the tracking system; updating the three-dimensional model based on the tracking of the bone removal instrument in the common reference frame to reflect bone removal; and displaying the updated three-dimensional model. The endoscopic imager and bone removal instrument may have been inserted into a subject prior to start of the method for guiding.

The updated three-dimensional model may be displayed while the bone removal instrument is removing bone.

An exemplary system for guiding bone removal includes one or more processors and memory storing one or more programs for execution by the one or more processors, the one or more programs including instructions that, when executed by the one or more processors, cause the system to: receive at least one two-dimensional image of bony anatomy captured by a two-dimensional imaging system and at least one endoscopic image of the bony anatomy captured by an endoscopic imager; register a three-dimensional model of the bony anatomy with the endoscopic imager in a common reference frame using the at least one two-dimensional image and a tracking system; track a bone removal instrument in the common reference frame using the tracking system; update the three-dimensional model based on the tracking of the bone removal instrument in the common reference frame to reflect bone removal; and display the updated three-dimensional model.

The one or more programs may further include instructions for displaying the updated three-dimensional model while the bone removal instrument is removing bone.

An exemplary method for guiding bone removal includes: receiving at least one two-dimensional image of bony anatomy captured by a two-dimensional imaging system and at least one endoscopic image of the bony anatomy captured by an endoscopic imager; registering a three-dimensional model of the bony anatomy with the endoscopic imager in a common reference frame using the at least one two-dimensional image and a tracking system; determining a position and orientation of the three-dimensional model relative to the at least one endoscopic image based on the registration of the three-dimensional model with the endoscopic imager in the common reference frame; and displaying a simulated endoscopic image based on the determined position and orientation of the three-dimensional model relative to the at least one endoscopic image, the simulated endoscopic image simulating the endoscopic image using the three-dimensional model. The endoscopic imager may have been inserted into a subject prior to start of the method for guiding.

An exemplary system for guiding bone removal includes one or more processors and memory storing one or more programs for execution by the one or more processors, the one or more programs including instructions that, when executed by the one or more processors, cause the system to: receive at least one two-dimensional image of bony anatomy captured by a two-dimensional imaging system and at least one endoscopic image of the bony anatomy captured by an endoscopic imager; register a three-dimensional model of the bony anatomy with the endoscopic imager in a common reference frame using the at least one two-dimensional image and a tracking system; determine a position and orientation of the three-dimensional model relative to the at least one endoscopic image based on the registration of the three-dimensional model with the endoscopic imager in the common reference frame; and display a simulated endoscopic image based on the determined position and orientation of the three-dimensional model relative to the at least one endoscopic image, the simulated endoscopic image simulating the endoscopic image using the three-dimensional model.

An exemplary method for guiding bone removal includes: receiving at least one endoscopic image of bony anatomy captured by an endoscopic imager; determining a position and orientation of the three-dimensional model relative to the at least one endoscopic image; and displaying a simulated endoscopic image based on the determined position and orientation of the three-dimensional model relative to the at least one endoscopic image, the simulated endoscopic image simulating the endoscopic image using the three-dimensional model. The endoscopic imager may have been inserted into a subject prior to start of the method for guiding.

An exemplary system for guiding bone removal includes one or more processors and memory storing one or more programs for execution by the one or more processors, the one or more programs including instructions that, when executed by the one or more processors, cause the system to: receive at least one endoscopic image of bony anatomy captured by an endoscopic imager; determine a position and orientation of the three-dimensional model relative to the at least one endoscopic image; and display a simulated endoscopic image based on the determined position and orientation of the three-dimensional model relative to the at least one endoscopic image, the simulated endoscopic image simulating the endoscopic image using the three-dimensional model.

An exemplary method for guiding bone removal includes: receiving a first two-dimensional image of bony anatomy and a radiopaque object captured from a first perspective and a second two-dimensional image of the bony anatomy and the radiopaque object captured from a second perspective; determining an alignment between the first two-dimensional image and the second two-dimensional image based on predetermined characteristics of the radiopaque object; generating three-dimensional image data by back-projecting the first and second two-dimensional images in three-dimensional space in accordance with the determined alignment; generating a three-dimensional model of the bony anatomy based on the three-dimensional image data; and displaying a visualization based on the three-dimensional model of the bony anatomy.

The three-dimensional model of the bony anatomy may include an indication of planned bone removal. The indication of planned bone removal may be a heat map. The radiopaque object may include plurality of radiopaque features attached to a non-radiopaque object. The plurality of radiopaque features may be pins, beads, bars, or markers. The radiopaque object may be removably attached to a surgical instrument. The surgical instrument may have been inserted into a subject prior to start of the method for guiding.

The method may include tracking the surgical instrument with respect to a tracking system based on a first tracking device associated with the surgical instrument. The first tracking device may be an electromagnetic tracking device. The method may include registering the three-dimensional model of the bony anatomy with respect to the tracking system based on the first tracking device associated with the surgical instrument. The surgical instrument may be a bone removal instrument. The method may include updating the three-dimensional model of the bony anatomy based on tracking of the bone removal instrument to reflect removal of bone. Updating the three-dimensional model of the bony anatomy based on tracking of the bone removal instrument may include updating an indication of planned bone removal.

The method may include tracking an endoscopic imager with respect to the tracking system based on a second tracking device associated with the endoscopic imager. The second tracking device may be an electromagnetic tracking device. Displaying a visualization based on the three-dimensional model of the bony anatomy may include displaying at least a portion of the three-dimensional model of the bony anatomy in association with endoscopic video captured by the endoscopic imager. The at least a portion of the three-dimensional model of the bony anatomy may be displayed in alignment with the endoscopic video.

Displaying at least a portion of the three-dimensional model of the bony anatomy in association with endoscopic video captured by the endoscopic imager may include displaying an overlay of the at least a portion of the three-dimensional model of the bony anatomy on the endoscopic video. The overlay may be at least partially transparent.

Displaying at least a portion of the three-dimensional model of the bony anatomy in association with endoscopic video captured by the endoscopic imager may include displaying the at least a portion of the three-dimensional model of the bony anatomy separately from the endoscopic video. The at least a portion of the three-dimensional model of the bony anatomy may be displayed adjacent to the endoscopic video. A representation of the surgical instrument may be displayed with the at least a portion of the three-dimensional model of the bony anatomy. The representation of the surgical instrument may be displayed based on tracking the surgical instrument.

Displaying at least a portion of the three-dimensional model of the bony anatomy in association with endoscopic video captured by the endoscopic imager may include displaying a simulated endoscopic image that corresponds to the at least a portion of the three-dimensional model of the bony anatomy. The at least a portion of the three-dimensional model of the bony anatomy may include an indication of planned bone removal. The indication of planned bone removal may include a heat map.

The method may include tracking the bony anatomy with respect to the tracking system based on a third tracking device associated with the bony anatomy. The third tracking device may be an electromagnetic tracking device. The radiopaque object may be removably attached to an endoscopic imager.

The method may include tracking the endoscopic imager with respect to a tracking system based on a first tracking device associated with the endoscopic imager. The method may include registering the three-dimensional model of the bony anatomy with respect to the tracking system based on the first tracking device associated with the endoscopic imager. The first tracking device may be an electromagnetic tracking device.

The method may include tracking a bone removal instrument with respect to the tracking system based on a second tracking device associated with the bone removal instrument. The method may include updating the three-dimensional model of the bony anatomy based on the tracking of the bone removal instrument to reflect removal of bone. Updating the three-dimensional model of the bony anatomy based on the tracking of the bone removal instrument may include updating an indication of planned bone removal. The second tracking device may be an electromagnetic tracking device.

Displaying a visualization based on the three-dimensional model of the bony anatomy may include displaying at least a portion of the three-dimensional model of the bony anatomy in association with endoscopic video captured by the endoscopic imager. The at least a portion of the three-dimensional model of the bony anatomy may be displayed in alignment with the endoscopic video.

Displaying at least a portion of the three-dimensional model of the bony anatomy in association with endoscopic video captured by the endoscopic imager may include displaying an overlay of the at least a portion of the three-dimensional model of the bony anatomy on the endoscopic video. The overlay may be at least partially transparent.

Displaying at least a portion of the three-dimensional model of the bony anatomy in association with endoscopic video captured by the endoscopic imager may include displaying the at least a portion of the three-dimensional model of the bony anatomy separately from the endoscopic video. The at least a portion of the three-dimensional model of the bony anatomy may be displayed adjacent to the endoscopic video. A representation of the surgical instrument may be displayed with the at least a portion of the three-dimensional model of the bony anatomy. The representation of the surgical instrument may be displayed based on tracking the surgical instrument.

Displaying at least a portion of the three-dimensional model of the bony anatomy in association with endoscopic video captured by the endoscopic imager may include displaying a simulated endoscopic image that corresponds to the at least a portion of the three-dimensional model of the bony anatomy. The at least a portion of the three-dimensional model of the bony anatomy may include an indication of planned bone removal. The indication of planned bone removal may be a heat map.

The method may include tracking the bony anatomy with respect to the tracking system based on a third tracking device associated with the bony anatomy. The third tracking device may be an electromagnetic tracking device.

Generating a three-dimensional model of the bony anatomy based on the three-dimensional image data may include processing the three-dimensional image data with a machine learning model.

An exemplary system for guiding bone removal includes one or more processors and memory storing one or more programs for execution by the one or more processors, the one or more programs including instructions that, when executed by the one or more processors, cause the system to: receive a first two-dimensional image of bony anatomy and a radiopaque object captured from a first perspective and a second two-dimensional image of the bony anatomy and the radiopaque object captured from a second perspective; determine an alignment between the first two-dimensional image and the second two-dimensional image based on predetermined characteristics of the radiopaque object; generate three-dimensional image data by back-projecting the first and second two-dimensional images in three-dimensional space in accordance with the determined alignment; generate a three-dimensional model of the bony anatomy based on the three-dimensional image data; and display a visualization based on the three-dimensional model of the bony anatomy.

The three-dimensional model of the bony anatomy may include an indication of planned bone removal. The indication of planned bone removal may be a heat map. The radiopaque object may include a plurality of radiopaque features attached to a non-radiopaque object. The plurality of radiopaque features may be pins, beads, bars, or markers. The radiopaque object may be removably attached to a surgical instrument.

The one or more programs may further include instructions that, when executed by the one or more processors, cause the system to track the surgical instrument with respect to a tracking system based on a first tracking device associated with the surgical instrument. The first tracking device may be an electromagnetic tracking device. The one or more programs may further include instructions that, when executed by the one or more processors, cause the system to register the three-dimensional model of the bony anatomy with respect to the tracking system based on the first tracking device associated with the surgical instrument. The surgical instrument may be a bone removal instrument. The one or more programs may further include instructions that, when executed by the one or more processors, cause the system to update the three-dimensional model of the bony anatomy based on tracking of the bone removal instrument to reflect removal of bone. Updating the three-dimensional model of the bony anatomy based on tracking of the bone removal instrument may include updating an indication of planned bone removal.

The one or more programs may further include instructions that, when executed by the one or more processors, cause the system to track an endoscopic imager with respect to the tracking system based on a second tracking device associated with the endoscopic imager. The second tracking device may be an electromagnetic tracking device. Displaying a visualization based on the three-dimensional model of the bony anatomy may include displaying at least a portion of the three-dimensional model of the bony anatomy in association with endoscopic video captured by the endoscopic imager. The at least a portion of the three-dimensional model of the bony anatomy may be displayed in alignment with the endoscopic video.

Displaying at least a portion of the three-dimensional model of the bony anatomy in association with endoscopic video captured by the endoscopic imager may include displaying an overlay of the at least a portion of the three-dimensional model of the bony anatomy on the endoscopic video. The overlay may be at least partially transparent.

Displaying at least a portion of the three-dimensional model of the bony anatomy in association with endoscopic video captured by the endoscopic imager may include displaying the at least a portion of the three-dimensional model of the bony anatomy separately from the endoscopic video. The at least a portion of the three-dimensional model of the bony anatomy may be displayed adjacent to the endoscopic video. A representation of the surgical instrument may be displayed with the at least a portion of the three-dimensional model of the bony anatomy. The representation of the surgical instrument may be displayed based on tracking the surgical instrument.

Displaying at least a portion of the three-dimensional model of the bony anatomy in association with endoscopic video captured by the endoscopic imager may include displaying a simulated endoscopic image that corresponds to the at least a portion of the three-dimensional model of the bony anatomy. The at least a portion of the three-dimensional model of the bony anatomy may include an indication of planned bone removal. The indication of planned bone removal may be a heat map.

The one or more programs may further include instructions that, when executed by the one or more processors, cause the system to track the bony anatomy with respect to the tracking system based on a third tracking device associated with the bony anatomy. The third tracking device may be an electromagnetic tracking device. The radiopaque object may be removably attached to an endoscopic imager.

The one or more programs may further include instructions that, when executed by the one or more processors, cause the system to track the endoscopic imager with respect to a tracking system based on a first tracking device associated with the endoscopic imager. The one or more programs may further include instructions that, when executed by the one or more processors, cause the system to register the three-dimensional model of the bony anatomy with respect to the tracking system based on the first tracking device associated with the endoscopic imager. The first tracking device may be an electromagnetic tracking device.

The one or more programs may further include instructions that, when executed by the one or more processors, cause the system to track a bone removal instrument with respect to the tracking system based on a second tracking device associated with the bone removal instrument. The one or more programs may further include instructions that, when executed by the one or more processors, cause the system to update the three-dimensional model of the bony anatomy based on the tracking of the bone removal instrument to reflect removal of bone. Updating the three-dimensional model of the bony anatomy based on the tracking of the bone removal instrument may include updating an indication of planned bone removal. The second tracking device may be an electromagnetic tracking device.

Displaying a visualization based on the three-dimensional model of the bony anatomy may include displaying at least a portion of the three-dimensional model of the bony anatomy in association with endoscopic video captured by the endoscopic imager. The at least a portion of the three-dimensional model of the bony anatomy may be displayed in alignment with the endoscopic video.

Displaying at least a portion of the three-dimensional model of the bony anatomy in association with endoscopic video captured by the endoscopic imager may include displaying an overlay of the at least a portion of the three-dimensional model of the bony anatomy on the endoscopic video. The overlay may be at least partially transparent.

Displaying at least a portion of the three-dimensional model of the bony anatomy in association with endoscopic video captured by the endoscopic imager may include displaying the at least a portion of the three-dimensional model of the bony anatomy separately from the endoscopic video. The at least a portion of the three-dimensional model of the bony anatomy may be displayed adjacent to the endoscopic video. A representation of the surgical instrument may be displayed with the at least a portion of the three-dimensional model of the bony anatomy. The representation of the surgical instrument may be displayed based on tracking the surgical instrument.

Displaying at least a portion of the three-dimensional model of the bony anatomy in association with endoscopic video captured by the endoscopic imager may include displaying a simulated endoscopic image that corresponds to the at least a portion of the three-dimensional model of the bony anatomy. The at least a portion of the three-dimensional model of the bony anatomy may include an indication of planned bone removal. The indication of planned bone removal may be a heat map.

The one or more programs may further include instructions that, when executed by the one or more processors, cause the system to track the bony anatomy with respect to the tracking system based on a third tracking device associated with the bony anatomy. The third tracking device may be an electromagnetic tracking device.

Generating a three-dimensional model of the bony anatomy based on the three-dimensional image data may include processing the three-dimensional image data with a machine learning model.

It will be appreciated that any of the variations, aspects, features, and options described in view of the systems apply equally to the methods and vice versa. It will also be clear that any one or more of the above variations, aspects, features, and options can be combined.

### BRIEF DESCRIPTION OF THE FIGURES

The patent or application file contains at least one drawing executed in color. Copies of this patent or patent application publication with color drawing(s) will be provided by the Office upon request and payment of the necessary fee.

The invention will now be described, by way of example only, with reference to the accompanying drawings, in which:
FIG. 1 is a schematic view of an exemplary surgical suite;
FIG. 2 illustrates an exemplary method for guiding a surgeon in the removal of bone from a portion of a joint during a surgical procedure;
FIG. 3 illustrates an exemplary three-dimensional model of bony anatomy;
FIG. 4 illustrates an exemplary electromagnetic tracking system;
FIG. 5A illustrates an exemplary electromagnetic bone tracking device and insertion tool used to register a two-dimensional image in a common reference frame;
FIG. 5B illustrates an exemplary surgical tool used to register a two-dimensional image in a common reference frame;
FIGS. 6A and 6B illustrate examples of registering a three-dimensional model of bony anatomy with an endoscopic imager in a common reference frame using an electromagnetic tracking system;
FIG. 7 illustrates an exemplary method for determining an alignment of a three-dimensional model of bony anatomy to a two-dimensional image of the bony anatomy;
FIG. 8 illustrates aspects of an exemplary application of the method of FIG. 7 to a femur;
FIG. 9 illustrates an exemplary optical tracking system;
FIG. 10 illustrates an example of registering a three-dimensional model of bony anatomy with an endoscopic imager in a common reference frame using an optical tracking system;
FIG. 11 illustrates an exemplary endoscopic image of bony anatomy;
FIG. 12A illustrates an exemplary visualization of a three-dimensional model of bony anatomy overlaid on an endoscopic image of the bony anatomy;
FIG. 12B illustrates an exemplary visualization of a three-dimensional model of bony anatomy overlaid on an endoscopic image of the bony anatomy and extending outside of the field of view shown in the endoscopic image;
FIG. 13 illustrates an exemplary side-by-side visualization of an endoscopic image of bony anatomy and a three-dimensional model of the bony anatomy;
FIGS. 14A and 14B illustrate exemplary simulated endoscopic images;
FIG. 15 illustrates an exemplary method for updating a three-dimensional model to reflect bone removal during a bone removal procedure;
FIG. 16 illustrates an exemplary updated three-dimensional model showing bone removal during a bone removal procedure;
FIG. 17 illustrates an exemplary computing system;
FIG. 18 illustrates an exemplary method for guiding bone removal;
FIG. 19 illustrates exemplary two-dimensional images of bony anatomy captured from two different perspectives;
FIG. 20 illustrates an exemplary two-dimensional image of bony anatomy and a radiopaque object;
FIG. 21 illustrates an exemplary surgical instrument with a radiopaque object attached;
FIG. 22 illustrates an exemplary back-projection process for transforming two-dimensional images into three-dimensional imaging data;
FIG. 23 illustrates an exemplary visualization of an endoscopic image of bony anatomy and a three-dimensional model of the bony anatomy; and
FIG. 24 illustrates an exemplary graphical user interface for guiding a surgeon in generating a three-dimensional model of bony anatomy.

### DETAILED DESCRIPTION

Reference will now be made in detail to implementations of various aspects and variations of the invention, examples of which are illustrated in the accompanying drawings. Various devices, systems, and methods are described herein. Although at least two variations of the devices, systems, and methods are described, other variations may include aspects of the devices, systems, and methods described herein combined in any suitable manner having combinations of all or some of the aspects described. Exemplary aspects will now be described more fully hereinafter with reference to the accompanying drawings; however, they may be embodied in different forms and should not be construed as limited to the examples set forth herein. Rather, these examples are provided so that this disclosure will be thorough and complete, and will fully convey exemplary implementations to those skilled in the art.

According to various aspects, systems and methods according to the principles described herein include displaying a three-dimensional model of a joint or a portion thereof based on a determined position and orientation of the three-dimensional model relative to an endoscopic image of the joint. The position and orientation of the three-dimensional model relative to the endoscopic image can be determined based on the registration of the three-dimensional model with the endoscopic imager used to capture the endoscopic image in a common reference frame.

The three-dimensional model can be registered with the endoscopic imager in the common reference frame using a two-dimensional image of the joint. The two-dimensional image can be, for example, an ultrasound image, an infrared image, or an X-ray image captured prior to or during a surgical procedure on a patient. Optionally, other image types may be used to register the three-dimensional model with the endoscopic imager in the common reference frame, such as a three-dimensional image (e.g., a CT scan). The common reference frame may be a virtual world reference frame associated with the three-dimensional model, a reference frame of the tracking system, a camera reference frame, a world reference frame, or any other suitable reference frame.

The three-dimensional model can be a pre-generated three-dimensional model. Alternatively, the three-dimensional model can be generated intraoperatively. For example, a three-dimensional model of bony anatomy can be generated based on two-dimensional images that include the bony anatomy and a radiopaque object. The two-dimensional images can be captured from different imaging perspectives, such that the images show different views of the bony anatomy and radiopaque object. An alignment between the two-dimensional images can be determined based on predetermined characteristics of the radiopaque object (e.g., an arrangement of radiopaque features associated with the radiopaque object or a unique geometry of the radiopaque object), and the two-dimensional images can be back-projected into three-dimensional space based on the determined alignment. A three-dimensional model of the bony anatomy can then be generated based on the three-dimensional image data.

Registering the three-dimensional model with the endoscopic imager in the common reference frame using the two-dimensional image can include tracking a tracking device that is captured in the two-dimensional image. The two-dimensional image can be analyzed to determine the position and orientation of the tracking device, or a device associated with the tracking device, relative to the two-dimensional image based on predetermined knowledge of the configuration of the tracking device or its associated device. The position and orientation of the tracking device (and/or associated device) relative to the two-dimensional image can be used to register the tracking device and/or the three-dimensional model in the common reference frame. For instance, in examples in which the common reference frame is a virtual world reference frame associated with the three-dimensional model, the position and orientation of the tracking device relative to the two-dimensional image (i.e., relative to the tracking device as captured in the two-dimensional image) can be used to register the tracking device in the virtual world reference frame based on an alignment between the three-dimensional model and the two-dimensional image. The two-dimensional image may be analyzed to determine the position and orientation of the three-dimensional model that aligns with the bony anatomy captured in the two-dimensional image. The alignment of the three-dimensional model to the two-dimensional image can be determined by an iterative process that includes setting an orientation of the three-dimensional model, projecting the three-dimensional model onto a two-dimensional plane, and computing a similarity metric between the projected three-dimensional model and the two-dimensional image until an orientation with the highest similarity metric or an orientation that exceeds a threshold similarity metric is determined. The alignment of the three-dimensional model and the two-dimensional image can be combined with the position and orientation of the tracking device relative to the two-dimensional image to register the tracking device in the virtual world reference frame. A relationship between the virtual world reference frame and the tracking system reference frame can be determined based on the position and orientation of the tracking device in the virtual world reference frame and in the tracking system reference frame such that anything tracked by the tracking system can be registered in the virtual world reference frame.

In examples in which the common reference frame is the tracking system reference frame, the position and orientation of the tracking device in the common reference frame can be combined with the position and orientation of the tracking device relative to the two-dimensional image and an alignment between the three-dimensional model and the two-dimensional image to register the three-dimensional model in the common reference frame.

The endoscopic imager used to capture the endoscopic image can be registered in the common reference frame using any suitable registration technique. According to one aspect, a tracking system (e.g., an electromagnetic tracking system or an optical tracking system) may be used to track a tracking device and/or a fiducial marker affixed to the endoscopic imager to determine the relative positioning of the endoscopic imager with respect to the tracking system. The endoscopic imager may then be registered in the common reference frame, and thus registered with the three-dimensional model, based on the relative position and orientation of the endoscopic imager with respect to the tracking system. With both the endoscopic imager and the three-dimensional model registered to each other in the common reference frame, properties of the endoscopic imager and/or attributes of the endoscopic image can be used to determine the position and orientation of the three-dimensional model relative to the endoscopic image.

Alternatively, the three-dimensional model may be registered with the endoscopic imager in the common reference frame by mapping a surface of the bony anatomy using a tracking system and matching the mapped surface of the bony anatomy with a surface of the three-dimensional model. For example, a surgical instrument that is tracked by the tracking system may be dragged across a surface of the bony anatomy to map the surface in the tracking system reference frame and a surface in the three-dimensional model can be matched to the mapped surface to register the three-dimensional model in the tracking system reference frame.

As noted above, the position and orientation of a tracking device relative to the two-dimensional image can be used to register the three-dimensional model with the endoscopic imager in the common reference frame. According to an aspect, at least one tracking device may be affixed to a bone prior to acquiring a two-dimensional image. For example, an electromagnetic tracking device may be rigidly affixed to the bone (e.g., embedded in the bone or otherwise attached to the bone) prior to acquiring the two-dimensional image. Once the electromagnetic tracking device has been affixed to the bone, a two-dimensional image of the bone may be acquired. The position and orientation of the electromagnetic tracking device as captured in the two-dimensional image may be analyzed (e.g., the geometrical characteristics of the electromagnetic tracking device and/or a fiducial marker) to determine the position and orientation of the electromagnetic tracking device relative to the two-dimensional image. Where the tracking system reference frame is the common reference frame, the position and orientation of the electromagnetic tracking device in the common reference frame can be combined with the position and orientation of the electromagnetic tracking device relative to the two-dimensional image and an alignment between the three-dimensional model and the two-dimensional image to register the three-dimensional model in the common reference frame. Alternatively, where the common reference frame is a virtual world reference frame associated with the three-dimensional model, the position and orientation of the tracking device relative to the two-dimensional image (i.e., relative to the tracking device as captured in the two-dimensional image) can be used to register the tracking device in the virtual world reference frame based on an alignment between the three-dimensional model and the two-dimensional image. A relationship between the virtual world reference frame and the tracking system reference frame can be determined based on the position and orientation of the tracking device in the virtual world reference frame and in the tracking system reference frame such that anything tracked by the tracking system can be registered in the virtual world reference frame.

According to aspects where the electromagnetic tracking device is rigidly affixed to the bone, any movement of the bone will be tracked by the tracking system and the registration of the three-dimensional model with the endoscopic imager in the common reference frame can be updated based on the tracked movement. Thus, even if the bone is moved during a surgical procedure (e.g., if the patient's anatomy is repositioned), the position and orientation of the three-dimensional model and the endoscopic imager relative to one another can be updated without repeating registration. This process can ensure accurate registration of the three-dimensional model to the bone and accurate tracking throughout a surgical procedure. For example, in aspects involving the hip, a femur of the patient may be tracked by positioning an electromagnetic tracking device in the greater trochanter, which can ensure that the femur remains registered to the three-dimensional model. According to some aspects, the position of an additional bone other than the bony anatomy shown in the three-dimensional model may also be tracked. Continuing with the example of the hip, the pelvis of a patient may be tracked in addition to the femur by positioning an additional tracking device in the patient's anterior superior iliac spine. Tracking the pelvis may enhance the accuracy of the registration of the three-dimensional model to the bone.

In some examples, an optical tracking system may be used to register a three-dimensional model with an endoscopic imager in a common reference frame. For instance, an optical tracker may be attached to bone (e.g., by placing one or more pins in the bone and positioning reflectors for the optical tracking system on top of the pins), and the process described above with reference to the electromagnetic tracking system may be used with the optical tracking system to register the three-dimensional model with the endoscopic imager in a common reference frame. Alternatively, the optical tracking system may be used to register the three-dimensional model with the endoscopic imager in the common reference frame by tracking a surgical instrument. In some examples, the position and orientation of a surgical instrument or portion thereof captured in a two-dimensional image may be used to register the three-dimensional model with an endoscopic imager in the common reference frame. For example, a surgical instrument captured in a two-dimensional image may include one or more fiducial markers (e.g., light emitters, ArUcos, QR codes, etc.) that are external to the patient's body and tracked by the optical tracking system. A two-dimensional image may be captured while the surgical instrument is touching the bone, and based on the fiducial marker(s) and known information about the surgical instrument (e.g., its geometry and/or known attributes of a radiopaque fiducial), the position and orientation of the surgical instrument (and the bone that the surgical instrument is touching) with respect to the two-dimensional image may be determined. Because the position and orientation of the surgical instrument in the tracking system reference frame is known from tracking the surgical instrument based on the fiducial marker(s) on the portion of the tool external to the patient's body, and the position and orientation of the surgical instrument relative to the three-dimensional model can be determined based on their respective alignments to the two-dimensional image, the three-dimensional model can be registered in the tracking system reference frame (which may be the common reference frame) or the tracking system may be registered to a virtual world reference frame associated with the three-dimensional model. This process may be repeated if the bone is moved during a surgical procedure to ensure an accurate registration of the three-dimensional model to the bone.

As noted above, with both the endoscopic imager and the three-dimensional model registered to each other in the common reference frame, properties of the endoscopic imager and/or attributes of the endoscopic image can be used to determine the position and orientation of the three-dimensional model relative to the endoscopic image. At least a portion of the three-dimensional model can then be displayed based on the determined position and orientation of the three-dimensional model relative to the endoscopic image. For example, the three-dimensional model or portion thereof can be overlaid on the endoscopic image or displayed simultaneously with but separately from (e.g., next to) the endoscopic image. The three-dimensional model can be displayed in a simulated endoscopic image that shows only the portion of the three-dimensional model corresponding to the endoscopic view, rather than the endoscopic image. Optionally, the displayed three-dimensional model or portion thereof can include a representation of planned bone removal in three-dimensional space so that a surgeon can visualize where bone should be removed in the endoscopic view.

The displayed three-dimensional model may be updated as the surgical procedure progresses in order to track the removal of bone. A bone removal instrument (e.g., a bur, shaver, drill, etc.) can be tracked using the tracking system (e.g., an electromagnetic tracking system or an optical tracking system). When a cutting portion of the bone removal instrument is determined to be in a location corresponding to a bone volume (i.e., the instrument extends past the known bone surface into the volume) in the three-dimensional model, the three-dimensional model can be updated by removing that portion of bone. The three-dimensional model can be updated as bone is being removed, such that a surgeon can view the progress of the bone removal surgery in real-time.

As used herein, "bone removal" encompasses any method of resecting bone, including drilling, sawing, burring, and bone removal using an osteotome. As used herein, the term "endoscopic image" encompasses single snapshot images and one or more video frames.

In the following description, it is to be understood that the singular forms "a," "an," and "the" used in the following description are intended to include the plural forms as well, unless the context clearly indicates otherwise. It is also to be understood that the term "and/or" as used herein refers to and encompasses any and all possible combinations of one or more of the associated listed items. It is further to be understood that the terms "includes," "including," "comprises," and/or "comprising," when used herein, specify the presence of stated features, integers, steps, operations, elements, components, and/or units but do not preclude the presence or addition of one or more other features, integers, steps, operations, elements, components, units, and/or groups thereof.

Certain aspects of the present disclosure include process steps and instructions described herein in the form of an algorithm. It should be noted that the process steps and instructions of the present disclosure could be embodied in software, firmware, or hardware and, when embodied in software, could be downloaded to reside on and be operated from different platforms used by a variety of operating systems. Unless specifically stated otherwise as apparent from the following discussion, it is appreciated that, throughout the description, discussions utilizing terms such as "processing," "computing," "calculating," "determining," "displaying," "generating," or the like, refer to the action and processes of a computer system, or similar electronic computing device, that manipulates and transforms data represented as physical (electronic) quantities within the computer system memories or registers or other such information storage, transmission, or display devices.

The present disclosure in some aspects also relates to devices or systems for performing the operations herein. The devices or systems may be specially constructed for the required purposes, may comprise a general-purpose computer selectively activated or reconfigured by a computer program stored in the computer, or may include any combination thereof. Computer instructions for performing the operations herein can be stored in any combination of non-transitory, computer readable storage medium, such as, but not limited to, any type of disk, including floppy disks, USB flash drives, external hard drives, optical disks, CD-ROMs, magnetic-optical disks, read-only memories (ROMs), random access memories (RAMs), EPROMs, EEPROMs, magnetic or optical cards, or any type of media suitable for storing electronic instructions, and each coupled to a computer system bus. One or more instructions for performing the operations herein may be implemented in or executed by one or more Application Specific Integrated Circuits (ASICs), Field Programmable Gate Arrays (FPGAs), Digital Signal Processing units (DSPs), Graphics Processing Units (GPUs), Central Processing Units (CPUs), or any other suitable processing unit. Furthermore, the computers referred to herein may include a single processor or may be architectures employing multiple processor designs for increased computing capability.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present invention as described herein.

Although the following examples often refer to hip joints, hip joint pathologies, and hip joint characteristics and measurements, it is to be understood that the systems, methods, techniques, visualizations, etc., described herein can be used for analyzing and visualizing other joints, including knees, shoulders, elbows, the spine, the ankle, etc.

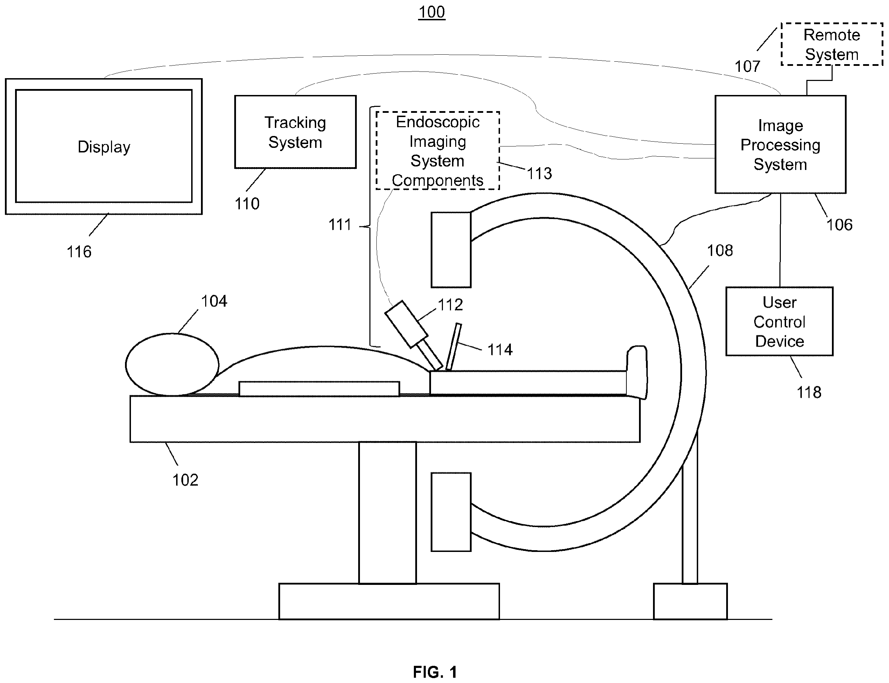

Systems and methods for guiding a surgeon during a surgical procedure on a joint can include displaying a three-dimensional model based on a determined position and orientation of the three-dimensional model relative to an endoscopic image. FIG. 1 illustrates an exemplary surgical suite incorporating a system 100 configured to display a three-dimensional model based on a determined position and orientation of the three-dimensional model relative to an endoscopic image. System 100 may include a surgical table 102 on which a patient 104 may be positioned. A two-dimensional imaging system 108, such as a C-arm X-ray machine, can be used to capture two-dimensional images of bony anatomy of the patient 104. An endoscopic imaging system 111 can be used to image a surgical space within the patient 104. Endoscopic imaging system 111 may include an endoscopic imager 112 (which may include an endoscope removably attached to an endoscopic camera head) and one or more additional endoscopic imaging system components 113, such as a light source for providing light to a surgical cavity via the endoscope of the endoscopic imager 112 and a camera control unit (CCU) that is configured to receive imaging data from the camera head and generate endoscopic images.

The system 100 may include one or more displays 116 on which the surgeon can view information related to a surgical procedure. For example, two-dimensional images of the bony anatomy captured by the two-dimensional imaging system 108 and/or endoscopic images (e.g., single snapshot images and/or video) captured using endoscopic imaging system 111 can be displayed on the one or more displays 116. A three-dimensional model of the bony anatomy may also be displayed on the one or more displays 116. One or more visual indicators may be displayed on one or more displays 116 to guide a user (e.g., a surgeon) in removing bone during the surgical procedure. For example, visual indicators that provide real-time guidance on bone removal may be displayed to the surgeon. The visual indicators may include, for example, arrows (or any other suitable graphical indicators) overlaid on the three-dimensional model that point the surgeon toward a bony protrusion. The visual indicators may be dynamic (e.g., arrows pointing to a bony protrusion may change color depending on the proximity of a surgical tool to the bony protrusion). Alternatively, or additionally, a checklist for a surgical procedure can be displayed on the one or more displays 116. For example, a checklist can include a list of standard leg positions in which a surgeon should image a patient's leg during a given surgical procedure and/or a series of prompts for the surgeon regarding bone to be resected. A display 116 can be any suitable type of display, including but not limited to a boom-mounted display, a display on an imaging cart, a touchscreen display of a tablet or smartphone, and an augmented or virtual reality display.

System 100 may include an image processing system 106 that may be configured to generate visualizations for display on the one or more displays 116 for guiding a surgical procedure on the patient 104. As used herein, an "image processing system" refers to any computing system configured to process image data as well as non-image data, such as tracking data from a tracking system 110. The image processing system 106 may include one or more processors. The image processing system 106 may be communicatively connected to the endoscopic imaging system 111 and to the two-dimensional imaging system 108 to receive and process images from the endoscopic imaging system 111 and two-dimensional imaging system 108. Image processing system 106 may generate visualizations for guiding bone removal that display at least a portion of a three-dimensional model of bony anatomy that is associated with planned bone removal for the bony anatomy. The three-dimensional model may be stored in a memory of the image processing system 106, received directly from another imaging system, or received via a USB flash drive or other transportable storage device inserted into, or otherwise coupled to, image processing system 106. Alternatively, the image processing system 106 may obtain the three-dimensional model from a remote system 107. Remote system 107 may be a cloud storage system, a data repository within the network of the treatment facility in which system 100 is located, a data repository outside of the network of the treatment facility, or any other suitable system for storing three-dimensional model data. The visualizations may include the three-dimensional model displayed in a position and orientation that aligns with the bony anatomy as captured in an endoscopic image generated by endoscopic imaging system 111.

The image processing system 106 may be configured to determine a position and orientation of a three-dimensional model relative to an endoscopic image captured by endoscopic imaging system 111 in order to generate visualizations for guiding bone removal in which the three-dimensional model is displayed in the same position and orientation as the bony anatomy in the endoscopic image. As discussed in detail below, the determination of the position and orientation of the three-dimensional model relative to the endoscopic image may be based on one or more two-dimensional images captured by two-dimensional imaging system 108 and a tracking system 110. Image processing system 106 may generate one or more visualizations based on the determined position and orientation of the three-dimensional model relative to the endoscopic image, such as an overlay image in which the three-dimensional model is overlaid on the endoscopic image, a side-by-side view of the three-dimensional model and the endoscopic image, and/or a simulated endoscopic image based on the three-dimensional model. Alternatively, image processing system 106 may generate one or more visualizations in which at least a portion of the three-dimensional model is displayed without the endoscopic image. Image processing system 106 may provide the visualizations to one or more displays 116.

Tracking system 110 may be used to track the position and orientation of various objects for determining a position and orientation of a three-dimensional model relative to an endoscopic image. Tracking system 110 may be an electromagnetic tracking system or an optical tracking system (e.g., an infrared camera tracking system). Alternatively, tracking system 110 may include one or more inertial measurement units (IMUs) affixed to objects in the surgical environment. Tracking system 110 may be used to track two-dimensional imaging system 108 and/or endoscopic imaging system 111. Tracking system 110 may be used to track a surgical instrument 114 used during a surgical procedure, such as a bone removal tool (e.g., a bur, shaver, drill, or saw) or any other suitable orthopedic surgical tool. Alternatively, or additionally, tracking system 110 may be used to track one or more bones or other anatomical structures of a patient. For example, in aspects involving the hip, tracking system 110 may be used to track movement of the femur (e.g., the greater trochanter) and/or the pelvis during the surgical procedure. Tracking system 110 may provide information about the position and orientation of any of the devices or anatomy that it tracks (e.g., in a tracking system reference frame) to image processing system 106.

A user control device 118 may be communicatively coupled to image processing system 106. User control device 118 may have user input/output functionality, such as a touchscreen, touchpad, trackball, keyboard, mouse, gesture recognition device, voice activation feature, or pupil reading device. A surgeon may use user control device 118 to interact with what is displayed on the display 116. For instance, the surgeon may select one or more visualizations for use in guiding the surgeon during the surgical procedure. User control device 118 may also be configured to allow the surgeon to customize aspects of the visualizations, for example by adjusting the transparency of an overlay image, zooming in or out on a visualization, or displaying a visualization of planned bone removal on an image. User control device 118 may be at least partially located in the sterile field, such that the surgeon may control aspects of display 116 during the surgical procedure. For example, user control device 118 may comprise a touchscreen tablet mounted to surgical table 102 or to a boom-type tablet support and covered with a sterile drape to maintain the surgeon's sterility as he or she operates the touchscreen tablet.

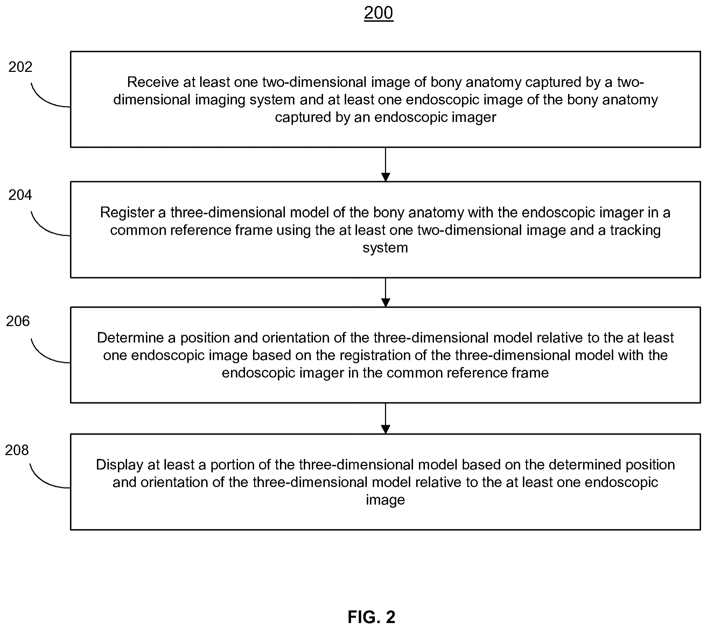

As noted above, image processing system 106 may be configured to generate visualizations that can be displayed via one or more displays 116 to guide a surgeon during a bone removal procedure. FIG. 2 illustrates an exemplary method 200 that may be performed by one or more processors of image processing system 106, or any other suitable computing system, for guiding a surgeon in the removal of bone from a portion of a joint during a surgical procedure. For example, performing method 200, image processing system 106 may generate a visualization for display by display 116 that includes a three-dimensional model of bony anatomy displayed in a position and orientation that aligns with the bony anatomy in an endoscopic image. Method 200 includes determining the position and orientation of the three-dimensional model relative to the endoscopic image and displaying at least a portion of the three-dimensional model based on the determined position and orientation so that a surgeon can better associate the bony anatomy as a whole with the limited view that the surgeon sees in the endoscopic image. In some aspects, the displayed three-dimensional model may include a visualization of planned bone removal to indicate to the surgeon where and how much bone should be removed from various portions of the bony anatomy. This can be advantageous to the surgeon by enabling the surgeon to better associate the planned bone removal with the endoscopic image. Method 200 may be used for any joint of the body, including the hip joint, shoulder joint, knee joint, spinal joints, etc., and/or for any anatomy of the body.

At step 202, at least one two-dimensional image of bony anatomy captured by a two-dimensional imaging system and at least one endoscopic image of the bony anatomy captured by an endoscopic imager are received. The two-dimensional image and the endoscopic image can be received, for example, by image processing system 106 from two-dimensional imaging system 108 and endoscopic imaging system 111 of FIG. 1, respectively. The two-dimensional image(s) may include the portion of the bone that is being or will be surgically treated, as well as surrounding portions of the bone that enable the surgeon to compare what is shown in the two-dimensional image(s) to what the surgeon sees endoscopically. For instance, in examples involving debridement of a bony pathology (e.g., a cam pathology), the two-dimensional image(s) may include the head and neck of the femur. The two-dimensional image(s) may be pre-generated (i.e., generated prior to performance of method 200) and retrieved from a suitable image storage system. Pre-generated two-dimensional images may be captured when the patient is in the same position as they are when method 200 is performed (e.g., immediately prior to the commencement of method 200) so that the pre-generated two-dimensional images accurately reflect the position and orientation of the patient's bony anatomy at step 202.

Optionally, one or more pre-processing operations may be applied to the two-dimensional image(s), such as one or more scaling operations, cropping operations, down-sampling, up-sampling, etc. A dewarping operation may be applied to correct for warping caused by the imaging system. Dewarping of a given two-dimensional image may be performed based on the determined relationship between a known pattern of reference markers attached to the detector of the imaging system and the reference markers visible in the two-dimensional image. For example, the reference markers in a two-dimensional image may be detected, a non-rigid transformation that maps the known positions of the reference markers to the markers visible in the image may be calculated, and the transformation may be applied to the image, resulting in a dewarped image. The reference markers may then be removed from the image.

The at least one endoscopic image of the bony anatomy received at step 202 may be captured by an endoscopic imager, such as endoscopic imager 112 of FIG. 1. The at least one endoscopic image may be captured intra-operatively (e.g., during a bone removal procedure) or pre-operatively. The endoscopic image(s) may be generated prior to performance of method 200 and retrieved from a suitable image storage system. Pre-generated endoscopic images may be captured when the patient is in the same position as they are when the two-dimensional image is captured so that the pre-generated endoscopic image captures the patient's anatomy in the same state it was in when the two-dimensional image was captured.

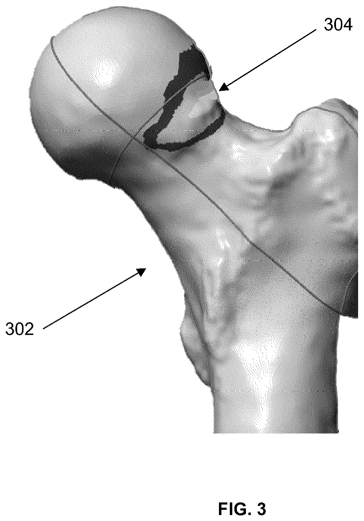

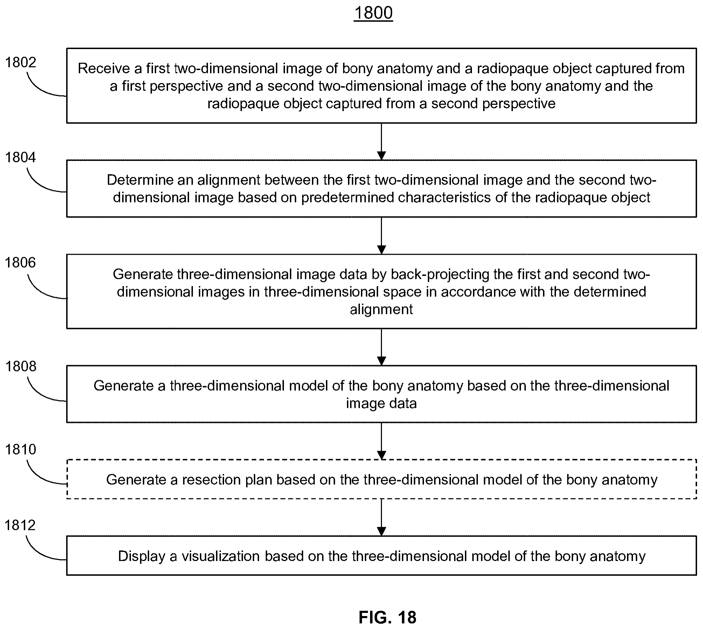

At step 204, a three-dimensional model of the bony anatomy is registered with the endoscopic imager in a common reference frame using the two-dimensional image(s) received at step 202 and a tracking system. The three-dimensional model of the bony anatomy may represent at least a portion of the joint that is targeted for surgical treatment. FIG. 3 illustrates an example of a three-dimensional model 302 of a portion of a femur. The three-dimensional model 302 may be generated based on a pre-operative three-dimensional scan of the patient's joint. For example, for a hip surgery, a patient's hip joint can be imaged, and a model can be built from the imaging that includes various portions of the hip joint, including, for example, the femoral head, the femoral neck, the acetabular cup, the pelvis, femoral condyles, etc. The joint may be imaged using any suitable imaging system for generating three-dimensional image data, including, for example, a CT imaging system, an ultrasound imaging system, a C-arm imaging system, a cone beam CT imaging system, or an MRI imaging system. The three-dimensional model 302 may be pre-generated and stored for use during the surgical session. Alternatively, the three-dimensional model 302 may be generated intraoperatively, such as by an intraoperative CT system or by combining information from multiple two-dimensional images (e.g., multiple images captured by a C-arm imaging system) to construct the three-dimensional model 302, as disclosed herein with reference to FIG. 18. The three-dimensional model 302 may be updated based on images captured during the surgical procedure to reflect the removal of bone during the surgical procedure.

In some examples, the three-dimensional model 302 may be generated before or during the surgical procedure by touching a plurality of points on the bony anatomy with a tracked surgical instrument. The surgical instrument may be tracked using the endoscopic imager, an electromagnetic tracking system, an optical tracking system, one or more inertial measurement units (IMUs) attached to the surgical instrument, or any other suitable tracking system. The tracked surgical instrument may be a pointer tool, a bone removal tool (e.g., a bur or shaver tool), or any other surgical tool. The tip of the tracked surgical instrument may be gently dragged across the surface of the bony anatomy of interest in order to define the contours of the bone surface. Alternatively, the tip of the tracked surgical instrument may not be dragged across the entire bone surface but instead may be touched to a plurality of points on the surface of the bony anatomy or used to grasp various portions of the bony anatomy.

The plurality of points touched (or grasped) by the tracked surgical instrument may be selected in an ad hoc manner. Alternatively, the plurality of points touched with the tracked surgical instrument may be predetermined. In some aspects, the plurality of points on the surface of the bony anatomy may include landmarks of the bony anatomy. For example, in a hip surgery, a three-dimensional model that includes the femoral head may be generated based on a tracked surgical instrument touching a plurality of points on the femoral head. Optionally, landmarks near the femoral head, such as the greater trochanter or femoral neck, may be touched with the tracked surgical instrument to improve accuracy of the three-dimensional model and/or provide a more comprehensive three-dimensional model.

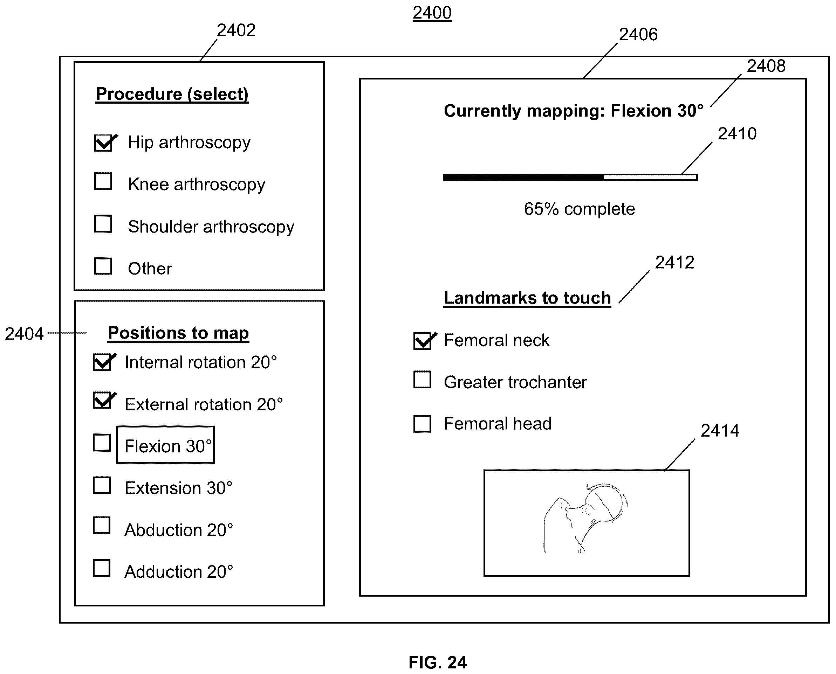

A surgeon may receive guidance regarding which and how many locations on the bony anatomy to touch with the tracked surgical instrument. For example, a graphical user interface may be displayed (e.g., via display(s) 116 of system 100) that guides the surgeon to touch the tracked surgical instrument to particular areas based on the type of bony anatomy being modeled. FIG. 24 illustrates an exemplary graphical user interface 2400 for guiding a surgeon in touching a tracked surgical instrument to bony anatomy in order to generate a three-dimensional model. Graphical user interface 2400 includes a procedure type panel 2402. Procedure type panel 2402 may include a list of surgical procedures (e.g., hip arthroscopy, knee arthroscopy, shoulder arthroscopy, other), and a surgeon may indicate which type of procedure is ongoing. A position panel 2404 may populate with a list of positions in which the bony anatomy should be placed for touching the bony anatomy with the tracked surgical instrument based on the procedure type selected in procedure panel 2402. The surgeon may check boxes corresponding to positions in which the bony anatomy has already been touched with the tracked surgical instrument. The surgeon may also select the current position of the bony anatomy. For example, in FIG. 24, position panel 2404 indicates that the leg is in a 30° flexion position.

Graphical user interface 2400 further includes a status panel 2406 that includes information about how much of the bony anatomy in the current position has been touched with the tracked surgical instrument and how much more needs to be touched in order to collect enough information to generate a three-dimensional model. The amount of information needed to generate a three-dimensional model may be predetermined. For example, the system may be configured to generate a three-dimensional model of the bony anatomy using at least 10, 20, 30, 40, 50, 60, 70, 80, 90, 100, or more unique points on the bony anatomy. As shown in FIG. 24, status panel 2406 includes a position indicator 2408 showing a current position of the bony anatomy and a corresponding progress indicator 2410. Progress indicator 2410 may indicate the current amount of information about the bony anatomy gathered based on touching the tracked surgical instrument to the bony anatomy relative to the amount of information required to generate a three-dimensional model.

Optionally, status panel 2406 may include a list 2412 of landmarks to touch with the tracked surgical instrument. A surgeon may indicate when a landmark has been touched with the tracked surgical instrument by checking a corresponding checkbox. For example, in the example shown in FIG. 24, the femoral neck has been touched with the tracked surgical instrument, but the greater trochanter has yet to be touched. Status panel 2406 may also include a preliminary version of a three-dimensional model 2414 generated based on touching the tracked surgical instrument to the bony anatomy. The preliminary version of the three-dimensional model 2414 may update in real-time as the tracked surgical instrument is touched to the bony anatomy. The preliminary version of the three-dimensional model 2414 may also provide real-time feedback to the surgeon regarding additional landmarks that should be touched in order to build a detailed three-dimensional model. For example, the display of the preliminary version of the three-dimensional model 2414 may include one or more text alerts and/or visual indications on the preliminary version of the three-dimensional model 2414 (e.g., outlining, highlighting, etc.) that identify areas and/or landmarks of the bony anatomy that should be touched in order to improve the three-dimensional model.

The three-dimensional model of the bony anatomy may be determined based on the plurality of locations touched by the tracked surgical instrument. For example, touching the tracked surgical instrument to a plurality of locations may generate a three-dimensional coordinate corresponding to each touched location. The three-dimensional model can be determined by stitching together the plurality of coordinates.

In some examples, information about the tissue surrounding the bony anatomy may be used to generate the three-dimensional model of the bony anatomy. The tissue information may be used to supplement the information about the bony anatomy gathered by touching a tracked surgical instrument to the bony anatomy. Generating the three-dimensional model of the bony anatomy may include identifying at least one type of tissue associated with the bony anatomy, such as a ligament, tendon, cartilage, fibrocartilage, muscle, and/or bone.

The at least one type of tissue may be identified using a database of tissue types. A representation of the bony anatomy (e.g., a preliminary version of a three-dimensional model) may be compared to a database of tissue types in order to determine one or more tissue types present in the representation of the bony anatomy. Identifying types of tissue may help to identify the bony anatomy being mapped. For example, the arrangement of ligaments, tendons, cartilage, fibrocartilage, muscle, and/or bone in the representation of the bony anatomy may indicate that the bony anatomy is a hip. The indication that the bony anatomy is a hip may be used to generate the three-dimensional model of the bony anatomy. For example, the generated three-dimensional model may be compared to a generic three-dimensional model of a hip to ensure that the generated three-dimensional model roughly matches the expected form of a hip.

Alternatively or additionally, the at least one type of tissue may be identified using machine learning techniques. For example, a machine learning model may be configured to receive endoscopic images of anatomy and identify the types of tissue present in the endoscopic image. Optionally, the machine learning model may be configured to receive multi-spectral imaging data that includes images captured within various wavelength ranges across the electromagnetic spectrum. The machine learning model may be configured to analyze the multi-spectral imaging data to identify tissue shown in the images. Multi-spectral imaging data may provide a more comprehensive view of the anatomy due to the images in different spectral bands capturing different details about the anatomy, which may improve the accuracy of the tissue identification process.

Alternatively or additionally, the at least one type of tissue may be identified by comparing a representation of the bony anatomy to a pre-operatively generated model of the bony anatomy that includes tissue information. The pre-operatively generated model may be a subject-specific model that is generated from pre-operative images such as MRI or ultrasound images, which may provide a detailed view of the various tissues associated with the imaged bony anatomy. Alternatively, the pre-operatively generated model of the bony anatomy that includes tissue information may not be subject-specific. For example, the pre-operatively generated model of the bony anatomy may be a statistical shape model of the bony anatomy that includes tissue information. Using tissue information to generate a three-dimensional model may improve the accuracy of the three-dimensional model. For example, tissue information may be used to confirm the accuracy of the locations of various anatomical structures in the three-dimensional model.

Returning to FIG. 3, the three-dimensional model 302 may include a representation of at least one region of the portion of the joint that deviates from a target morphology. The three-dimensional model 302 may be analyzed to determine the deviation of the joint from the target morphology. The representation of the deviation can be used to assist a practitioner in planning for a surgical procedure on region(s) of the joint, such as by including indications of where and how much bone should be removed. For example, a three-dimensional model of a portion of a hip joint of a subject can be generated that includes information identifying a location of a hip joint pathology (e.g., a condition or a disorder), and an amount of bone that may be removed to match a baseline or "target" anatomy. In the illustrated example, the three-dimensional model 302 includes a heat map 304 covering a hip joint pathology. Heat map 304 may be overlaid on three-dimensional model 302 in order to indicate the location and amount of bone that may be removed from the hip joint pathology to match a target morphology. Heat map 304 includes variation in color that indicates a degree of deviation from the target morphology. In other examples, heat map 304 may include variations in contrast, shading, pattern, or any other suitable visual indicator to indicate a degree of deviation from the target morphology. A user, such as the surgeon or a third party, can tailor the settings of heat map 304 for surgical planning purposes, such as by altering one or more parameters that determine the deviations from the target bone morphology, which can increase or decrease the size of the region indicated for bone removal and/or increase or decrease the amount of bone indicated for removal. It should be understood that heat map 304 is merely illustrative and other types of indications of planned bone removal may be displayed. For example, an indication of planned bone removal may include a map (e.g., a contour map) that defines a plurality of regions and that indicates the depth of planned bone removal in those regions. The depth of each region may be indicated by using different types of outlining to identify the boundaries of the region and/or by labeling each region with a numerical depth value.

In some examples, three-dimensional model 302 may include only the bony anatomy of a joint, as shown in FIG. 3. Alternatively, three-dimensional model 302 may include the cartilage layer of the joint. Information about the cartilage layer may be obtained using MRI imaging or any other imaging modality that is configured to visualize cartilage. In some implementations, if information about the cartilage layer of the joint is available, display of the cartilage layer in three-dimensional model 302 may be toggled on and off by a user. Optionally, the indication of planned bone removal (e.g., heat map 304) may include the cartilage layer, and the indication of planned bone removal may indicate the location and depth of cartilage to be removed. The display of the cartilage layer in the indication of planned bone removal may also be toggled on and off by a user.

As used herein, "common reference frame" may refer to any abstract coordinate system in which various tracked objects and images may be registered. In some examples, the common reference frame may be a tracking system reference frame established relative to a tracking system. For example, if an electromagnetic tracking system is used, the electromagnetic field generated by the system may serve as a common reference frame. If an optical tracking system is used, the common reference frame may be a camera-based reference frame, in which a camera of the optical tracking system is assigned the reference position (0,0,0). In some examples, the common reference frame may be a reference frame other than a tracking system reference frame. For instance, the common reference frame may be a virtual world reference frame associated with the three-dimensional model (e.g., a reference frame in which the three-dimensional model is in a fixed position and orientation) or any other suitable reference frame. One skilled in the art would understand that any object registered in one reference frame described herein may be transformed into any other reference frame based on a known relationship between the reference frames (including the relationships determined via the methods described herein). For example, in general, an object registered in a camera-based reference frame may be registered in a world reference frame by multiplying its position in the camera-based reference frame by a transformation matrix representing the position and orientation of the camera-based reference frame relative to the world reference frame. As such, as used herein, a common reference frame is any reference frame in which a three-dimensional model and an endoscopic image are registered relative to one another. The common reference frame can be a virtual world reference frame, a tracking system reference frame, a camera-based reference frame, an endoscopic image reference frame, a world reference frame, or any other suitable reference frame.

The tracking system used in step 204 (e.g., tracking system 110 of FIG. 1) may track one or more objects in a surgical suite in order to help determine a position and orientation of the three-dimensional model with respect to an endoscopic image and/or patient anatomy and to track the removal of bone during a surgical procedure. The tracking system may be an electromagnetic tracking system, an optical tracking system, and/or a tracking system including one or more inertial measurement units (IMUs) for tracking movement of various objects within the surgical suite.

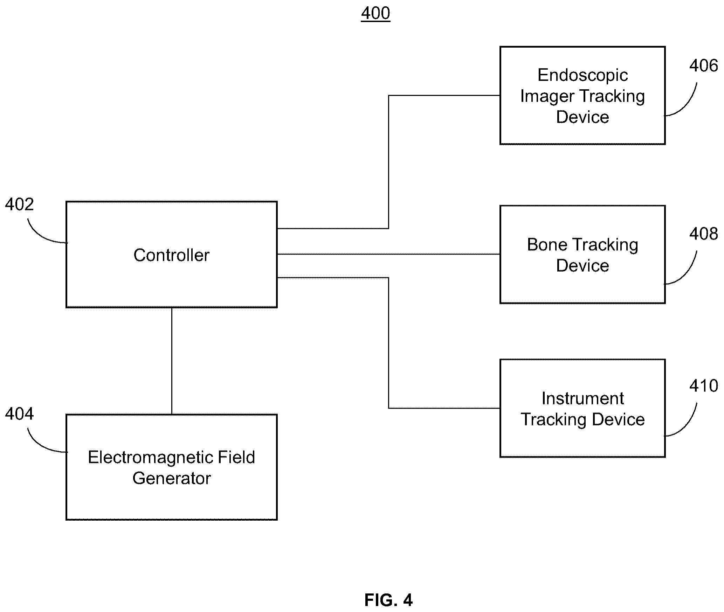

In some examples, the tracking system may be an electromagnetic tracking system. FIG. 4 illustrates an exemplary electromagnetic tracking system 400. Electromagnetic tracking system 400 includes an electromagnetic field generator 404 and a plurality of electromagnetic tracking devices (endoscopic imager tracking device 406, bone tracking device 408, and instrument tracking device 410) communicatively coupled to a controller 402. The electromagnetic tracking devices are also be referred to herein as electromagnetic trackers, EM tracking devices, tracking devices, or EM trackers.

Controller 402 may be configured to control the operation of electromagnetic field generator 404, endoscopic imager tracking device 406, bone tracking device 408, and/or instrument tracking device 410. For example, controller 402 may be used to turn electromagnetic field generator 404 on and off, control the size and/or strength of the generated electromagnetic field, or enable or disable tracking of any of endoscopic imager tracking device 406, bone tracking device 408, or instrument tracking device 410. Electromagnetic field generator 404, endoscopic imager tracking device 406, bone tracking device 408, and/or instrument tracking device 410 may be connected to controller 402 wirelessly or via a wired connection. Controller 402 may be a computing system including one or more processors and may include user input/output functionality, such as a touchscreen, keyboard, mouse, or other interactive feature to enable a user to control aspects of the tracking system. In some aspects, controller 402 may be communicatively coupled to one or more processors that can use information from tracking system 400 to guide a surgeon during a surgical procedure, such as image processing system 106 described above with reference to FIG. 1.

Electromagnetic field generator 404 may be configured to produce an electromagnetic field, the characteristics of which are known. Electromagnetic field generator 404 may be placed within a surgical suite such that the generated electromagnetic field encompasses the area to receive surgical treatment, thus enabling the tracking of objects within the area. For example, if the area to receive surgical treatment includes a hip joint, electromagnetic field generator 404 may be affixed to the underside of the surgical table underneath the patient's hip joint. Electromagnetic field generator 404 may alternatively be incorporated into the surgical table. For example, a tabletop extension of the surgical table can include electromagnetic field generator 404, such that the tabletop extension serves both to support the patient and function as the electromagnetic field generator 404. Alternatively, electromagnetic field generator 404 may be affixed to a nearby piece of equipment, such as two-dimensional imaging system 108 described above with reference to FIG. 1.

Various sensors within the electromagnetic field generated by electromagnetic field generator 404 (e.g., sensors of endoscopic imager tracking device 406, bone tracking device 408, and/or instrument tracking device 410) may sense characteristics of the electromagnetic field and provide that information to controller 402. Based on the electromagnetic field characteristics received from the sensors, controller 402 may determine the position and orientation of the sensors within a tracking system reference frame. In some examples, the tracking system reference frame may be associated with the electromagnetic field generator (e.g., the reference frame may be centered at the electromagnetic field generator).

Tracking system 400 may include a plurality of tracking devices that use one or more electromagnetic field sensors to track various objects within the generated electromagnetic field. For instance, tracking system 400 may include an endoscopic imager tracking device 406, which may be affixed to an endoscopic imager (e.g., to an endoscope, camera head, and/or handle of endoscopic imager 112 of FIG. 1). The endoscopic imager may be tracked in order to determine the position and orientation of an endoscopic image taken using the endoscopic imager in a common reference frame (e.g., the tracking system reference frame).

Tracking system 400 may further include a bone tracking device 408. Bone tracking device 408 may be secured to the bone of a patient such that the movement of the bone itself can be tracked within the common reference frame. For example, if a patient's leg is repositioned during a procedure, such as an arthroscopic hip surgery, the change in position of the leg can be tracked using bone tracking device 408. Bone tracking device 408 may be secured to a portion of the bony anatomy that is easily accessible to a surgeon, thus facilitating surgical insertion and removal. For instance, in an arthroscopic hip surgery, bone tracking device 408 may be secured to the greater trochanter, which is a bony prominence that is easily located and accessible during a surgical procedure.

Tracking system 400 may also include an instrument tracking device 410. Instrument tracking device 410 may be affixed to a surgical instrument, such as a bone removal tool (e.g., a bur, drill, or saw). The surgical instrument may be tracked in order to help a surgeon visualize the location of the instrument within a common reference frame during surgery. An electromagnetic tracking system may be used to track the surgical instrument. For motorized surgical instruments, such as motorized bone removal tools, driving of the motor can be intermittently paused in correspondence with electromagnetic tracking signal generation to limit interference of the motor's electromagnetic field with the electromagnetic tracking. For example, the motor may be pulsed to create intermittent periods of time during which signals from instrument tracking device 410 may be transmitted to controller 402 without interference from the motor. Alternatively, or additionally, controller 402 may perform signal conditioning or analysis on the signals received from instrument tracking device 410 to remove or ignore portions of the signals corresponding to interference from the motor (e.g., portions of the signal having low signal to noise ratio).

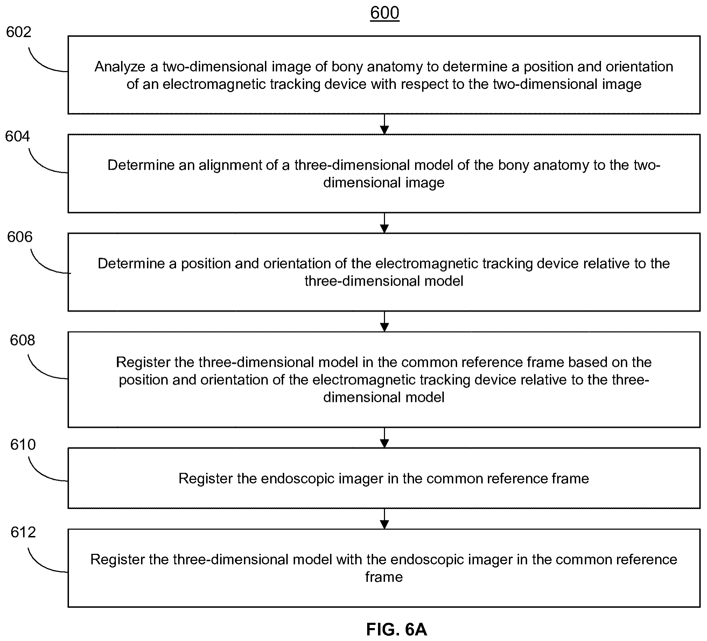

An electromagnetic tracking system, such as system 400, may be used to register a three-dimensional model with an endoscopic imager in a common reference frame in step 204 of method 200. FIG. 6A illustrates an exemplary method 600 for registering a three-dimensional model with an endoscopic imager in a common reference frame using an electromagnetic tracking system, such as electromagnetic tracking system 400, where the common reference frame is a reference frame of the electromagnetic tracking system. Method 600 may be used for step 204 of method 200. The steps of method 600 may be performed by one or more processors, such as one or more processors of image processing system 106 described above with reference to FIG. 1.

Method 600 includes analyzing a two-dimensional image of bony anatomy to determine a position and orientation of an electromagnetic tracking device with respect to the two-dimensional image at step 602. The two-dimensional image analyzed may be the two-dimensional image of the bony anatomy received in step 202 of method 200. The two-dimensional image may be analyzed using one or more machine learning models. For example, image processing system 106 may be configured to use one or more machine learning models to determine the position and orientation of an electromagnetic tracking device within an image. The machine learning model(s) may be used to determine the position and orientation of the electromagnetic tracking device (e.g., bone tracking device 408 of FIG. 4) based on known characteristics of the geometry of the electromagnetic tracking device. According to other aspects, image processing techniques may be used instead of or in addition to machine learning models to determine a position and orientation of an electromagnetic tracking device with respect to the two-dimensional image.

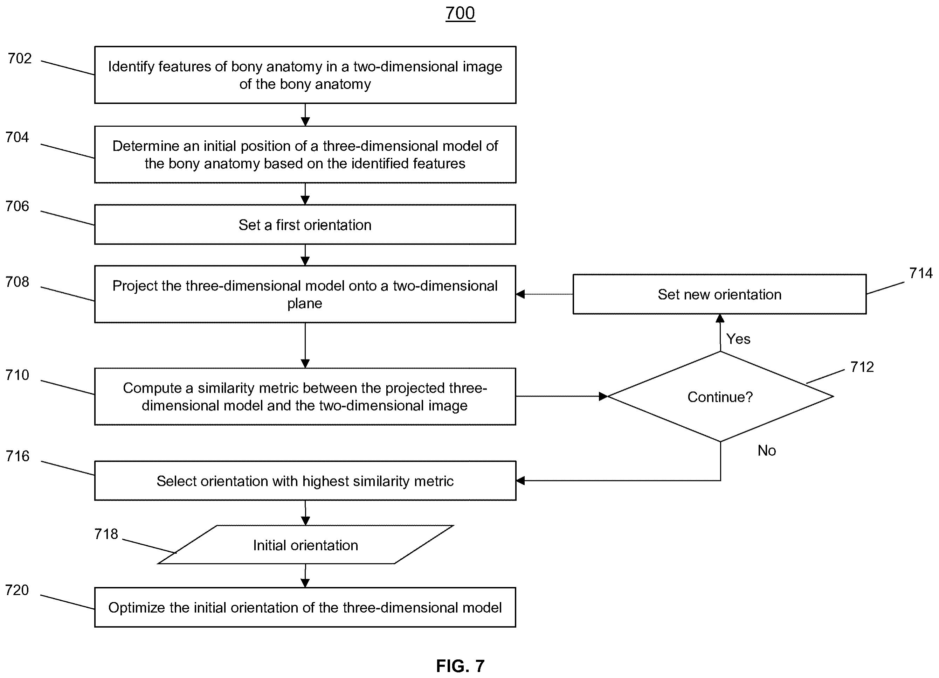

In some examples, determining the position and orientation of the electromagnetic tracking device with respect to the two-dimensional image may include manipulating a three-dimensional model of the electromagnetic tracking device. A three-dimensional model of the electromagnetic tracking device may be retrieved from the memory of the image processing system 106 or a remote system 107. The position and orientation of the model of the electromagnetic tracking device relative to the electromagnetic tracking device captured in the two-dimensional image may be determined by manipulating the model according to the available degrees of freedom (e.g., translation in the x, y, and z directions, rotation about these axes, and scaling relative to the viewpoint of a simulated camera), such that a projection of the model onto a two-dimensional plane that corresponds with the imaging plane aligns with the electromagnetic tracking device in the two-dimensional image. In some examples, the process for determining the position and orientation of the electromagnetic tracking device with respect to the two-dimensional image may share one or more characteristics with the process for aligning a three-dimensional model of bony anatomy to a two-dimensional image of the same bony anatomy, as described in greater detail herein with reference to FIG. 7. In other examples, different processes may be used to determine the position of the electromagnetic tracking device. According to some aspects, the processes may use one or more fiducials and/or other types of distinct features/markings on the electromagnetic tracking device that are captured in the two-dimensional image. For example, the electromagnetic tracking device may include one or more radiopaque fiducials, and the pre-determined geometry and/or relative positions of the one or more radiopaque fiducials may be used to determine the position of the electromagnetic tracking device. In some examples, the one or more fiducials on an electromagnetic tracking device may include fiducials that are visible in multiple different imaging modalities. For instance, an electromagnetic tracking device may include both a radiopaque fiducial that is visible in fluoroscopic images, and an ArUco that is visible in endoscopic images. The fiducials captured using different imaging modalities can be used to relate the reference frames associated with the different imaging modalities. According to another aspect, an electromagnetic tracking device may include only one of a radiopaque fiducial or an ArUco or other fiducial designed to be visible in endoscopic images.

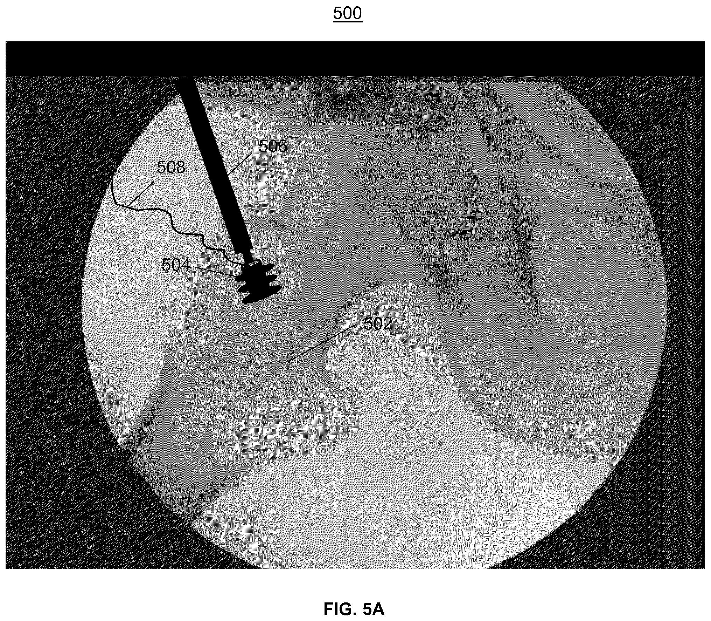

An example of an electromagnetic tracking device for which the position and orientation with respect to the two-dimensional image may be determined is shown in FIG. 5A. An electromagnetic tracking device 504 may be a roughly cylindrical device with one or more protrusions (e.g., grooves or barbs) configured to secure electromagnetic tracking device 504 to bone 502. Electromagnetic tracking device 504 may include a radiopaque material so that the device is visible under fluoroscopic imaging. For example, electromagnetic tracking device 504 may be made from metal (e.g., titanium) or made from plastic and filled with a radiopaque material (e.g., barium sulfate). Electromagnetic tracking device 504 may be configured to track the movement of bone 502. Electromagnetic tracking device 504 may track movement of the bony anatomy using one or more sensors and send the sensor signals to a controller (e.g., controller 402 of FIG. 4) via a wire 508. The controller may cause at least a portion of a three-dimensional model of bone 502 to be displayed to a display (e.g., display 116 of FIG. 1) in an updated position and/or orientation based on the movement of the bony anatomy.

Alternatively, or additionally, the position and orientation of an insertion tool with respect to the two-dimensional image may be determined. An insertion tool 506 may be used to secure electromagnetic tracking device 504 to bone 502. In some examples, electromagnetic tracking device 504 may be too small to achieve accurate results from the alignment process described above (i.e., aligning a three-dimensional model of electromagnetic tracking device 504 to electromagnetic tracking device 504 in the two-dimensional image). Thus, the two-dimensional image may be captured while insertion tool 506 is still inside of the body of the patient so that the position and orientation of insertion tool 506 in the image may be determined. In some examples, the position and orientation of insertion tool 506 may be determined by manipulating a three-dimensional model of insertion tool 506 according to the available degrees of freedom (e.g., translation in the x, y, and z directions, rotation about these axes, and scaling relative to the viewpoint of a simulated camera), such that a projection of the model onto a two-dimensional plane that corresponds with the imaging plane aligns with insertion tool 506 in the two-dimensional image. In other examples, the position and orientation of insertion tool 506 may be determined based on unique optical properties of insertion tool 506. For instance, insertion tool 506 may have a unique geometry or may include one or more fiducial markers (e.g., one or more radiopaque fiducial markers) that may be used to determine the position and orientation of insertion tool 506 with respect to the two-dimensional image.

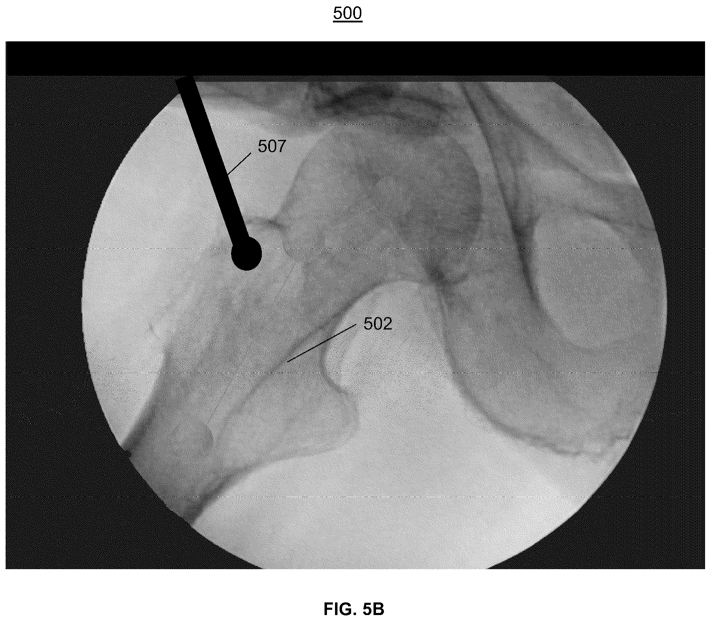

In some examples, the position and orientation of a surgical instrument tracked by the electromagnetic tracking system with respect to the two-dimensional image may be determined instead of the position and orientation of an electromagnetic tracking device and/or insertion tool. For example, as shown in FIG. 5B, a surgical instrument 507 (e.g., a bone removal tool, such as a bur, shaver, drill, saw, etc.) may be touched against bone 502 when two-dimensional image 500 is captured. The position and orientation of surgical instrument 507 may be determined by manipulating a three-dimensional model of surgical instrument 507 according to the available degrees of freedom (e.g., translation in the x, y, and z directions, rotation about these axes, and scaling relative to the viewpoint of a simulated camera), such that a projection of the model onto a two-dimensional plane that corresponds with the imaging plane aligns with surgical instrument 507 in the two-dimensional image. Because surgical instrument 507 is not affixed to bone 502 like electromagnetic tracking device 504, this process may be repeated if the patient's bone moves after the two-dimensional image is captured (e.g., if the patient's leg is repositioned during surgery). Optionally, one or more divots may be formed in the bone 502 (e.g., by burring one or more divots in the bone) to facilitate re-registration of the three-dimensional model to the bone 502. If re-registration is required later in the surgical procedure (e.g., if the patient's leg is repositioned), the surgical instrument 507 may be placed in the same location as it was in for the initial registration based on the one or more divots. This may reduce the number of steps required for re-registration-because the divots are fixed, a user can simply place the surgical instrument in each of the divots to re-register the three-dimensional model to the bone 502 instead of touching the surgical instrument to several points on the surface of the bone or dragging the surgical instrument along multiple lines to establish the contours of the bone.

Returning to FIG. 6A, at step 604, an alignment of a three-dimensional model of the same bony anatomy captured in the two-dimensional image to the two-dimensional image is determined. Determining the alignment of the three-dimensional model to the two-dimensional image generally involves manipulating the three-dimensional model according to the available spatial degrees of freedom until a projection of that model onto a two-dimensional plane aligns with the bony anatomy captured in the two-dimensional image. FIG. 7 illustrates an exemplary method 700 for determining an alignment of a three-dimensional model to a two-dimensional image, which may be used for step 604 of method 600. Method 700 may be performed by one or more processors, such as one or more processors of image processing system 106 described above with reference to FIG. 1.

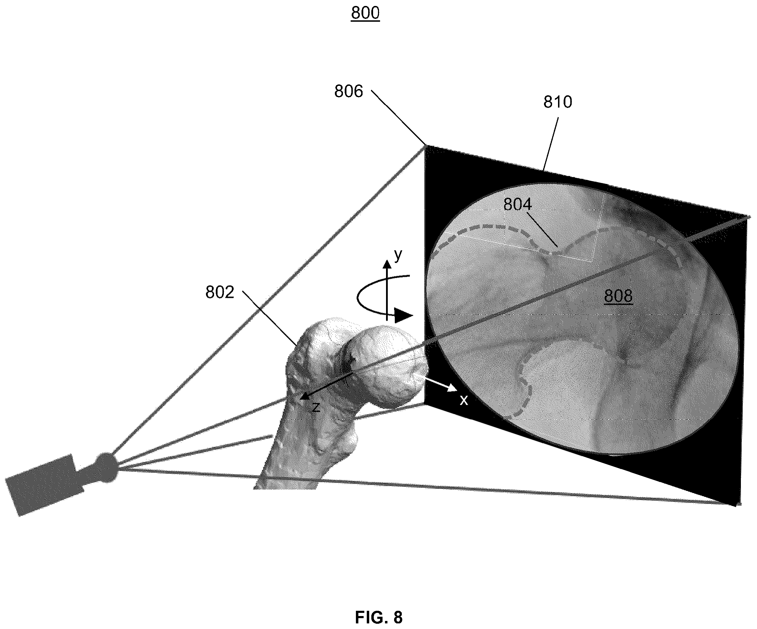

An example of method 700 related to debridement of a lesion (e.g., a cam pathology) on the head of a femur of a hip joint is shown in FIG. 8 to illustrate aspects of method 700. In the illustrated example, a three-dimensional model 802 models the upper portion of a particular patient's femur. A portion of bone may deviate from a target morphology and, as such, be identified for removal (e.g., via debridement). As used herein, "target morphology" may refer to any joint morphology that may be desired for a given subject. Target morphology can be based on the anatomy representative of any reference patient population, such as a normal patient population. For example, baseline data can be a model of a "normal" joint that is derived from studies of a healthy patient population and/or from a model generated based on measurements, computer simulations, calculations, etc.

As discussed further below, method 700 includes steps for determining a position and orientation of three-dimensional model 802 relative to the portion of the femur 808 captured in the two-dimensional image 810, such that a projection 804 of the model onto a two-dimensional plane 806 that corresponds with the imaging plane aligns with femur 808 in two-dimensional image 810. Conceptually speaking, the three-dimensional model 802 is manipulated according to the available degrees of freedom - such as translations in the x, y, and z directions, rotations about these axes, and scaling relative to the viewpoint of a simulated camera - until its projection 804 aligns sufficiently with the femur 808 in a two-dimensional image of the joint. Once this satisfactory alignment is achieved, the alignment may be used to register three-dimensional model 802 to an endoscopic image of the same bony anatomy according to the principles described herein.

Returning to FIG. 7, step 702 includes identifying features of bony anatomy in a two-dimensional image of the bony anatomy. The two-dimensional image analyzed may be the two-dimensional image registered in the common reference frame in step 604 of method 600, which may also be the two-dimensional image received in step 202 of method 200. The features of bony anatomy identified in the two-dimensional image may include, for example, the position of the center of the femoral head in the two-dimensional image, the direction of the midline of the femoral neck, the radius of the femoral head, or any other suitable anatomical landmarks.

At step 704, an initial position of the three-dimensional model may be determined based on the features identified in step 702. The center of the femoral head and the midline of the femoral neck in the image may be used to set an initial x and y position of the model. The center of the femoral head in the model may be positioned in the same location as the center of the femoral head in the two-dimensional image, and the midline of the femoral neck in the model may be positioned such that it extends in the same direction from the center of the femoral head as the midline of the femoral neck in the two-dimensional image. The radius of the femoral head may be used to determine an initial z position of the three-dimensional model. The z position of the three-dimensional model may be determined by calculating the distance between the three-dimensional model and a simulated camera that causes a projection of the three-dimensional model onto a two-dimensional plane to align with the two-dimensional image. The distance between the simulated camera and the pre generated three-dimensional model may be calculated by first determining the radius of the femoral head in the two-dimensional image and the radius of the femoral head in the pre-generated three-dimensional model. The distance between the pre-generated three-dimensional model and the simulated camera that causes the radius of the three-dimensional femoral head shown in the three-dimensional model to become the radius of the two-dimensional femoral head shown in the two-dimensional image when projected into a two-dimensional plane may then be computed.

Once the initial position of the three-dimensional model is determined, a first orientation of the three-dimensional model is set in step 706. In some examples, the first orientation may be set based on the likely orientation of the bone represented by the model during a surgical procedure. In other examples, the first orientation may be a predetermined orientation. For instance, an ultimate "initial orientation" (which may later be optimized) may be determined by performing a grid search that identifies a plurality of rotated positions of the three-dimensional model and computes a similarity metric between the two-dimensional image and a two-dimensional projection of the three-dimensional model for each position. In that case, the first orientation may be a predetermined rotation of the model, such as (-90, -90, -90).

Once the first orientation has been set, the three-dimensional model is projected onto a two-dimensional plane at step 708. The three-dimensional model may be projected onto the two-dimensional plane in the first orientation in order to visualize how closely the model in the first orientation aligns with the bone in the two-dimensional image.

A similarity metric is then computed between the projected three-dimensional model and the two-dimensional image at step 710. In some examples, the similarity metric used may be a cost function such as the intersection over union (IoU). IoU is a measure of overlap. To calculate IoU, the number of pixels that are shared between a pixel mask corresponding to the two-dimensional image and a pixel mask corresponding to the projection of the three-dimensional model onto the two-dimensional plane of the image is calculated. The total number of unique pixels contained in both masks combined is also calculated. IoU is then computed by dividing the number of shared pixels (the intersection) by the total number of unique pixels across both masks (the union). The possible values of loU range from 0 (indicating there is no overlap between the pixel masks) to 1 (indicating a perfect overlap between the pixel masks).

Cost functions other than IoU may be used to evaluate alignment, such as reprojection error, iterative closest point (ICP) error, mutual information (MI), sum of squared distances (SSD), or normalized cross correlation (NCC). Optionally, the cost function used may have smooth gradients (e.g., IoU, MI, SSD, NCC) such that local minima do not interfere with locating the absolute minimum of the cost function (which may indicate the optimal orientation of the pre-generated three-dimensional model with respect to the two-dimensional image).

At step 712, a decision is made whether to continue to search for an initial orientation by setting a new orientation or to move on to the determination of the initial orientation based on the orientations already tested, such as by determining which orientation had the highest similarity metric when compared with the two-dimensional image. In some examples, if the similarity metric computed at step 710 for a given orientation exceeds a predetermined threshold value, the decision may be to stop iterating. In other examples, the decision of whether to continue to iterate is based on a predetermined criterion, such as whether a predetermined number of iterations have been completed. For instance, the initial orientation may be determined by performing a grid search in which a similarity metric is computed for multiple rotations of the three-dimensional model between the rotated positions (-90, -90, -90) and (90, 90, 90) by changing one variable at a time in 20-degree increments. The grid search may check the rotated positions (-90, -90, -90), (-90, -90, -70), (-90, -90, -50), and so on until reaching (90, 90, 90). The rotated position for which the similarity metric indicates the highest degree of similarity between the projection of the three-dimensional model and the two-dimensional image may be selected as the initial orientation of the 3D model. Thus, the decision may be to continue iterating until each 20-degree rotated position between (-90, -90, -90) and (90, 90, 90) has been checked. It should be appreciated that in other aspects, increments other than 20-degrees may be used.

If the decision at step 712 is to continue, a new orientation is set at step 714. As described above, the new orientation may be a predetermined rotated position between (-90, - 90, -90) and (90, 90, 90). Once the new orientation is set, steps 708 and 710 may be repeated for the new orientation.

If the decision at step 712 is to stop iterating, the orientation with the highest similarity metric out of all orientations evaluated in the previous steps (i.e., the first orientation set in step 706 and all new orientations set in each iteration of step 714) may be selected. The selected orientation may be set as the initial orientation 718.

Once the initial orientation of the three-dimensional model is determined, the initial orientation is optimized at step 720 in order to complete registration of the three-dimensional model to the two-dimensional image. The orientation of the three-dimensional model may be optimized using a gradient descent algorithm. Optionally, the gradient descent algorithm may be the Adam algorithm. The gradient descent algorithm may compute the approximate gradient of the cost function (i.e., IoU) with respect to each variable. In the context of orientation, the variables include the six degrees of freedom: rotation in the x, y, and z directions and translation in the x, y, and z directions. The gradient descent algorithm may use the computed gradients to optimize each variable. The gradient descent algorithm may optimize each variable by using the finite difference method, partial differentiation, or any other suitable technique.

Using translation in the x-direction as an example, if the initial orientation of the three-dimensional model has an x-axis translation value of 10 mm, and the IoU for that value is 90, the gradient descent algorithm may compute the IoU for an x-axis translation value of 9 mm and an x-axis translation value of 11 mm. If the IoU for 9 mm is less than 90 and the IoU for 11 mm is greater than 90, the gradient descent algorithm may recognize that increasing the x-axis translation value above 10 mm improves IoU. The gradient descent algorithm may subsequently use an x-axis translation value greater than 10 mm in the next iteration. The subsequent x-axis translation value may be based on the computed gradient with respect to x-axis translation and the learning rate of the gradient descent algorithm. The learning rate may decay over time.

The gradient descent algorithm may continue to iteratively guess values for each variable using the techniques described above until the cost function for each variable is minimized or satisfies a predetermined criterion. For example, if IoU is used as the cost function, the gradient descent algorithm may continue to iteratively guess values for each variable until the IoU is equal to 1 (indicating a perfect alignment) or until the IoU exceeds a predetermined threshold (e.g., about 0.95). Alternatively, the gradient descent algorithm may continue to iterate for a predetermined amount of time or for a predetermined number of iterations. The end result of the iterative process is a determined three-dimensional model orientation that, when projected into the two-dimensional plane, aligns with the two-dimensional image, thus completing registration of the three-dimensional model to the two-dimensional image.

Returning to FIG. 6A, at step 606, a position and orientation of the electromagnetic tracking device relative to the three-dimensional model is determined. The position and orientation of the electromagnetic tracking device relative to the two-dimensional image determined in step 602 is combined with the alignment of the three-dimensional model to the two-dimensional image determined in step 604 to determine the position and orientation of the electromagnetic tracking device relative to the three-dimensional model.

At step 608, the three-dimensional model is registered in the common reference frame (i.e., the tracking system reference frame) based on the position and orientation of the electromagnetic tracking device relative to the three-dimensional model determined in step 606. The position and orientation of the electromagnetic tracking device relative to the three-dimensional model determined in step 606 is combined with the position and orientation of the electromagnetic tracking device in the common reference frame to register the three-dimensional model in the common reference frame.

At step 610, the endoscopic imager is registered in the common reference frame. The endoscopic imager may be registered in the common reference frame using the tracking system used to track the endoscopic imager. As described above with reference to FIG. 4, the endoscopic imager may be tracked by electromagnetic tracking system 400. For example, an electromagnetic tracking device (e.g., endoscope tracking device 406) may be affixed to the endoscopic imager, such that the position and orientation of the endoscopic imager can be tracked within the electromagnetic field generated by an electromagnetic field generator (e.g., electromagnetic field generator 404). The position and orientation of the endoscopic imager in the tracking system reference frame may therefore be known. The position and orientation of the endoscopic imager in the tracking system reference frame may then be transformed into the common reference frame (if the common reference frame is a reference frame other than the tracking system reference frame) to register the endoscopic imager in the common reference frame.

At step 612, the three-dimensional model is registered with the endoscopic imager in the common reference frame. Because three-dimensional model is registered in the common reference frame as a result of step 608, and the endoscopic imager is registered in the common reference frame as a result of step 610, the three-dimensional model and endoscopic imager can be registered with each other in the common reference frame.

As described above, method 600 is only one example of a process that can be used for step 204 of method 200. Method 600 illustrates a process that can be used to register a three-dimensional model with an endoscopic imager in a common reference frame (e.g., the tracking system reference frame) using an electromagnetic tracking system, such as system 400 described above with reference to FIG. 4. In some examples, the common reference frame in which the three-dimensional model is registered with the endoscopic imager may be a virtual world reference frame associated with the three-dimensional model (e.g., a reference frame in which the three-dimensional model is fixed).

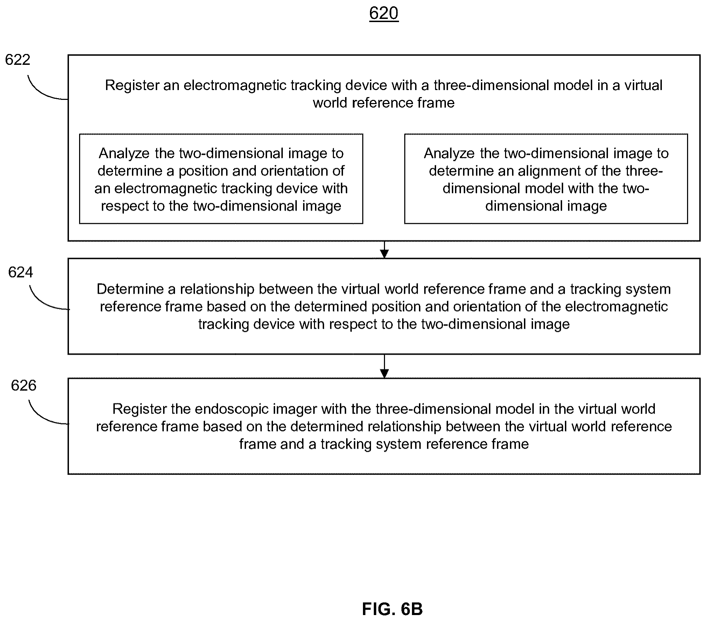

FIG. 6B illustrates an exemplary method 620 for registering a three-dimensional model with an endoscopic imager in a virtual world reference frame using an electromagnetic tracking system. At step 622, an electromagnetic tracking device is registered with a three-dimensional model of the bony anatomy in a virtual world reference frame. The two-dimensional image may be analyzed to determine an alignment of the three-dimensional model with the two-dimensional image, such as by using method 700 described above or any other suitable method. The two-dimensional image may be analyzed to determine a position and orientation of an electromagnetic tracking device with respect to the two-dimensional image. The two-dimensional image may be analyzed using any of the methods described above with reference to step 602 of FIG. 6A (e.g., by using one or more machine learning models or image processing techniques). The determined alignment of the three-dimensional model with the two-dimensional image and the determined position and orientation of the electromagnetic tracking device with respect to the two-dimensional image may be combined to register the electromagnetic tracking device with the three-dimensional model in the virtual world reference frame.

At step 624, a relationship between the virtual world reference frame and a tracking system reference frame is determined based on the determined position and orientation of the electromagnetic tracking device with respect to the two-dimensional image. Since the position and orientation of the electromagnetic tracking device is known in both the virtual world reference frame (from step 622) and in the tracking system reference frame (based on the tracking of the electromagnetic tracking device), the relationship between the tracking system reference frame and the virtual world reference frame can be determined using well-known reference frame transformation techniques. With this relationship established, any component tracked by the tracking system can be registered in the virtual world reference frame using the relationship between the tracking system reference frame and the virtual world reference frame.

At step 626, the endoscopic imager is registered with the three-dimensional model in the virtual world reference frame based on the determined relationship between the tracking system reference frame and the virtual world reference frame. As discussed above with reference to FIG. 4, the endoscopic imager may be tracked by electromagnetic tracking system 400 (e.g., using endoscope tracking device 406). The position and orientation of the endoscopic imager in the tracking system reference frame may then be transformed into the virtual world reference frame based on the relationship between the tracking system reference frame and the virtual world reference frame determined in step 624.

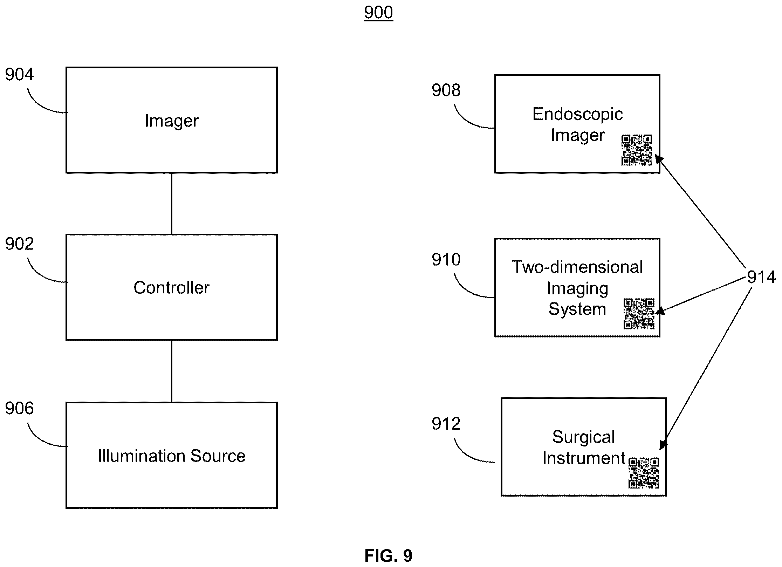

Both methods 600 and 620 are examples of using an electromagnetic tracking system to register a three-dimensional model with an endoscopic imager in a common reference frame. Other examples of processes that can be used for step 204 of method 200 may use other types of tracking systems. For example, a three-dimensional model can be registered with an endoscopic imager in a common reference frame using an optical tracking system, such as an infrared-camera based tracking system, instead of an electromagnetic tracking system. FIG. 9 illustrates an exemplary optical tracking system 900 that may be used to register a three-dimensional model in a common reference frame. Optical tracking system 900 may include a controller 902 configured to control an imager 904 and an illumination source 906 (e.g., by turning them on and off or changing various imaging and light settings). Imager 904 and illumination source 906 may be connected to controller 902 wirelessly or via a wired connection. Controller 902 may be a computing system including one or more processors and may include user input/output functionality, such as a touchscreen, keyboard, mouse, or other interactive feature to enable a user to control aspects of the tracking system. Controller 902 may be communicatively coupled to an image processing system, such as imaging processing system 106 of system 100, that can use information from tracking system 900 to guide a surgeon during a surgical procedure.

Imager 904 may be a camera, such as an infrared camera. Imager 904 may be a stereoscopic camera. Illumination source 906 may be configured to provide light (e.g., infrared light or any other suitable light) for imager 904. Imager 904 may be used to image various objects in order to track their position and orientation within a common reference frame. The objects may be tracked by imager 904 based on one or more light emitters (e.g., LEDs) affixed to the objects, reflective articles (e.g., light reflectors) affixed to the objects, and/or fiducial markers 914 affixed to (e.g., attached to, printed on, etched into, formed into, etc.) the objects. For example, fiducial markers 914 visible to imager 904 under infrared light provided by illumination source 906 may be affixed to an endoscopic imager 908 (e.g., to an endoscope, camera head, and/or handle of the endoscopic imager), a two-dimensional imaging system 910, and/or a surgical instrument 912. Fiducial markers 914 may be used to determine the position and orientation of each object within the common reference frame. Based at least partially on the position and orientation of one or more of endoscopic imager 908, two-dimensional imaging system 910, and surgical instrument 912, a three-dimensional model may be registered in the common reference frame.

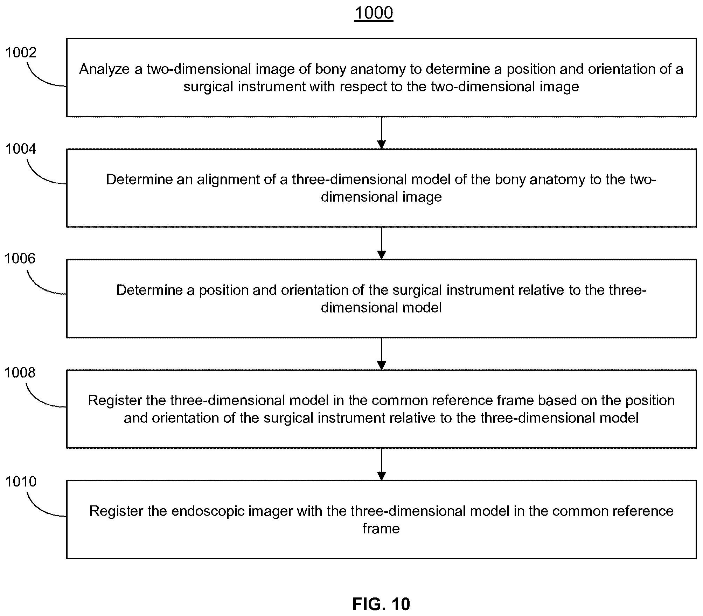

As described above, optical tracking system 900 can be used to register a three-dimensional model with an endoscopic imager in a common reference frame in step 204 of method 200. FIG. 10 illustrates an exemplary method 1000 for registering a three-dimensional model of bony anatomy with an endoscopic imager in a common reference frame using an optical tracking system. Method 1000 may be used for step 204 of method 200 instead of methods 600 or 620. Method 1000 may be performed by one or more processors, such as one or more processors of image processing system 106 described above with reference to FIG. 1.

At step 1002, a two-dimensional image of bony anatomy is analyzed to determine a position and orientation of a surgical instrument with respect to the two-dimensional image. The two-dimensional image analyzed may be the two-dimensional image of the bony anatomy received in step 202 of method 200. The two-dimensional image may include a surgical instrument, such as a bur, shaver, drill, or any other suitable surgical instrument that can be positioned against the bony anatomy, visible outside of the body, and captured in the two-dimensional image. Optionally, the surgical instrument may include one or more radiopaque fiducial markers on a portion of the surgical instrument that is inside of a patient's body, which may be used to determine the position and orientation of the surgical instrument with respect to the two-dimensional image. The surgical instrument may include one or more light emitters and/or one or more fiducial markers that are external to the patient's body and are configured to be tracked by an imager of an optical tracking system (e.g., imager 904 of optical tracking system 900). For example, one or more glyphs (e.g., a QR code or an ArUco), or any other suitable optical marker may be affixed to the portion of the surgical instrument that remains external to the patient's body. Optionally, a fiducial marker may be visible under infrared light, such that the surgical instrument can be tracked using an infrared camera but may not be visible in the two-dimensional image.

The two-dimensional image may be analyzed using one or more machine learning models. For example, image processing system 106 of FIG. 1 may be configured to use one or more machine learning models to determine a position and orientation of a surgical instrument with respect to the two-dimensional image. Optionally, as described above with reference to FIGS. 7 and 8, the position and orientation of the surgical instrument within the image may be determined by manipulating a three-dimensional model of the surgical instrument according to the available degrees of freedom (e.g., translation in the x, y, and z directions, rotation about these axes, and scaling relative to the viewpoint of a simulated camera), such that a projection of the model onto a two-dimensional plane that corresponds with the imaging plane aligns with the surgical instrument in the two-dimensional image. Additionally, or alternatively, the position and orientation of one or more radiopaque fiducial markers of the surgical instrument appearing in the two-dimensional image may be determined and compared to known attributes of the one or more radiopaque fiducial markers to determine the position and orientation of a surgical instrument with respect to the two-dimensional image.

At step 1004, an alignment of a three-dimensional model of the same bony anatomy captured in the two-dimensional image to the two-dimensional image is determined. Step 1004 may be performed by applying method 700 described above with reference to FIG. 7.

At step 1006, a position and orientation of the surgical instrument relative to the three-dimensional model is determined. The position and orientation of the surgical instrument relative to the two-dimensional image determined in step 1002 is combined with the alignment of the three-dimensional model to the two-dimensional image determined in step 1004 to determine the position and orientation of the surgical instrument relative to the three-dimensional model.

At step 1008, the three-dimensional model is registered in the common reference frame (i.e., the tracking system reference frame) based on the position and orientation of the surgical instrument relative to the three-dimensional model determined in step 1006. The position and orientation of the surgical instrument relative to the three-dimensional model determined in step 1006 is combined with the position and orientation of the surgical instrument in the common reference frame to register the three-dimensional model in the common reference frame.

At step 1010, the endoscopic imager is registered with the three-dimensional model in the common reference frame. The endoscopic imager may be registered in the common reference frame using the tracking system used to track the endoscopic imager. As described above with reference to FIG. 9, an endoscopic imager 908 may be tracked by optical tracking system 900 based on a visual fiducial 914 affixed to the endoscopic imager. The position and orientation of the endoscopic imager in the tracking system reference frame may therefore be known. The position and orientation of the endoscopic imager in the tracking system reference frame may then be transformed into the common reference frame (if the common reference frame is a reference frame other than the tracking system reference frame) to register the endoscopic imager in the common reference frame.

In some examples, the common reference frame in which the three-dimensional model and endoscopic imager are registered to one another using an optical tracking system that tracks a surgical instrument may be a reference frame associated with the three-dimensional model (e.g., a virtual world reference frame, as discussed above with reference to FIG. 6B). Registering an endoscopic imager with a three-dimensional model in a virtual world reference frame using an optical tracking system that tracks a surgical instrument may be done by using the two-dimensional image to determine a relationship between the surgical device tracked by the optical tracking system and the three-dimensional model. For example, an approach analogous to step 622 of method 620 of FIG. 6B may be used register the optically tracked surgical instrument with the three-dimensional model in the virtual world reference frame. The position and orientation of the optically tracked surgical instrument in the optical tracking system reference frame can then be used to determine a relationship between the virtual world reference frame and an optical tracking system reference frame in similar fashion to step 624 of FIG. 6B. Anything tracked by the optical tracking system, such as the endoscopic imager, can then be registered in the virtual world reference frame based on the relationship between the virtual world reference frame and the optical tracking system reference frame.

It should be appreciated that techniques other than those described above may be used to register a three-dimensional model of bony anatomy with an endoscopic imager in a common reference frame. For example, a three-dimensional model may be registered to bone based on the movement of a tracked surgical instrument being touched to multiple locations of a bone. The surgical instrument may be tracked using an electromagnetic tracking system, an optical tracking system, and/or one or more IMUs attached to the surgical instrument. To register the three-dimensional model to bone, a user may touch the tracked surgical instrument against multiple points on the bone to establish the contour of the bone in the coordinate system of the tracking system. Alternatively, the user may drag the tracked surgical instrument along the surface of the bone to establish the contour of the bone. If the bone is moved during surgery (e.g., if the patient's leg is repositioned), a user may repeat this process to ensure the registration remains accurate as the patient moves. In some aspects, touching the tracked surgical instrument against the bone may also increase the quality of the three-dimensional model. For example, during a bone removal procedure, if the tracked surgical instrument is a bur tool, a user may drag the bur along the surface of the bone that was just burred during a pause in burring in order to better reflect bone removal in the three-dimensional model. This may improve the quality of the three-dimensional model in at least the region where bone has been resected.

Returning to FIG. 2, at step 206, the position and orientation of the three-dimensional model relative to the at least one endoscopic image may be determined (e.g., using one or more processors of image processing system 106). The position and orientation of the three-dimensional model relative to the at least one endoscopic image may be determined based on the registration of the three-dimensional model with the endoscopic imager in the common reference frame. One or more attributes of the endoscopic imager may be used to transform the registration of the three-dimensional model relative to the endoscopic imager to registration of the three-dimensional model relative to the endoscopic image. One example of an attribute of the endoscopic imager is the rotation of the endoscope of the endoscopic imager relative to the bony anatomy. For example, the endoscope of the endoscopic imager may be rotatable with respect to the camera head of the endoscopic imager and its rotation may determine the orientation of the three-dimensional model relative to the endoscopic image.

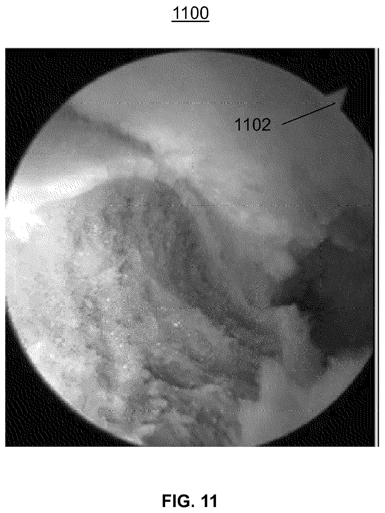

The endoscopic image may be analyzed (e.g., by one or more processors of image processing system 106) to determine a rotation of an endoscope of an endoscopic imager relative to the camera head. This may be used in examples in which the endoscope is rotatable relative to the imaging sensor(s) and the camera head is tracked by the tracking system but not the endoscope. Rotational position of the scope relative to the camera head may be determined based on the position of a caret-shaped protrusion on the perimeter of the field of view edge of the endoscopic image, which results from a corresponding caret-shaped feature in the optical pathway of the endoscope. FIG. 11 shows an exemplary endoscopic image 1100 that includes a caret 1102 at the top right-hand side of the image. The location of caret 1102 relative to the imaging sensor of the endoscopic imager in a nominal scope orientation may be known. Thus, the rotational angle of the endoscope may be determined based on the deviation of the location of caret 1102 relative to its baseline location when the endoscopic imager is in the nominal orientation. Determining the rotational angle of the endoscope may help determine the orientation of the bony anatomy in the endoscopic image captured by the endoscopic imager (e.g., endoscopic imager 112) relative to the tracked endoscopic imager, and thus the orientation of the three-dimensional model relative to the endoscopic image.

In some examples, the rotational angle of the endoscope of the endoscopic imager may be determined relative to a camera head of the endoscopic imager using the tracking system. For instance, an electromagnetic tracking device or a fiducial marker may be affixed to the endoscopic imager, and the rotational angle of the endoscope of the endoscopic imager may be determined based on the position and orientation of the electromagnetic tracking device or fiducial marker in the common reference frame. Alternatively, or additionally, a fiducial marker may be affixed to a surgical instrument (e.g., a bone removal instrument) that is visible in endoscopic images captured by the endoscopic imager. The endoscopic imager may be tracked, such as by using an electromagnetic tracking device, a fiducial marker, or one or more IMUs, and the position and orientation of the endoscopic imager in the common reference frame may be determined. The rotational angle of the endoscope of the tracked endoscopic imager may be determined based on the position and orientation of the fiducial marker affixed to the surgical instrument in the common reference frame, the position and orientation of the fiducial marker affixed to the surgical instrument in the endoscopic images, and the position and orientation of the tracked endoscopic imager in the common reference frame.

Furthermore, the depth of the bony anatomy captured in the endoscopic image relative to the endoscopic imager may be determined (e.g., by one or more processors of image processing system 106) and used to transform the registration of the three-dimensional model relative to the endoscopic imager to registration of the three-dimensional model relative to the endoscopic image. The depth of the bony anatomy captured in the endoscopic image relative to the endoscopic imager may be used to determine the scale of the three-dimensional model that aligns with the endoscopic image. The determination of the depth of the bony anatomy relative to the endoscopic imager may be based on a value associated with the distance between the bony anatomy captured in the endoscopic image and the camera of the endoscopic imager. The value associated with the distance may be determined from optical attributes of the endoscopic imager, such as the zoom, focus setting, or depth of field, or from analysis of the endoscopic image by one or more machine learning models.

Determining the depth of the bony anatomy in the endoscopic image relative to the endoscopic imager may include analyzing the endoscopic image to determine at least one value associated with a depth of the bony anatomy relative to the endoscopic imager. The at least one value associated with a depth of the bony anatomy relative to the endoscopic imager may be, for example, the actual distance between the bony anatomy and the endoscopic imager (e.g., the endoscope or another location on the camera head of the endoscopic imager) or a relative distance of the bony anatomy (e.g., relative to other features in the image). The at least one value may optionally be determined using one or more machine learning models. For example, image processing system 106 of FIG. 1 may be configured to use one or more machine learning models configured to determine the depth of bony anatomy from an endoscopic imager. For example, a machine learning model may be configured to generate a depth map in which a relative depth value is provided for each pixel of the image corresponding to the relative depth of that portion of the anatomy.

Optionally, the determination of the depth of the bony anatomy relative to the endoscopic imager may be based on a position and orientation of at least a portion of the endoscopic imager captured in the at least one two-dimensional image received at step 202. The position and orientation of the portion of the endoscopic imager in the two-dimensional image relative to the bony anatomy in the two-dimensional image may be determined by analyzing the image (for example, using one or more machine learning models implemented by image processing system 106 of FIG. 1). Based on the position and orientation of the endoscopic imager relative to the bony anatomy and a priori knowledge of one or more optical settings of the endoscopic imager (e.g., focus, depth of field, zoom, etc.), the depth of the bony anatomy relative to the endoscopic imager may be determined.

Optionally, the determination of the depth of the bony anatomy from the endoscopic imager may additionally or alternatively be based on a position and orientation of a surgical instrument tracked by the tracking system. The surgical instrument may be visible in an endoscopic image captured by the endoscopic imager. The endoscopic image may be analyzed (for example, using one or more machine learning models implemented by image processing system 106 of FIG. 1) to determine one or more features of the surgical instrument in the image, such as the size of the instrument within the endoscopic image or the presence of a fiducial marker on the instrument. Based on the analysis of the endoscopic image and a priori knowledge of the characteristics of the surgical instrument, the relationship between the surgical instrument and the surgical instrument as captured in the endoscopic image may be determined. The relationship may be used to determine the depth of the bony anatomy relative to the endoscopic imager. For example, one or more processors of image processing system 106 may determine the distance between the bony anatomy and the endoscopic imager by comparing the known size of the instrument with a size of the instrument as captured the endoscopic image.

Alternatively, or additionally, the position and orientation of the three-dimensional model relative to the at least one endoscopic image may be determined using computer vision techniques. For example, one or more machine learning models may be used to identify pixels that correspond to tissue within an endoscopic image. The one or more machine learning models may be configured to determine a three-dimensional surface based on the identified tissue. For instance, the one or more machine learning models may be configured to perform monocular depth estimation, which may include processing an endoscopic image (or portion thereof that corresponds to tissue) with a machine learning model that is trained to estimate depth values in images. A three-dimensional surface may be determined based on the depth values. The determined three-dimensional surface may then be aligned with a corresponding portion of the three-dimensional model of the bony anatomy, thereby aligning the endoscopic image with the three-dimensional model.

Finding the portion of the three-dimensional model to which a determined three-dimensional surface corresponds may be easier the larger the determined surface is. A larger surface may be determined by stitching together multiple endoscopic image images to visualize more of the bony anatomy and analyzing the stitched-together images to determine the surface. For example, multiple endoscopic images may be input into a Local Feature Transformer (LoFTR) algorithm, which may return a list of two-dimensional coordinates in the images that correspond to the same three-dimensional point in space (e.g., the same object). The list of points may be used to determine a spatial relationship between the images, and the images may be stitched together accordingly. The stitched-together images may then be used to determine a three-dimensional surface, such as by using monocular depth estimation and/or by analyzing parallax effects. The three-dimensional surface may then be compared to the three-dimensional model to determine an alignment of the three-dimensional surface, and thus the endoscopic image(s) used to determine the three-dimensional surface, to the three-dimensional model.

At step 208, the three-dimensional model is displayed based on the determined position and orientation of the three-dimensional model relative to the endoscopic image. The three-dimensional model may be displayed to a surgeon to guide bone removal, for example via display 116 described above with reference to FIG. 1. The three-dimensional model may be displayed with or without the endoscopic image. According to some aspects, the three-dimensional model may be displayed in the same pose as the bony anatomy shown in the endoscopic image, such that a projection of the three-dimensional model into two-dimensions aligns with the bony anatomy shown in the endoscopic image. The alignment of the three-dimensional model relative to the endoscopic image may be determined using the techniques discussed above with respect to step 206. To ensure that the three-dimensional model is shown in the same pose as the bony anatomy shown in subsequent endoscopic images over time (e.g., as the endoscopic imager moves), the endoscopic imager may be tracked. For example, the endoscopic imager may be tracked using a tracking system (e.g., an electromagnetic tracking system and/or an optical tracking system) or by using one or more image processing techniques (e.g., computer vision techniques and/or other artificial intelligence or machine learning techniques). Any one or more tracking techniques may be combined (e.g., an electromagnetic tracking system may be used in combination with computer vision techniques).

In some aspects, the bony anatomy itself may be moved intentionally or unintentionally during the surgical procedure. To account for movement of the bony anatomy, the bony anatomy and/or other anatomical structures may be tracked using any one or more of the tracking techniques disclosed herein. Tracking the endoscopic imager and bony anatomy ensures that display of the three-dimensional model remains accurate as the endoscope and/or bony anatomy move throughout the surgical procedure and the display is updated. Tracking the endoscopic imager and bony anatomy enables real-time updating of the position of the three-dimensional model relative to the endoscopic imager, such that the displayed three-dimensional model remains in alignment with endoscopic images captured by the endoscopic imager as the endoscopic imager and/or bony anatomy move.

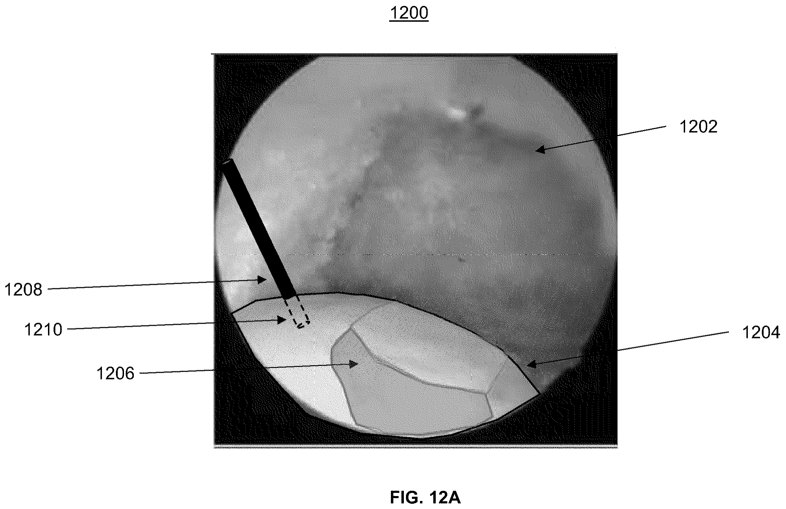

The three-dimensional model may be displayed in various manners. For example, the three-dimensional model may be displayed as an overlay on the endoscopic image. An exemplary overlay image 1200 is shown in FIG. 12A. FIG. 12A shows a portion of a three-dimensional model 1204 overlaid on an endoscopic image 1202. Three-dimensional model 1204 includes a heat map 1206 covering a portion of the bony anatomy that deviates from a target bone morphology. Heat map 1206 may serve as a plan for a surgical procedure that indicates to the surgeon where and how much bone should be removed to achieve the target morphology. Heat map 1206 may be color-coded according to the planned depth of bone removal. For example, as shown in FIG. 12A, heat map 1206 includes a teal blue portion corresponding to a first depth of bone removal and a dark blue portion corresponding to a different, second depth of bone removal. Alternatively, or additionally, an outline of the planned bone removal area, a contour map indicating amounts of bone removal, or any other suitable visual aid may be used to represent planned bone removal. Optionally, three-dimensional model 1204 (and heat map 1206) may be at least partially transparent, such that the bony anatomy in the endoscopic image is visible through the overlay of three-dimensional model 1204 and heat map 1206.

In some examples, the endoscopic image 1202 may capture a surgical instrument 1208 (e.g., a bone removal tool such as a bur, shaver, drill, saw, etc.). Surgical instrument 1208 may be located in a portion of endoscopic image 1202 that is overlaid with three-dimensional model 1204. For example, the distal portion 1210 of surgical instrument 1208 is located in the region of endoscopic image 1202 covered by three-dimensional model 1204. As discussed above, three-dimensional model 1204 may be partially transparent. Thus, the portion of surgical instrument 1208 covered by three-dimensional model 1204 (e.g., distal portion 1210) may be visible through the partially transparent three-dimensional model 1204. In implementations where surgical instrument 1208 is a bone removal tool, this may allow a user to visualize where bone is being removed. In some aspects, an indication of planned bone removal (e.g., heat map 1206) may be displayed on top of at least a portion of surgical instrument 1208. The indication of planned bone removal may be opaque, or alternatively may be at least partially transparent, such that surgical instrument 1208 is at least partially visible through the indication of planned bone removal. In some examples, surgical instrument 1208 may be identified in the endoscopic image (e.g., by one or more machine learning models). The one or more machine learning models may determine that a portion of surgical instrument 1208 overlaps with the area of endoscopic image 1202 covered by three-dimensional model 1204 (and, optionally, heat map 1206). Image processing system 106 may indicate the portion of the surgical instrument 1208 that overlaps with three-dimensional model 1204 and/or heat map 1206 using coloring, shading, or patterns. For example, in FIG. 12A, distal portion 1210 represents the portion of the surgical instrument 1208 that overlaps with three-dimensional model 1204 (the overlapping portion is schematically represented using dashed lines). In implementations involving a bone removal instrument, the one or more machine learning models may identify the bone removing feature of the instrument (e.g., distal portion 1210 of surgical instrument 1208) in the endoscopic image 1202. Image processing system 106 may generate a visualization in which the three-dimensional model 1204 is overlaid on the entirety of the endoscopic image 1202 except for a region corresponding to distal portion 1210, such that a user can visualize where bone is being removed.

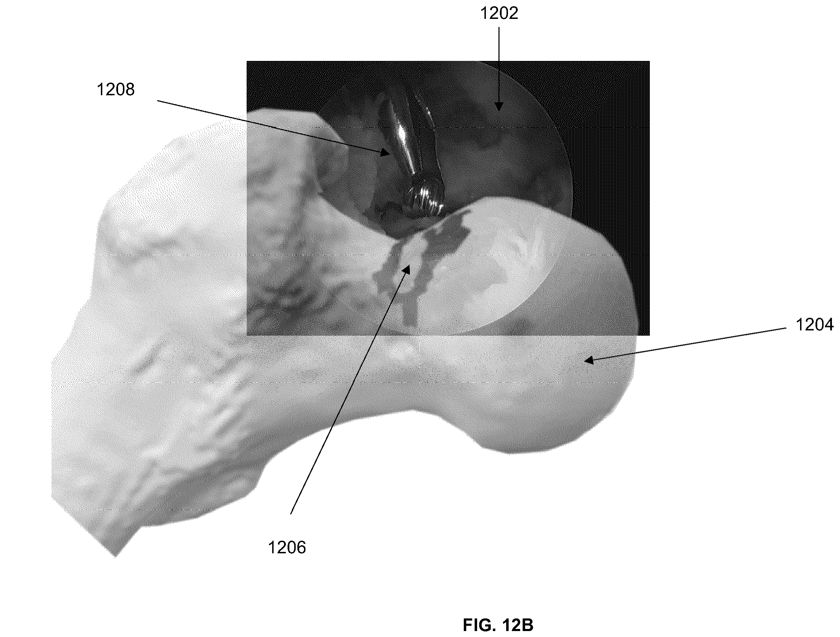

Optionally, the three-dimensional model 1204 overlaid on the endoscopic image 1202 may extend beyond the boundaries of the endoscopic image. For example, as shown in FIG. 12B, the three-dimensional model 1204 (and its corresponding heat map 1206) extends outside of the endoscopic image 1202, such that anatomical structures outside of the endoscopic field of view are visible to a user.

Optionally, overlay image 1200 may be updated throughout the surgical procedure as new endoscopic images are captured. Three-dimensional model 1204 may be displayed in the same pose as the bony anatomy in each new endoscopic image 1202. According to other aspects, the pose of three-dimensional model 1204 may be updated less frequently than endoscopic images are captured (e.g., the pose of the three-dimensional model 1204 may be updated once for every N endoscopic images captured), and three-dimensional model 1204 may be displayed in a slightly different pose as compared to the bony anatomy in a given endoscopic image 1202. The pose of three-dimensional model 1204 relative to the bony anatomy in an endoscopic image may be determined using the techniques disclosed above with reference to step 206 of method 200. As discussed above, the pose of three-dimensional model 1204 relative to the bony anatomy in subsequent endoscopic images 1202 may be maintained by tracking the endoscopic imager used to capture endoscopic images 1202. Optionally, the bony anatomy may also be tracked to account for movement of the bony anatomy itself during the surgical procedure.

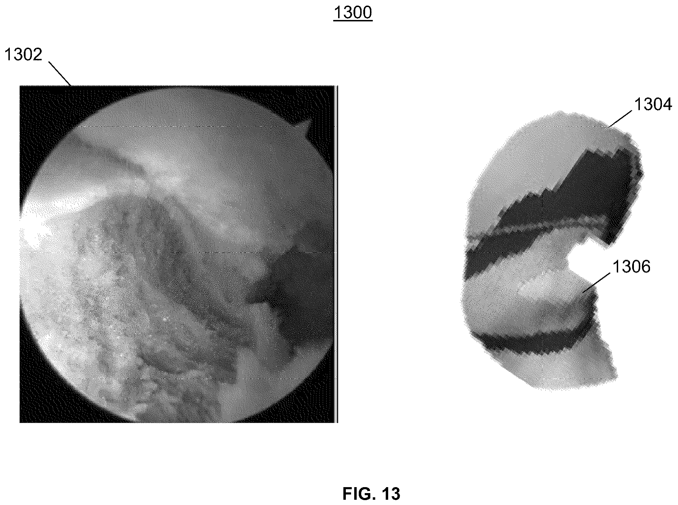

In some aspects, the three-dimensional model may be displayed simultaneously with but separately from the at least one endoscopic image. For example, as illustrated in FIG. 13, a visualization 1300 may include an endoscopic image 1302 displayed next to a corresponding portion of a three-dimensional model 1304 that aligns with endoscopic image 1302. As shown in FIG. 13, the three-dimensional model 1304 may include an indication of planned bone removal 1306.

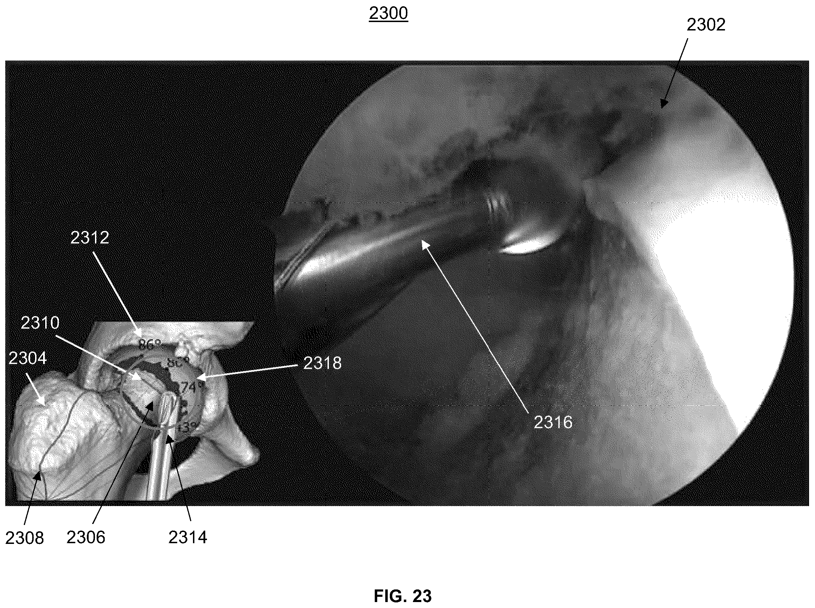

As illustrated in FIG. 13, the displayed endoscopic image and corresponding portion of a three-dimensional model may be shown at the same scale. Alternatively, the endoscopic image and the three-dimensional model may be shown at different scales. For example, the three-dimensional model and endoscopic image may be displayed using "picture-in-picture" techniques, in which the endoscopic image is displayed prominently, and the three-dimensional model is displayed in a smaller window overlaid on a portion of the endoscopic image. FIG. 23 illustrates a visualization 2300 in which endoscopic video 2302 is displayed prominently in a first portion of visualization 2300, and a portion of the three-dimensional model 2304 corresponding to the displayed endoscopic video 2302 is displayed in a second, less prominent portion of visualization 2300. The portion of the three-dimensional model 2304 may include an indication of planned bone removal, such as heat map 2306. Alternatively, the portion of the three-dimensional model 2304 may be displayed without heat map 2306.

Optionally, visualization 2300 may indicate which region of the displayed portion of three-dimensional model 2304 is being shown in endoscopic image 2302. For example, as shown in FIG. 23, a circle 2318 encloses the region of three-dimensional model 2304 that corresponds to endoscopic image 2302. Alternatively, the region of three-dimensional model 2304 corresponding to what is shown in endoscopic image 2302 may be indicated by highlighting the region of three-dimensional model 2304 being shown and/or by graying out the region of three-dimensional model 2304 that is not being shown. Alternatively, or additionally, the region of three-dimensional model 2304 being shown may be displayed using a different colorization (e.g., by brightening the region being viewed, increasing the contrast in the region being viewed, etc.).

Optionally, the portion of the three-dimensional model 2304 may include one or more clock-face lines 2308. As is known in the art, clock-face lines are useful for surgeons to identify positions within a joint (e.g., within a hip joint, clock-face lines may help a surgeon identify rotational positions about the femoral head or the acetabular cup). The portion of the three-dimensional model 2304 may also include one or more Alpha Angle lines 2310. An Alpha Angle line 2310 may represent the set of circumferential locations where the bony anatomy first extends outside of a best-fit sphere (or a plurality of best-fit circles) around the femoral head. The portion of the three-dimensional model 2304 may also include one or more Alpha Angle points 2312, which may indicate the location of the start of a given pathology relative to a predetermined clock-face position.

A representation of a surgical instrument 2314 may be displayed with respect to the portion of the three-dimensional model 2304. The representation of the surgical instrument 2314 may be a representation of surgical instrument 2316, which is shown in endoscopic image 2302. The representation of the surgical instrument 2314 may be displayed in a location relative to the portion of the three-dimensional model 2304 that corresponds to the location of surgical instrument 2316 relative to the bony anatomy in endoscopic image 2302. The representation of the surgical instrument 2314 may be opaque, as shown in FIG. 23, or may be at least partially transparent. The user may selectively adjust the transparency of the representation of the surgical instrument 2314. The representation of the surgical instrument 2314 may be toggled on and off by a user.

Optionally, visualizations 1300 and/or 2300 may be updated throughout the surgical procedure as new endoscopic images are captured. The respective three-dimensional model may be displayed in the same pose as the bony anatomy in each new endoscopic image. According to other aspects, the pose of the respective three-dimensional model may be updated less frequently than endoscopic images are captured (e.g., the pose of the respective three-dimensional model may be updated once for every N endoscopic images captured), and the respective three-dimensional model may be displayed in a slightly different pose as compared to the bony anatomy in a given endoscopic image. The pose of the three-dimensional model relative to the bony anatomy in an endoscopic image may be determined using the techniques disclosed above with reference to step 206 of method 200. As discussed above, the pose of the three-dimensional model relative to the bony anatomy in subsequent endoscopic images may be maintained by tracking the endoscopic imager used to capture the endoscopic image. Optionally, the bony anatomy may also be tracked to account for movement of the bony anatomy itself during the surgical procedure.

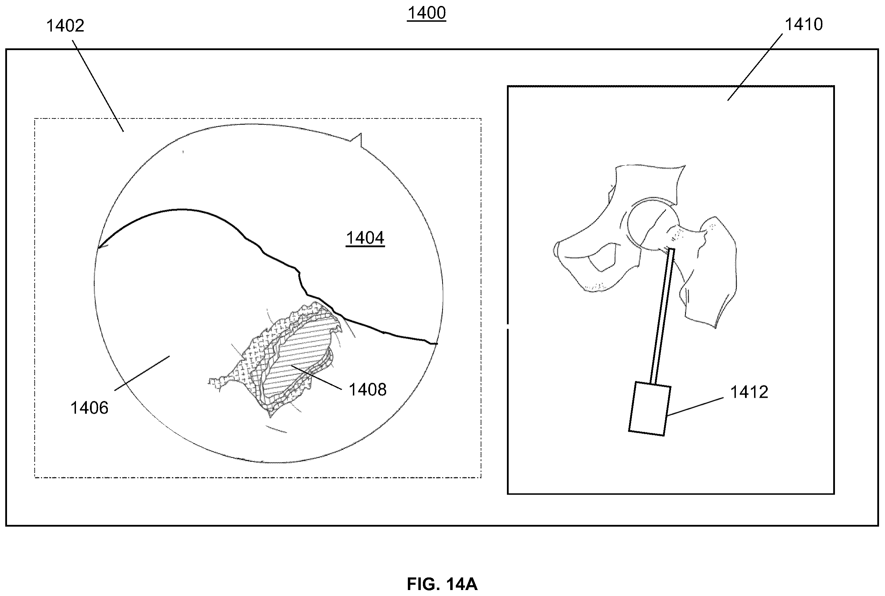

In some aspects, the three-dimensional model may be displayed as a simulated endoscopic image that includes the portion of the three-dimensional model that corresponds to the bony anatomy captured in the endoscopic image. Exemplary visualizations including a simulated endoscopic image are illustrated in FIGS. 14A-B. FIG. 14A illustrates a first visualization 1400 including a simulated endoscopic image 1402 simulating an endoscopic image captured from a first perspective. Simulated endoscopic image 1402 shows a simulated field of view 1404 of an endoscope. The simulated endoscopic image 1402 includes only the portion of the three-dimensional model 1406 that corresponds to what is shown in the endoscopic image being simulated. Optionally, the simulated endoscopic image 1402 includes more of the three-dimensional model 1406 beyond what is shown in the endoscopic image being simulated (e.g., the three-dimensional model may extend beyond the boundaries of what is shown in the simulated endoscopic field of view). The portion of the three-dimensional model 1406 that corresponds to what is shown in the endoscopic image being simulated may be determined based on the position and orientation of the endoscopic imager relative to the bony anatomy, which may be determined using any of the techniques discussed herein with reference to step 206 (e.g., by determining the rotation of the endoscope relative to the camera head of the endoscopic imager, determining a depth of the bony anatomy relative to the endoscopic imager, etc.).

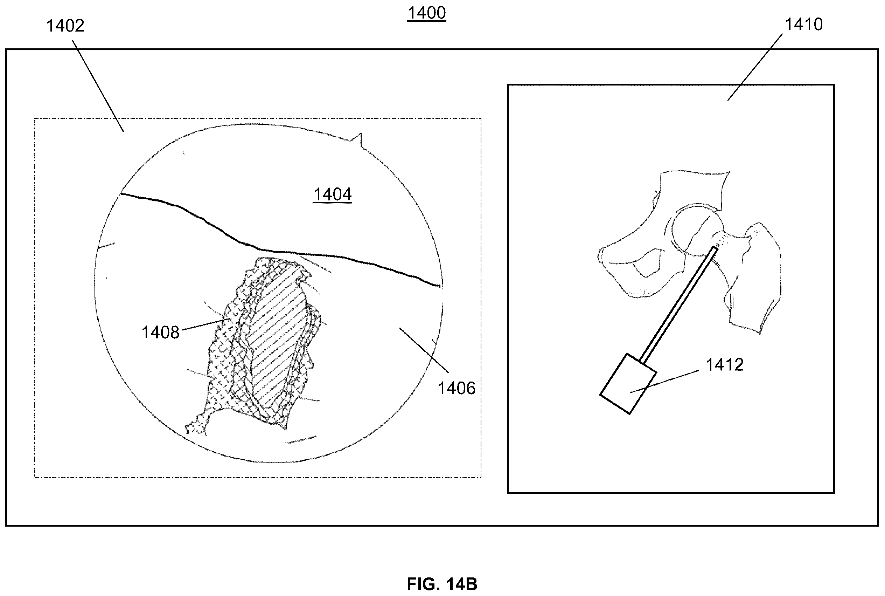

Visualization 1400 may include an indication 1410 of the perspective of simulated endoscopic image 1402. For instance, in the example of FIG. 14A, indication 1410 is included in a panel adjacent to simulated endoscopic image 1402. Indication 1410 illustrates the perspective of the endoscopic camera 1412 used to capture the endoscopic image upon which simulated endoscopic image 1402 is based relative to the bony anatomy. When the endoscopic camera 1412 is moved, simulated endoscopic image 1402 and indication 1410 may be updated accordingly. For example, as shown in FIG. 14B, indication 1410 shows that the endoscopic camera 1412 has moved to a different position relative to the bony anatomy compared to where the endoscopic camera 1412 was in FIG. 14A.

Display of the simulated endoscopic image 1402 may be advantageous in providing the surgeon with the portion of the three-dimensional model 1406 that corresponds to what is shown in the endoscopic image but without obscuring the endoscopic image. As shown in FIGS. 14A-B, three-dimensional model 1406 may include an indication of planned bone removal 1408. The indication of planned bone removal 1408 may be in the form of a color-coded heat map, where different colors indicate differing depths of bone removal. The simulated endoscopic image 1402 may provide a simplified view to a user. For instance, the simulated endoscopic image 1402 may omit unnecessary tissues and/or blockages from view. The simulated endoscopic image 1402 may also result in enhanced image display (e.g., by removing jitter from the displayed image). Furthermore, the simulated endoscopic image 1402 may be shown in a non-distorted manner as compared to a regular, non-simulated endoscopic image, which may appear distorted.

Visualization 1400 may be updated throughout the surgical procedure as new endoscopic images are captured. Simulated endoscopic image 1402 may be updated such that the position and orientation of three-dimensional model 1406 matches the position and orientation of the bony anatomy shown in the real endoscopic field of view. According to other aspects, the position and orientation of three-dimensional model 1406 may be updated less frequently than endoscopic images are captured (e.g., the pose of three-dimensional model 1406 may be updated once for every N endoscopic images captured), and three-dimensional model 1406 may be displayed in a slightly different pose as compared to the bony anatomy in the real endoscopic field of view. The pose of three-dimensional model 1401 relative to the bony anatomy shown in an endoscopic image may be determined using the techniques disclosed above with reference to step 206 of method 200. The pose of three-dimensional model 1401 relative to the bony anatomy in subsequent endoscopic images may be maintained by tracking the endoscopic imager. Optionally, the bony anatomy may also be tracked to account for movement of the bony anatomy itself during the surgical procedure.

One or more steps of method 200 may be repeated, such as to update a visualization based on new endoscopic images (e.g., to update a visualization as a series of video frames are received). For instance, steps 204, 206, and 208 may be repeated when a new endoscopic image is received in order to align the three-dimensional model to the new endoscopic image and update a corresponding visualization and/or steps 202-208 can be repeated one or more times to re-register the three-dimensional model with the endoscopic imager in the common reference frame, such as to re-register the three-dimensional model with the endoscopic imager in the common reference frame when the bony anatomy has been moved (e.g., when a leg has been repositioned). Tracking information from the tracking system for the bone position and orientation and the endoscopic imager position and orientation is used to update the three-dimensional model position and orientation to account for bone movement and endoscopic imager movement. In some examples, the alignment process described above may be performed in real-time, enabling a surgeon to view an endoscopic video on which the three-dimensional model is overlaid or otherwise aligned with. The alignment process may be performed for each frame or may be performed after a predetermined number of frames (e.g., every other frame). In examples in which the bone position is not tracked (e.g., no tracking device is affixed to the bone), bone movement may require re-registration, in which case, method 200 may return to step 202 based on a new two-dimensional image generated to capture the bone in its position after the movement of the bone.

In some examples, the position of the bony anatomy may be tracked, and the visualization may be updated to account for movement of the bony anatomy. The bony anatomy may be tracked using an electromagnetic tracking system (e.g., by securing an electromagnetic tracking device to the bony anatomy). The bony anatomy may be tracked using an optical tracking system (e.g., by securing a fiducial marker, such as an ArUco, that is visible outside of the body of the subject to the bony anatomy and tracking the fiducial marker with the optical tracking system). Alternatively or in addition, the bony anatomy may be tracked by one or more IMUs associated with the bony anatomy. In aspects involving the hip, the electromagnetic tracking device, fiducial marker, and/or IMU may be attached to the greater trochanter of the patient. The greater trochanter is easily accessible by a surgeon for implantation, as it is easily located by the surgeon due to its close proximity to the skin. As a result, an electromagnetic tracking device, fiducial marker, and/or IMU may be placed percutaneously and without the assistance of endoscopic visualization. Additionally, a tracking device implanted into the greater trochanter will not block the movement of surgical instruments during a surgical procedure on the hip. Optionally, an additional electromagnetic tracking device, fiducial marker, and/or IMU may be attached to the pelvis of the patient in order to provide additional information about the relative position between the femur and pelvis of the patient.

Tracking the movement of the bony anatomy may help to maintain alignment of the three-dimensional model of the bony anatomy relative to endoscopic video during a surgical procedure. In some examples, the bony anatomy may be repositioned during a surgical procedure. If the movement of the bony anatomy is not tracked, the relationship between the bony anatomy and what is shown in endoscopic video may be unknown when the bony anatomy is repositioned. As a result, the alignment of the three-dimensional model of the bony anatomy relative to the endoscopic video may be incorrect. Tracking movement of the bony anatomy ensures that the three-dimensional model remains in alignment relative to the endoscopic camera (and thus to endoscopic video captured by the endoscopic camera) when the bony anatomy moves. For example, as discussed above, the movement of the endoscopic camera may be tracked by a tracking system or IMU. The movement of the bony anatomy may also be tracked by the tracking system or by another IMU. If the bony anatomy is determined to have moved 1 mm in the +x direction, and the endoscopic camera is determined to have moved 2 mm in the -x direction, then an image processing system may determine that the endoscopic camera has moved a net distance of 1 mm in the -x direction based on the tracked movement of the bony anatomy and endoscopic camera. The alignment of the three-dimensional model relative to the endoscopic video captured by the endoscopic camera may then be updated accordingly to ensure accuracy.

According to some aspects, movement of the bony anatomy may be accounted for using computer vision techniques instead of a tracking system or IMU. Movement of the bony anatomy may be determined by analyzing endoscopic video captured by the endoscopic camera to track movement of pixels (e.g., a set of pixels corresponding to the bony anatomy) across frames. The endoscopic video may be analyzed using one or more computer vision techniques. For example, the endoscopic video can be analyzed using visual simultaneous localization and mapping (vSLAM) techniques, in which a vSLAM algorithm is used to detect one or more features of the bony anatomy and track the one or more features across frames. Alternatively, the endoscopic video can be analyzed using monocular depth estimation, in which one or more trained machine learning models predict depth values for pixels corresponding to the bony anatomy and track changes in the predicted depth values across frames. Alternatively, the endoscopic video can be analyzed using structured light imaging techniques, in which a predetermined light pattern is projected onto the bony anatomy and distortions in the pattern are analyzed to determine information about the bony anatomy. The alignment of the three-dimensional model of the bony anatomy relative to the endoscopic video may be updated accordingly to reflect changes in the position of the bony anatomy.

In some examples, the bony anatomy may not be tracked, and re-registration of the three-dimensional model relative to the endoscopic imager may be required when the bony anatomy has moved. Re-registration may be performed by moving a tracked surgical instrument that is registered in the common reference frame to one or more predetermined positions. The tracked surgical instrument may be touched to the predetermined position(s) to re-establish the position of the bony anatomy within the common reference frame and thus reorient the three-dimensional model of the bony anatomy relative to the endoscopic imager. In some examples, the predetermined position may be a location on the bony anatomy. The predetermined position may be designated by placing one or more objects on or in the bony anatomy, such as one or more pins, beads, pieces of bone wax, or gel drops. Alternatively or additionally, the predetermined position may be designated by forming one or more depressions (e.g., small bur pockets) in the bony anatomy. In some implementations, the predetermined position may be located outside of the patient. For example, the predetermined position may be a predetermined location on a surgical table, an exterior portion of the patient's anatomy, or any other suitable external position within the surgical suite.

Optionally, a warning may be provided if re-registration is required. For example, a warning may be provided via display(s) 116 when the bony anatomy has moved. The warning may include an audio, visual, and/or tactile alert. The warning may include an indication that the bony anatomy has moved and/or an indication that re-registration is required. Movement of the bony anatomy may be detected using one or more sensors associated with the bony anatomy. For example, in aspects involving the hip, an electromagnetic tracking device, an optically tracked object or device (e.g., a reflector, ArUco, QR code, etc.), or one or more IMUs may be attached to the patient's leg or to a piece of surgical equipment associated with the leg, such as a distraction frame. Alternatively, movement of the patient's leg may be detected by analyzing the endoscopic video captured by the endoscopic imager or video of the patient's leg captured by a camera located external to the patient using computer vision techniques.

As noted above, the visualization may be updated as new endoscopic images are received so as to maintain alignment between the three-dimensional model and the endoscopic view. The visualization may be updated by re-aligning the three-dimensional model with newly received endoscopic images. For example, the three-dimensional model may be realigned with new endoscopic images by tracking the position of a feature (e.g., a fiducial marker, a landmark of the bony anatomy, etc.) within the endoscopic images as new endoscopic images are received. A motion tracking algorithm may be used to determine image-to-image motion of the feature. In some aspects, the feature may not be visible in the endoscopic view (e.g., if the bone or the endoscopic imager has moved). If the feature is not visible, alignment between the three-dimensional model and endoscopic images may be maintained by analyzing the endoscopic images to identify the bony anatomy pictured in the endoscopic images. For example, a machine learning model may be used to identify tissue in the new endoscopic images, and the identified tissue may be matched with a bone surface of the three-dimensional model. Other machine learning models that do not identify tissue may also be used to maintain alignment. For example, a machine learning model (e.g., a deep learning model) may use similarity metrics to determine changes in what is shown in the endoscopic images (and, thereby, motion of tissue) without identifying the tissue shown in the endoscopic images. Alternatively, or additionally, the alignment may be maintained using one or more sensors, such as one or more inertial measurement units (IMUs). For instance, IMUs may be attached to the endoscopic imager used to capture the endoscopic images and/or the bony anatomy in order to track their movement. Optionally, one or more machine learning models may be used to evaluate the accuracy of the data provided by the one or more sensors. The one or more machine learning models may be configured to estimate confidence scores pertaining to the sensor data. If the confidence score(s) for a given sensor reading falls below a predetermined threshold, an alert may be provided that indicates that the sensor reading may be inaccurate. For example, the visualization may include a warning indicating that the updated alignment shown in the visualization may be inaccurate due to a potentially inaccurate sensor reading.

As used herein, "maintaining alignment" of the three-dimensional model relative to endoscopic images refers to maintaining the three-dimensional model in a position and orientation relative to endoscopic images such that a projection of the three-dimensional model into two-dimensional space matches the bony anatomy shown in the endoscopic images. An endoscopic imager used to capture the endoscopic images or the bony anatomy captured by the endoscopic imager may move frequently. For example, a patient's leg may be repositioned during a hip surgery, thereby compromising the alignment between the three-dimensional model and the endoscopic images captured by the endoscopic imager. Thus, to accurately convey to the surgeon how the three-dimensional model aligns with what the surgeon is seeing in the endoscopic view, the alignment of the three-dimensional model relative to incoming endoscopic images may be maintained. In some examples, maintaining an alignment of the three-dimensional model relative to endoscopic images includes re-aligning the three-dimensional model with newly received endoscopic images. Re-alignment may be performed in real-time, enabling a surgeon to view an endoscopic video on which the three-dimensional model is overlaid or otherwise aligned with. The alignment process may be performed for each newly received endoscopic image frame or may be performed after a predetermined number of frames (e.g., every other frame, every third frame, every fifth frame, etc.).

An alignment of the three-dimensional model relative to endoscopic images may be maintained by tracking a feature associated with the bony anatomy. Tracking the feature refers to determining how the feature has moved between image frames, and thus how the endoscopic imager has moved relative to the bony anatomy. The feature may be tracked with the endoscopic imager used to capture the endoscopic images. The feature may be a fiducial marker with a predetermined pattern (e.g., an ArUco or QR code), a plurality of small objects (e.g., pins, beads gel drops, or pieces of bone wax), or depressions in the bony anatomy. As long as the feature is visible in the endoscopic view, alignment between the three-dimensional model and endoscopic images may be maintained.

In some examples, maintaining alignment between the three-dimensional model and endoscopic images by tracking the feature may not be possible. For example, the endoscopic imager or the bony anatomy may move such that the feature is no longer visible in the endoscopic view. Optionally, if the feature disappears from the endoscopic view, image processing system 106 may generate and output a notification to a surgeon. The notification may include, for example, a prompt to move the endoscopic imager in a specified direction in order to bring the feature back into view. The prompt may include text-based instructions, arrows indicating a direction in which to move the endoscopic imager, or any other suitable guidance.

To maintain alignment between the three-dimensional model and endoscopic images while the feature is out of view, motion of the endoscopic imager tracked using one or more sensors, such as one or more IMUs, may be used to determine a change in position and/or orientation of the endoscopic imager relative to the three-dimensional model. For example, an IMU may provide data about a change in position and/or orientation of the endoscopic imager between a time at which an image used to generate an initial alignment relative to the three-dimensional model was captured and a time at which a new image was captured. The image processing system 106 may analyze this data to determine a change in position and orientation of the endoscopic imager within a virtual world reference frame in order to determine a new alignment of the three-dimensional model relative to the new endoscopic image.

Maintaining alignment between the three-dimensional model and endoscopic images may include aligning the three-dimensional model and the endoscopic imager used to capture the endoscopic images. As discussed above, the endoscopic imager may be represented in a virtual world reference frame. Posing the endoscopic imager in the virtual world reference frame enables image processing system 106 to update the pose of the imager within the virtual world reference frame using IMU data. The endoscopic imager may be posed in the virtual world reference frame using characteristics of the optics of the endoscopic imager (e.g., focal length). For instance, a perspective of the endoscopic imager can be determined based on the feature shown in an endoscopic image captured by the endoscopic imager, the known geometry of the feature, and one or more characteristics of the optics of the endoscopic imager. This perspective can be combined with the position and orientation of the feature within the virtual world reference frame to determine the position and orientation of the endoscopic imager in the virtual world reference frame. Once the endoscopic imager is posed in the virtual world reference frame, motion data generated by the IMU(s) may be used to update the position and orientation of the endoscopic imager in the virtual world reference frame. The updated position and orientation of the endoscopic imager in the virtual world reference frame may be used to determine an updated alignment of the three-dimensional model relative to a newly received image.

For example, an endoscopic image may be captured at time t₀. The endoscopic image captured at t₀ may be used to determine an initial alignment between the three-dimensional model and the endoscopic image. A new endoscopic image may be captured at time t₁. One or more IMUs associated with the endoscopic imager may record the movement of the endoscopic imager between time t₀ and time t₁. Based on the motion data provided by the IMU(s), image processing system 106 can determine how the endoscopic imager has moved within the virtual world reference frame (and thus relative to the three-dimensional model) between times t₀ and t₁, resulting in an updated alignment between the three-dimensional model and the endoscopic imager. Based on the known characteristics of the optics of the endoscopic imager, the alignment between the three-dimensional model and the endoscopic imager can be transformed into an alignment between the three-dimensional model and the new endoscopic image.

The IMU(s) may be associated with (e.g., attached to or integrated with) the endoscopic imager. The IMU(s) may include one or more sensors such as accelerometers, gyroscopes, and/or magnetometers. The one or more sensors may detect motion of the endoscopic imager and output motion data, such as linear accelerations in three dimensions, rotational velocities in three dimensions, and/or magnetic field measurements in three dimensions. In some examples, the IMU(s) may be calibrated prior to using motion data from the IMU(s) to determine an alignment between the three-dimensional model and the endoscopic imager, for example at the start of an imaging session. An IMU may be calibrated by placing the endoscopic imager with which the IMU is associated in a known position and orientation and providing an input (e.g., a button press) indicating that the IMU can be calibrated. Upon receiving a user input, the readings from the IMU may be saved (e.g., by image processing system 106) as a reference point.

Optionally, calibrating an IMU may include defining an offset associated with a difference in position between the IMU and a reference position on the endoscopic imager. For example, it may be desirable for IMU data to be associated with the tip of the endoscope of the endoscopic imager. An offset between the location of the IMU and the tip of the endoscope may be used to compute a rigid transform between a reference frame centered at the IMU and a reference frame centered at the tip of the endoscope. Different endoscopic imager arrangements (e.g., different camera heads, endoscopes, and/or camera couplers) may have different offsets. Thus, the calibration step may include receiving a user input associated with the endoscopic imager arrangement. The user input may be the offset itself or a selection of a preprogrammed option associated with the imager arrangement. Alternatively, the offset may be calculated using an optical calibration routine (e.g., by generating and analyzing images of a calibration object of known size).

Optionally, motion of the bony anatomy may also be tracked using one or more IMUs, and the corresponding motion data may be used in determining the position and orientation of the three-dimensional model relative to the endoscopic imager. The IMU(s) associated with the bony anatomy may track small movements of the bony anatomy that would otherwise be unaccounted for without the IMU(s)-if the bony anatomy is not tracked by one or more IMUs, the bony anatomy is assumed to be stationary. Thus, measuring small movements of the bony anatomy with the IMU(s) may enable image processing system 106 to determine motion of the endoscopic imager relative to the bony anatomy. Accordingly, using one or more IMU(s) associated with the bony anatomy may result in a more accurate alignment of the three-dimensional model and the endoscopic imager (and thus a more accurate alignment of the three-dimensional model and endoscopic images captured by the endoscopic imager). For example, motion data from the IMU(s) associated with the bony anatomy may be combined with motion data from IMU(s) associated with the endoscopic imager to determine a new alignment of the three-dimensional model relative to the endoscopic imager. The motion data from the IMU(s) associated with the bony anatomy may indicate that the bony anatomy has moved 1 mm in the +x direction, while the motion data from the IMU(s) associated with the endoscopic imager may indicate that the endoscopic imager has moved 2 mm in the -x direction. Using both types of IMU data, image processing system 106 may determine that the endoscopic imager has moved a net distance of 1 mm in the -x direction and may update the alignment of the three-dimensional model relative to the endoscopic imager accordingly. If the bony anatomy did not have associated IMU(s) and was assumed to be stationary, the determination made by image processing system would be incorrect by 1 mm in the -x direction.

Alternatively or in addition, alignment between the three-dimensional model and endoscopic images may be maintained using image analysis techniques. For example, a motion tracking algorithm may be used to process a sequence of image frames and determine relative motion across the frames. The motion tracking algorithm may use optical flow techniques to estimate motion of the endoscopic imager based on the apparent motion of one or more pixels in the sequence of image frames. The motion tracking algorithm may determine frame-to-frame motion between every frame or between a predetermined number of frames (e.g., every other frame, every third frame, etc.). The display of the at least a portion of the three-dimensional model may be updated accordingly. Alternatively, a machine learning model may be used to identify tissue shown in a sequence of endoscopic image frames. For example, a machine learning model may be used to analyze endoscopic images to identify tissue (e.g., by identifying anatomical landmarks). The machine learning model may be trained using a plurality of images of the bone that is being or will be surgically treated and the surrounding tissue. The machine learning model may analyze the endoscopic images to determine the tissue that is shown in the image. Image processing system 106 may match the identified tissue to a corresponding portion of the three-dimensional model and update the displayed three-dimensional model accordingly. In some examples, machine learning models that do not identify tissue may be used to maintain alignment. For example, machine learning models (e.g., deep learning models) may use similarity metrics to determine movement of the endoscopic images relative to the three-dimensional model without identifying anatomical landmarks or specific types of tissue shown in the endoscopic images.

Optionally, the three-dimensional model may be updated to reflect bone that has been removed by the surgeon during the surgical procedure so that the visualization generated using the three-dimensional model reflects the current state of the bone. To update the three-dimensional model, bone removal may be tracked, and information corresponding to the bone removal may be provided to image processing system 106 to update the three-dimensional model accordingly. Bone removal may be tracked in a variety of ways. For example, bone removal may be tracked by tracking a fiducial marker associated with a bone removal instrument with an endoscopic imager. One or more IMUs may also be used for tracking in case the fiducial marker on the bone removal instrument is not visible (e.g., if the fiducial marker moves out of the endoscopic view). The one or more IMUs may also be used even if the fiducial marker is visible in order to provide additional information about motion of the bone removal instrument, endoscopic imager, and/or bony anatomy. This may improve the accuracy of the updated display of the three-dimensional model.

A bone removal instrument may be tracked by detecting the bone removal instrument in the endoscopic images and determining the location of its bone removing feature. For example, a fiducial marker may be associated with (e.g., affixed to or printed on) the bone removal instrument. The fiducial marker may be an optically visible marker that is visible in the endoscopic view. The fiducial marker may be an ArUco, QR code, marking (e.g., RF marking), or any other predetermined pattern. Motion of the bone removal instrument may be monitored by keeping the fiducial in the endoscopic view. When the endoscopic images show that a bone removal portion of the bone removal instrument is removing bone, image processing system 106 may update the three-dimensional model accordingly based on the endoscopic images. In some examples, one or more machine learning (e.g., deep learning) models may be used to identify the location of the bone removal portion of the bone removal instrument in the endoscopic images and determine that the bone removal portion is removing bone.

Alternatively or in addition, the bone removal instrument may be tracked using one or more IMUs. The one or more IMUs may be affixed to or integrated into the bone removal instrument. When a portion of the bone removal instrument used to resect bone is determined to be in a location that is known to correspond to bony anatomy, the three-dimensional model may be updated by removing the portion of the three-dimensional model that corresponds to the overlap between the portion of the bone removal instrument and the three-dimensional model. Using one or more IMUs may be particularly helpful if the bone removal instrument moves out of the endoscopic view. In that case, a fiducial associated with the bone removal instrument can no longer be tracked using the endoscopic imager, and the one or more IMUs may be used to track the bone removal instrument. In some examples, the one or more IMUs associated with the bone removal instrument may be used to supplement tracking of a fiducial associated with the bone removal instrument to improve the accuracy of the updated three-dimensional model.

In some examples, other system components may be tracked using IMUs instead of or in addition to the bone removal instrument. For example, one or more IMUs may be associated with the endoscopic imager. Tracking motion of the endoscopic imager using one or more IMUs enables image processing system 106 to determine the motion of the bone removal instrument relative to the endoscopic imager. This may result in a more accurate determination of bone removal and thus a more accurate updated three-dimensional model. In some examples, one or more IMUs may also be associated with the bony anatomy itself. Tracking the bony anatomy with one or more IMUs enables image processing system 106 to determine whether the patient has moved, which may also result in a more accurate updated three-dimensional model. Tracking the endoscopic imager and/or the bony anatomy with one or more IMUs may also help to maintain alignment of the three-dimensional model to the endoscopic view while bone is being removed.

Optionally, a bone removal instrument may be tracked in a common reference frame by a tracking system (e.g., an electromagnetic tracking system or an optical tracking system) and registered in the same common reference frame as the three-dimensional model. For example, the bone removal instrument may be tracked in the tracking system reference frame, and its position and orientation in the tracking system reference frame may be transformed into the virtual world reference frame using a transformation matrix. Interference between a cutting portion of the instrument and the three-dimensional model in the common reference frame can be used as an indicator of removed bone and the three-dimensional model can be updated by removing the portion that is interfered with by the instrument.

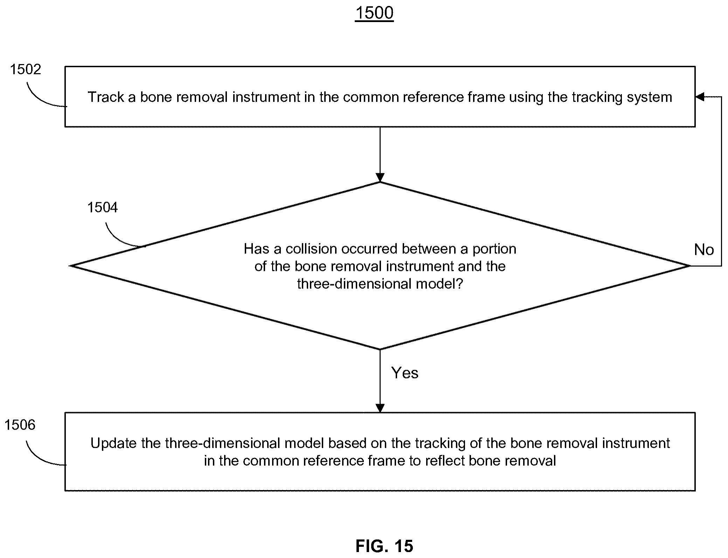

FIG. 15 illustrates an exemplary method 1500 for updating a three-dimensional model to reflect bone removal during a bone removal procedure. Method 1500 may be performed by one or more processors, such as one or more processors of image processing system 106 described above with reference to FIG. 1. Method 1500 may be used along with method 200 (e.g., while performing method 200 or after performing method 200) in order to update the three-dimensional model displayed in step 210.

At step 1502, a bone removal instrument is tracked in the common reference frame using the tracking system. The tracked bone removal instrument may be a bur, a shaver, a drill, or any other surgical instrument for removing bone. The bone removal instrument may be tracked in a common reference frame by a tracking system (e.g., an electromagnetic tracking system or an optical tracking system). The bone removal instrument may be tracked using one or more tracking devices affixed to the bone removal instrument. For instance, an electromagnetic tracking device for tracking by an electromagnetic tracking system may be affixed to the bone removal instrument or a fiducial marker for tracking by a camera tracking system may be affixed to the bone removal instrument. The bone removal instrument may be tracked by a tracking device positioned at a proximal portion of the instrument (e.g., outside of the body) and/or at a distal portion of the instrument (e.g., near the cutting portion of the instrument)

Optionally, the bone removal instrument may be tracked using a plurality of sensors. For example, a first sensor may be positioned at a portion of the bone removal instrument that is located externally of the body of the patient for tracking movement of the main body of the bone removal instrument, and a second sensor may be positioned at a portion of the bone removal instrument that is located within the body of the patient closer to the cutting portion of the instrument to track movement of the cutting portion (which may move relative to the main body of the instrument due to flexing of the instrument under pressure). The second sensor may be a tracking sensor for sensing absolute position and orientation of the distal end of the instrument in the tracking reference frame or may be a sensor used to determine movement of the distal end of the instrument relative to the portion of the instrument tracked by the first sensor. For example, the second sensor may be a strain gauge used to determine an amount of flex of a shaft of the instrument extending from a main body portion of the instrument having the first sensor to the cutting head. Additionally or alternatively, the sensors used to track the bone removal instrument may include one or more IMUs associated with (e.g., affixed to) the bone removal instrument.

Optionally, tracking the bone removal instrument in the common reference frame may include tracking the bone removal instrument in the tracking system reference frame and transforming its position and orientation in the tracking system reference frame into a different common reference frame (e.g., the virtual world reference frame). For example, the position and orientation of the bone removal instrument may be transformed into the virtual world reference frame by multiplying the coordinates of the bone removal instrument in the tracking system reference frame by a transformation matrix configured to convert tracking system reference frame measurements into virtual world reference frame measurements.

At step 1504, a determination is made whether a collision has occurred between a portion of the bone removal instrument (e.g., the cutting head of a bur tool or a metal shroud behind the cutting head of the bur tool) and the three-dimensional model in the common reference frame. The determination may be made based on whether a collision has occurred between the portion of the bone removal instrument and the three-dimensional model-i.e., based on whether the portion of the bone removal instrument and the three-dimensional model occupy the same space in the common reference frame. If the determination is that a collision has not occurred, the method may return to step 1502 to continue to track the bone removal instrument.

Optionally, the determination of whether a collision has occurred may be made based on the position of a non-bone removing portion of the bone removal instrument relative to the bone. For example, after bone has been removed, a user may be prompted (e.g., image processing system 106 may display a prompt on display 116) to touch the newly created bone surface with a non-bone removing portion (e.g., a metal shroud on the back side of the bur ball) of the bone removal instrument and the position of the non-bone removing portion of the bone removal instrument may be tracked to determine if and where there is a collision with the three-dimensional model.

If the decision at step 1504 is that a collision between the bone removal instrument and the three-dimensional model has occurred, at step 1506, the three-dimensional model is updated based on the tracking of the bone removal instrument in the common reference frame to reflect bone removal. Bone corresponding to an area presumed to have been resected may be removed from the model to reflect the removal of bone. Optionally, a heat map displayed on the three-dimensional model may also be updated to reflect the removal of bone. For example, a region of the heat map corresponding to bone that has recently been resected may be removed. Additionally, or alternatively, a region of the heat map corresponding to bone that has recently been resected may be highlighted using a predetermined color to indicate that the bone in that location was recently removed. In some examples, different color indicators may be used in the heat map to indicate the recency of bone removal (e.g., a portion of the heat map corresponding to bone that was removed one minute ago may be brighter or darker than a portion of the heat map corresponding to bone that was removed five minutes ago). The color indicators may change at a fixed or variable rate. In implementations where the color indicators change at a fixed rate, the color of a recently removed region of the heat map may fade or change continuously with time or may change color or brightness according to a step function. Additionally, or alternatively, the color of a region of the heat map corresponding to a region of removed bone may be updated to reflect how much more bone in that region should be removed (e.g., a yellow portion of a heat map may change to green and then to blue as the depth of bone removal increases.

Optionally, one or more interpolation and/or smoothing operations may be performed, such that resected areas of bone in the three-dimensional model are shaped like valleys with relatively smooth edges rather than holes with jagged or abrupt edges. The updated three-dimensional model may be shown to the surgeon while the bone removal instrument is removing bone so that the surgeon can see where and how much bone has been removed and still needs to be removed in real-time. Real-time updates to the three-dimensional model may occur frequently enough that the surgeon cannot perceive a difference between the bony anatomy shown in the three-dimensional model and the endoscopic view. In some examples, the three-dimensional model may be updated at a rate of at least about 5 frames per second, at least about 10 frames per second, at least about 20 frames per second, at least about 30 frames per second, at least about 40 frames per second, or at least about 50 frames per second. In some examples, the three-dimensional model may be updated at a rate of less than about 60 frames per second, less than about 50 frames per second, less than about 40 frames per second, less than about 30 frames per second, less than about 20 frames per second, or less than about 10 frames per second. In some examples, the three-dimensional model may be updated at least once every two seconds, at least once per second, at least two times per second, at least three times per second, at least five times per second, at least ten times per second, or at least 20 times per second, or at least 30 times per second. In some examples, the three-dimensional model may be updated at a frame rate of endoscopic video or at a fraction of the frame rate of the endoscopic video, such as at least half the frame rate, at least a third of the frame rate, at least a quarter of the frame rate, at least a fifth of the frame rate, or at least a tenth of the frame rate.

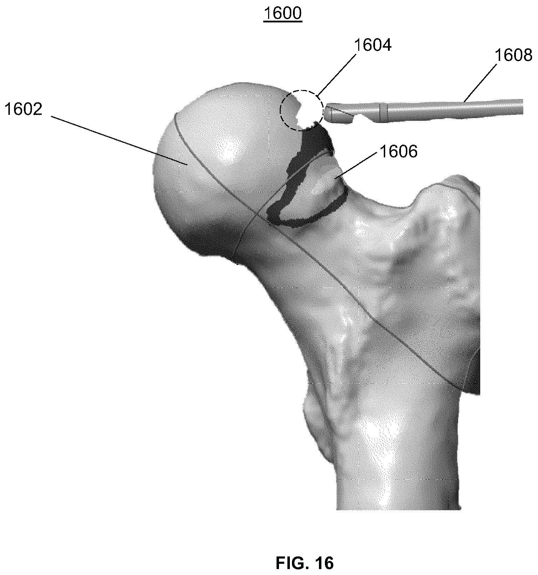

FIG. 16 illustrates a visualization 1600 that includes a three-dimensional model that has been updated to reflect bone removed during a bone removal procedure. FIG. 16 may be displayed to a surgeon to guide a bone removal procedure, for example via display 116 described above with reference to FIG. 1. As shown in FIG. 16, a three-dimensional model 1602 may include one or more regions 1604 corresponding to bone that has been removed. The representation of planned bone removal 1606 (e.g., a color-coded heat map as illustrated in FIG. 16) may be updated to reflect bone removed in the planned bone removal area such that a user can simultaneously visualize bone that has been removed and bone that still needs to be removed. Optionally, a representation of a bone removal instrument 1608 may be included in visualization 1600. Including a representation of bone removal instrument 1608 may enable a user to visualize where the bone removal instrument is relative to the region(s) 1604 of bone that have been removed and the region(s) 1606 of bone that still need to be removed.

The methods disclosed herein include determining a position and orientation of a three-dimensional model of bony anatomy relative to an endoscopic image and displaying the three-dimensional model based on the determined position and orientation in order to guide a bone removal procedure. In some examples, the three-dimensional model may be pre-generated. Alternatively, the three-dimensional model may be generated intraoperatively. For example, the three-dimensional model may be generated intraoperatively based on two-dimensional images of the bony anatomy. Intraoperatively generating a three-dimensional model may be advantageous when a pre-generated three-dimensional model is unavailable (e.g., if a patient is unavailable for a pre-operative imaging session to capture images for generating a three-dimensional model). Intraoperatively generating a three-dimensional model may also create a highly accurate reflection of the bony anatomy as it exists at the time of the surgical procedure. FIG. 18 illustrates a method 1800 for guiding bone removal that includes generating a three-dimensional model of bony anatomy. Method 1800 may be performed by one or more processors, such as one or more processors of image processing system 106 described above with reference to FIG. 1. Method 1800 includes generating a three-dimensional model from two-dimensional images of bony anatomy and a radiopaque object and displaying a visualization based on the three-dimensional model. The visualization may be updated throughout a bone removal procedure in order to reflect bone removal.

At step 1802, a first two-dimensional image of bony anatomy and a radiopaque object captured from a first perspective and a second two-dimensional image of the bony anatomy and the radiopaque object captured from a second perspective are received. The two-dimensional images may be captured using a two-dimensional imaging modality, such as two-dimensional imaging system 108, and received at a computing system, such as image processing system 106. The two-dimensional images may be captured pre-operatively or intraoperatively. It should be understood that in some examples, more than two two-dimensional images may be received. For example, three two-dimensional images captured from three different perspectives may be received, four two-dimensional images captured from four different perspectives may be received, five two-dimensional images captured from five different perspectives may be received, and so on.

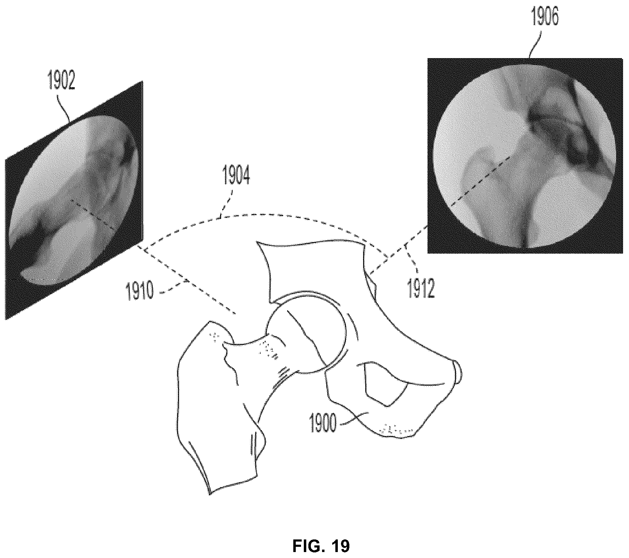

The first perspective from which the first two-dimensional image is captured is different from the second perspective from which the second two-dimensional image is captured. FIG. 19 illustrates an example of two-dimensional X-ray images of a hip joint 1900 captured from two different imaging perspectives. Image 1902 captures the patient's hip joint 1900 from a first perspective 1910 and image 1906 captures the patient's hip joint 1900 from a second perspective 1912. The first perspective 1910 is at a predetermined angle 1904 relative to the second perspective 1912. The difference in perspectives may be achieved by moving the imaging system used to capture the first two-dimensional image. For example, the first two-dimensional image may be captured using a C-arm imager and may provide an anterior-posterior (AP) view of the bony anatomy and the radiopaque object. The second two-dimensional image may be captured using the same C-arm imager and may provide a lateral or oblique perspective of the bony anatomy and the radiopaque object. The second two-dimensional image may be captured by rotating the C-arm imager between 45-90 degrees. Different angles may be used according to other aspects. Alternatively or additionally, the difference in perspectives of the images may be achieved by moving the bony anatomy itself.

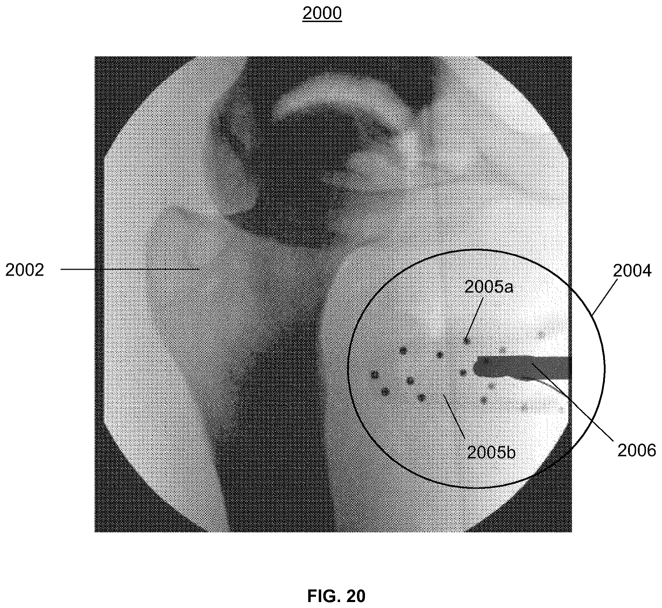

The first and second two-dimensional images received at step 1802 each include the bony anatomy and a radiopaque object. An example of a two-dimensional image that includes bony anatomy and a radiopaque object is illustrated in FIG. 20. Two-dimensional image 2000 is a fluoroscopic image that includes bony anatomy 2002 and radiopaque object 2004. Radiopaque object 2004 may be positioned adjacent to but not touching bony anatomy 2002 or may be positioned against bony anatomy 2002. Radiopaque object 2004 may include a plurality of radiopaque features 2005a attached to a non-radiopaque base 2005b. The plurality of radiopaque features 2005a may be radiopaque pins, beads, bars, or other radiopaque features. The plurality of radiopaque features 2005a may be a combination of different radiopaque features (e.g., pins and beads, pins and bars, beads and bars, etc.). The non-radiopaque base 2005b may be plastic, rubber, or any other non-radiopaque material. Alternatively, radiopaque object 2004 may be a single piece of radiopaque material.

Radiopaque object 2004 may be configured to be attached to various objects. For example, radiopaque object 2004 may be removably attachable to a surgical instrument 2006. Radiopaque object 2004 may be attachable to a distal end of surgical instrument 2006 or to any other portion of surgical instrument 2006. Surgical instrument 2006 may be a bone removal instrument (e.g., a bur or shaver), a pointer tool, or any other surgical instrument. Additionally, or alternatively, radiopaque object 2004 may be removably attachable to an endoscopic imager.

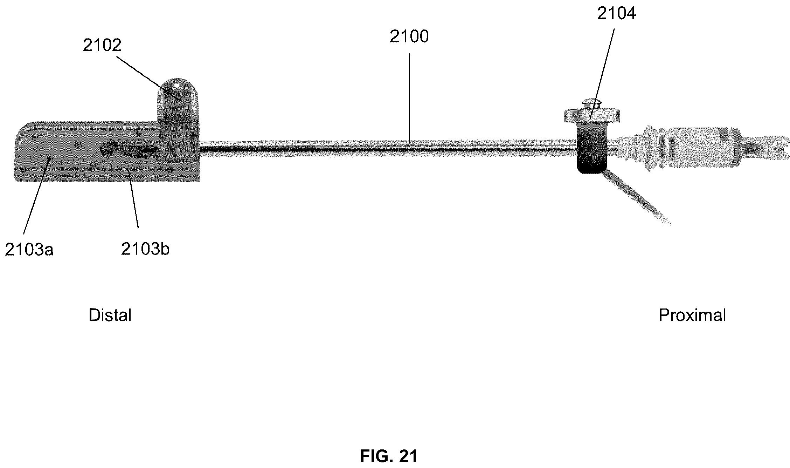

Returning to FIG. 18, at step 1804, an alignment between the first two-dimensional image and the second two-dimensional image is determined based on predetermined characteristics of the radiopaque object. The predetermined characteristics of the radiopaque object may be a predetermined arrangement of radiopaque features of the radiopaque object or a known geometry of the radiopaque object. For example, FIG. 21 illustrates a radiopaque object 2102 attached to a surgical instrument 2100. The radiopaque object 2102 includes a non-radiopaque base 2103b that clips onto the distal end of surgical instrument 2100 and a plurality of radiopaque beads 2103a interspersed throughout non-radiopaque base 2003b. The predetermined characteristics of radiopaque object 2102 may include the arrangement of the plurality of radiopaque beads 2103a in three-dimensional space.

The predetermined characteristics of the radiopaque object may be represented in a three-dimensional model of the radiopaque object. For example, a three-dimensional model of radiopaque object 2102 may indicate the arrangement of radiopaque beads 2103a in three-dimensional space. The alignment between the first and second two-dimensional images may be determined by determining what position and orientation of the three-dimensional model of the radiopaque object would result in the arrangement of the radiopaque object in the two-dimensional image. Continuing with the example of FIG. 21, the spatial arrangement of the radiopaque beads 2103a represented in the three-dimensional model of radiopaque object 2102 may be compared to the spatial arrangement of the radiopaque features captured in two-dimensional images to determine the alignment between the three-dimensional model and each of the first and second two-dimensional images. The alignment between the first and second two-dimensional images may then be determined based on the alignment of each two-dimensional image with the three-dimensional model.

An alignment between a two-dimensional image and the three-dimensional model of the radiopaque object may be determined using one or more algorithms. For instance, a nonlimiting example of an algorithm that can be used is the RANdom SAmple Consesus (RANSAC) algorithm. The RANSAC algorithm may randomly select a set of a particular number (e.g., three) of radiopaque features from the first two-dimensional image and a set of the same number of radiopaque features from the three-dimensional model of the radiopaque object. The position and orientation of the three-dimensional model relative to the first two-dimensional image may be manipulated until a projection of the selected set of radiopaque features from the three-dimensional model aligns with the selected set of radiopaque features from the first two-dimensional image. The system may determine whether a projection of the remaining radiopaque features of the three-dimensional model aligns with the remaining radiopaque features from the first two-dimensional image. If the remaining radiopaque features align, then the three-dimensional model of the radiopaque object is in alignment with the first two-dimensional image. If the remaining radiopaque features do not align, the alignment process may be repeated (e.g., hundreds or thousands of times) until an alignment between the three-dimensional model of the radiopaque object and the first two-dimensional image is found. This process may be repeated to determine an alignment between the second two-dimensional image and the three-dimensional model of the radiopaque object.

The alignment between the first two-dimensional image and the second two-dimensional image may be determined based on the alignment between the first two-dimensional image and the three-dimensional model of the radiopaque object and the alignment between the second two-dimensional image and the three-dimensional model of the radiopaque object. Alternatively or additionally, the alignment between the first and second two-dimensional images may be determined using edge detection techniques, by determining the difference between imaging angles of the imaging system used to capture the first and second two-dimensional images, and/or by using any other suitable techniques.

At step 1806, three-dimensional image data is generated by back-projecting the first and second two-dimensional images in three-dimensional space in accordance with the determined alignment. Back-projecting the first and second two-dimensional images transforms the images from two-dimensional space to three-dimensional space. Back-projecting the first and second two-dimensional images in three-dimensional space may include performing various transformations to account for properties of the imaging system used to capture the two-dimensional images and/or distortions of the bony anatomy shown in the two-dimensional images. For example, a two-dimensional image may be changed in scale as it is back-projected. Other transformations may be used to account for skew, distortion, and/or perspective effects.

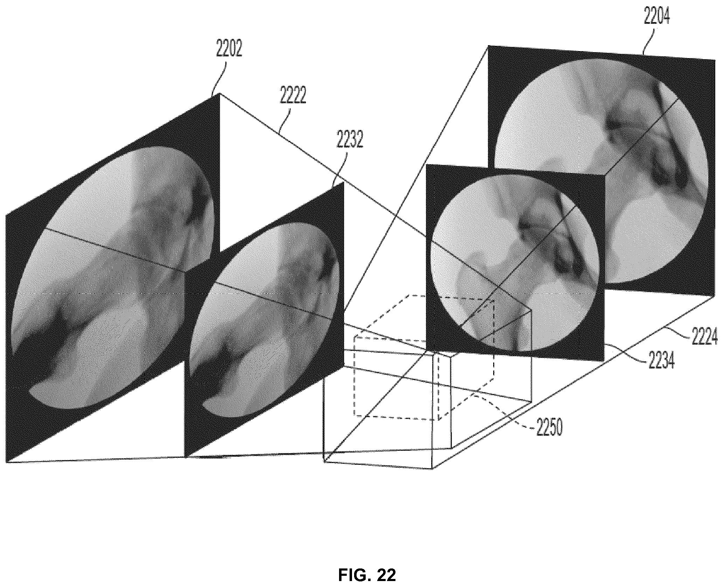

Back-projecting the first and second two-dimensional images into three-dimensional space is illustrated conceptually in FIG. 22. The first two-dimensional image 2202 and the second two-dimensional image 2204 are swept in the back-projection dimension. According to some examples, this sweeping may result in a "pyramid stump" 2222, 2224 in which pixel values of the two-dimensional images are replicated across the back-projection dimension based on the differences in perspectives of the images and various transformations discussed above. The pyramid stump shape may be appropriate, for example, when modeling an X-ray source generating the images 2202, 2204 as a pinhole camera. The X-ray source may be modeled in other ways, according to other aspects. Simplistic examples of a replication 2232 for the first two-dimensional image 2202 and a replication 2234 for the second two-dimensional image 2204 are provided in FIG. 22 to further illustrate the concept. The shape and position of each pyramid stump 2222, 2224 is based on the differences in perspectives of the images and various transformations discussed above.

A three-dimensional voxel grid 2250 is placed in the region in which the pyramid stumps 2222, 2224 of the two-dimensional images overlap. The voxel size of voxel grid 2250 may be chosen to match the desired resolution of the three-dimensional model (e.g., 1 mm). The overall size of the voxel grid 2250 can be any suitable size, and in some examples, may be selected based on the size of the area to be modeled (e.g., 128x128x128 at 1 mm voxel size for a region corresponding to a cube with 128 mm edge length). The values of the voxels over grid 2250 are assigned by sampling the images 2202, 2204 at the location of the projection of the respective voxel center, yielding one value per input image (e.g., two values for each voxel when using two input images). Thus, each voxel has a value for a respective image that is determined from the pixel(s) of the image that are at the corresponding location in the image. A voxel could have the same value as a corresponding pixel, such as where a voxel directly maps to a single pixel, or could have a value that is a function of multiple pixel values, such as an average of surrounding pixel values. According to various examples, a pixel value could contribute to the values of multiple voxels, such as where voxel resolution is finer than pixel resolution.

Back-projecting the first and second two-dimensional images in three-dimensional space in accordance with the determined alignment may result in the assignment of values to a set of voxels. For example, each voxel may be assigned a value corresponding to the first two-dimensional image and a value corresponding to the second two-dimensional image. The values may correspond to pixel values of the projection of the voxel center onto the respective two-dimensional images. Alternatively, each voxel may be assigned a single value corresponding to both two-dimensional images.

Returning to FIG. 18, at step 1808, a three-dimensional model of the bony anatomy is generated based on the three-dimensional image data. The three-dimensional model of the bony anatomy may be generated using at least one machine learning model. The at least one machine learning model may be trained using imaging data generated via a three-dimensional imaging modality (e.g., CT or MRI). For example, the at least one machine learning model may be configured to transform the three-dimensional image data into a three-dimensional model of the bony anatomy using an iso-surface extraction technique (e.g., a Marching Cubes technique or Marching Tetrahedra technique) to search the three-dimensional image data (e.g., the set of voxels) for the surface of the bony anatomy and generate a mesh associated with the surface.

According to other aspects, the at least one machine learning model used to generate the three-dimensional model may use other techniques instead of or in addition to iso-surface extraction techniques to generate the three-dimensional model of the bony anatomy. For example, the at least one machine learning model may use one or more computer vision techniques to generate the three-dimensional model from two-dimensional images. Suitable computer vision techniques may include monocular depth estimation, visual simultaneous localization and mapping (vSLAM), and structured light imaging, which may be used alone or in combination. Monocular depth estimation uses depth information obtained by analyzing two-dimensional images to estimate a three-dimensional model. vSLAM includes analyzing a series of endoscopic video frames to identify features and track their motion across video frames. The tracked motion of the features can then be used to triangulate three-dimensional points, which can be used to build a three-dimensional model. Structured light imaging includes projecting a predetermined light pattern onto a surface of bony anatomy and analyzing the distortions in the pattern to determine shape and depth information about the bony anatomy. Using one or more computer vision techniques instead of or in addition to other three-dimensional model generation techniques (e.g., iso-surface extraction techniques) may improve the accuracy of the three-dimensional model.

According to some aspects, the at least one machine learning model used to generate the three-dimensional model may be trained using three-dimensional imaging data, such as CT scan data. CT scan data may be modified to increase its resolution for use as training data to improve the resolution of the three-dimensional model generated by the machine learning model. In some examples, the CT scan data may be upsampled, such as by using a supersampling technique. The machine learning model may be trained to generate a three-dimensional model based on the upsampled CT imaging data, which may result in a more accurate three-dimensional model than would otherwise be generated using unaltered CT imaging data due to the higher resolution of the training data. Alternatively, the machine learning model may be trained on CT imaging data that has not been altered, and the three-dimensional model generated by the machine learning model may be adjusted based on X-ray images to achieve greater accuracy. For example, the machine learning model may generate a three-dimensional model of bony anatomy based on X-ray images that were input into the machine learning model. The same X-ray images may be segmented to isolate the bony anatomy modeled in the three-dimensional model. A projection of the three-dimensional model of the bony anatomy into two-dimensional space may then be compared to the segmented X-ray images. The three-dimensional model may then be adjusted based on deviations of the projection of the three-dimensional model from the segmented X-ray images. Adjusting the three-dimensional model based on the higher resolution X-ray images may result in a more accurate three-dimensional model.

Optionally, at step 1810, a resection plan is generated based on the three-dimensional model of the bony anatomy. The resection plan may indicate portions of the bony anatomy to be resected during a bone removal procedure and may be displayed with the three-dimensional model of the bony anatomy (e.g., as an overlay on the three-dimensional model). The resection plan may include the depth of bone to be resected from various areas of the bony anatomy. For example, different depths may be represented by different colors or patterns. The resection plan may be determined by comparing the three-dimensional model of the bony anatomy generated at step 1808 to a predetermined model or shape. For example, the generated three-dimensional model of the bony anatomy may be compared to a statistical shape model of the bony anatomy, and the resection plan may be generated by identifying deviations of the generated three-dimensional model from the statistical shape model. The generated three-dimensional model may alternatively be compared to a shape approximating the target morphology of the bony anatomy (e.g., if the three-dimensional model of the bony anatomy models a femoral head, the three-dimensional model may be compared to a sphere, since the target morphology of a femoral head is roughly spherical). Alternatively, the resection plan may be generated by comparing aspects of the generated three-dimensional model to target values. For instance, the alpha angle of the generated three-dimensional model may be compared to a target alpha angle, and the resection plan may be generated based on the deviation of the current alpha angle from the target alpha angle.

At step 1812, a visualization based on the three-dimensional model of the bony anatomy is displayed. The visualization may include at least a portion of the three-dimensional model of the bony anatomy. An indication of planned bone removal, such as a heat map, may be overlaid on the three-dimensional model. According to an aspect, the three-dimensional model (or portion thereof) may be displayed in association with endoscopic video. For example, the three-dimensional model may be displayed as an overlay on the endoscopic video, as illustrated in FIGS. 12A-12B, or the three-dimensional model may be displayed adjacent to the endoscopic video, as illustrated in FIGS. 13 and 23. The three-dimensional model may also be displayed in a simulated endoscopic video, as illustrated in FIGS. 14A-B. Alternatively, the three-dimensional model (or portion thereof) may be displayed without any association with the endoscopic video. For example, the three-dimensional model may be displayed on a separate display from the endoscopic video or may be displayed without showing the endoscopic video at all.

In examples where the three-dimensional model is displayed in association with the endoscopic video, the three-dimensional model of the bony anatomy may be displayed based on an alignment of the three-dimensional model of the bony anatomy and the endoscopic video. An alignment between the three-dimensional model of the bony anatomy and the endoscopic video may be determined based on the radiopaque object and a tracking system.

According to an aspect, an alignment between the three-dimensional model of the bony anatomy and the endoscopic video may be determined based on a radiopaque object that is removably attached to a surgical instrument, such as a bone removal instrument (e.g., a bur or shaver tool), a pointer tool, or any other surgical instrument. FIG. 21 illustrates an example of a radiopaque object 2102 attached to a surgical instrument 2100. Radiopaque object 2102 includes a plurality of radiopaque features 2103a disposed on a non-radiopaque base 2103b. Alternatively, radiopaque object may be a piece of radiopaque material. Radiopaque object 2102 may be clamped around, adhered to, or otherwise attached to the distal end of surgical instrument 2100.

A tracking device 2104 is associated with surgical instrument 2100. As shown in FIG. 21, tracking device 2104 may be secured to a proximal end of surgical instrument 2100. Tracking device 2104 may be used to track the movement of surgical instrument 2100 relative to a tracking system. Tracking device 2104 may be an electromagnetic tracking device, and the tracking system may be an electromagnetic tracking system. Alternatively, tracking device 2104 may be a light emitter (e.g., LED), reflective article (e.g., light reflector), and/or fiducial marker (e.g., an ArUco, QR code, or other visual marker), and the tracking system may be an optical tracking system.

The three-dimensional model may be registered with respect to the tracking system based on tracking device 2104. Radiopaque object 2102 and tracking device 2104 are both associated with surgical instrument 2100. A position and orientation of radiopaque object 2102 relative to tracking device 2104 may therefore be determined (e.g., based on known dimensions of surgical instrument 2100). The position and orientation of radiopaque object 2102 relative to the three-dimensional model may also be determined, since radiopaque object 2102 is used to generate the three-dimensional model. Accordingly, the position and orientation of three-dimensional model relative to tracking device 2104 (and thus relative to the tracking system) may be determined, and the three-dimensional model may be registered with respect to the tracking system. Since surgical instrument 2100 and the three-dimensional model are both registered with respect to the tracking system, the three-dimensional model or display thereof can be updated to reflect removal of bone during a bone removal procedure based on tracking movement of surgical instrument 2100 relative to the tracking system. For example, the three-dimensional model of the bony anatomy may include an indication of planned bone removal (e.g., a heat map), and the indication of planned bone removal may be updated based on tracking movement of surgical instrument 2100 relative to the tracking system.

An endoscopic imager may also be registered with respect to the tracking system. A tracking device similar to tracking device 2104 may be associated with the endoscopic imager. The tracking device may be an electromagnetic tracking device that can be tracked by an electromagnetic tracking system or a light emitter, light reflector, and/or fiducial marker that can be tracked by an optical tracking system. The tracking device may be attached to the endoscopic imager. By tracking the endoscopic imager based on the tracking device, the endoscopic imager may be registered with respect to the tracking system. Because the three-dimensional model is also registered with respect to the tracking system, the alignment between the three-dimensional model and the endoscopic imager may be determined. Accordingly, a visualization that is based on the three-dimensional model may be displayed based on the determined alignment. For example, the three-dimensional model may be displayed as an overlay on the endoscopic video (e.g., as shown in FIGS. 12A-12B) or adjacent to the endoscopic video (e.g., as shown in FIGS. 13 and 23). Alternatively, a simulated endoscopic image based on the portion of the three-dimensional model corresponding to the endoscopic video may be displayed (e.g., as shown in FIGS. 14A-B) with or without the actual endoscopic video.

Optionally, the bony anatomy may also be registered with respect to the tracking system. A tracking device similar to tracking device 2004 may be associated with the bony anatomy. The tracking device may be an electromagnetic tracking device that can be tracked by an electromagnetic tracking system or a light emitter, light reflector, and/or fiducial marker that can be tracked by an optical tracking system. The tracking device may be implanted in or otherwise attached to the bony anatomy. Movement of the bony anatomy may be tracked relative to the tracking system based on the tracking device associated with the bony anatomy. Tracking movement of the bony anatomy relative to the tracking system may enable more accurate tracking of bone removal.

In some examples, an alignment between the three-dimensional model of the bony anatomy and the endoscopic video may be determined based on a radiopaque object that is removably attached to an endoscopic imager, rather than to a surgical instrument. The radiopaque object may be clamped to, adhered to, or otherwise attached to the endoscopic imager. A tracking device, such as an electromagnetic tracking device trackable by an electromagnetic tracking system or a light emitter, light reflector, and/or fiducial marker trackable by an optical tracking system, is also associated with (e.g., attached to) the endoscopic imager. The endoscopic imager may be tracked with respect to a tracking system based on the tracking device associated with the endoscopic imager. The endoscopic imager may therefore be registered with respect to the tracking system.

The three-dimensional model of bony anatomy may also be registered with respect to the tracking system based on the tracking device associated with the endoscopic imager. A position and orientation of the radiopaque object relative to the tracking device associated with the endoscopic imager may be determined (e.g., based on known dimensions of the endoscopic imager). The position and orientation of the radiopaque object relative to the three-dimensional model of bony anatomy may also be determined, since the endoscopic imager with the radiopaque object is captured in the two-dimensional images used to generate the three-dimensional model. Accordingly, the position and orientation of three-dimensional model relative to the tracking device associated with the endoscopic imager, and thus relative to the tracking system, may be determined, and the three-dimensional model may be registered with respect to the tracking system.

Because the three-dimensional model and the endoscopic imager are both registered with respect to the tracking system, the alignment between the three-dimensional model and the endoscopic imager may be determined. The three-dimensional model may be displayed based on the determined alignment. The three-dimensional model may be displayed in association with endoscopic video captured by the endoscopic imager. For example, the three-dimensional model may be displayed as an overlay on the endoscopic video (e.g., as shown in FIG. 12) or adjacent to the endoscopic video (e.g., as shown in FIGS. 13 and 23). Alternatively, a simulated endoscopic image based on the portion of the three-dimensional model corresponding to the endoscopic video may be displayed (e.g., as shown in FIGS. 14A-B) with or without the actual endoscopic video.

A surgical instrument (e.g., a bone removal instrument) may also be registered with respect to the tracking system. A tracking device (e.g., an electromagnetic tracking device that can be tracked by an electromagnetic tracking system or a light emitter, light reflector, and/or fiducial marker that can be tracked by an optical tracking system) may be associated with the surgical instrument. For example, the tracking device may be attached to the surgical instrument. By tracking the surgical instrument based on the tracking device associated with the surgical instrument, the surgical instrument may be registered with respect to the tracking system. Because the three-dimensional model is also registered with respect to the tracking system, the alignment between the three-dimensional model and the surgical instrument may be determined. According to an aspect, the surgical instrument may be a bone removal instrument, and the three-dimensional model or display thereof may be updated to reflect removal of bone during a bone removal procedure based on tracking movement of the bone removal instrument relative to the tracking system. For example, the three-dimensional model of the bony anatomy may include an indication of planned bone removal (e.g., a heat map), and the indication of planned bone removal may be updated based on tracking movement of the bone removal instrument relative to the tracking system.

Optionally, the bony anatomy itself may also be registered with respect to the tracking system. A tracking device (e.g., an electromagnetic tracking device that can be tracked by an electromagnetic tracking system or a light emitter, light reflector, and/or fiducial marker that can be tracked by an optical tracking system) may be associated with the bony anatomy. For example, the tracking device may be implanted in or otherwise attached to the bony anatomy. Movement of the bony anatomy may be tracked relative to the tracking system based on the tracking device associated with the bony anatomy. Tracking movement of the bony anatomy relative to the tracking system may enable more accurate tracking of bone removal.

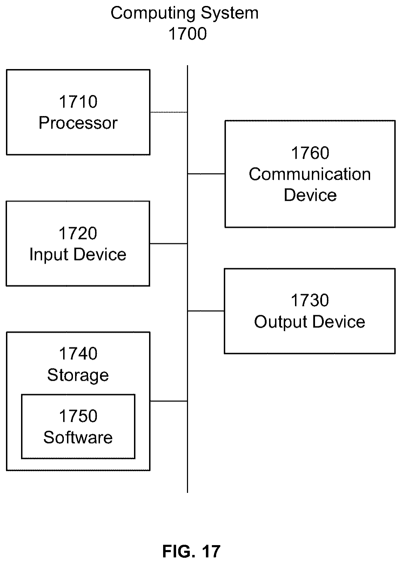

FIG. 17 illustrates an example of a computing system 1700 that may be used in any one of the systems described herein, such as in image processing system 106 of FIG. 1, controller 402 of FIG. 4, or controller 904 of FIG. 9. System 1700 can be a computer connected to a network. System 1700 can be a client computer or a server. As shown in FIG. 17, system 1700 can be any suitable type of microprocessor-based system, such as a personal computer, workstation, server, or handheld computing device (portable electronic device) such as a phone or tablet. The system can include, for example, one or more of a processor 1710, input device 1720, output device 1730, storage 1740, and communication device 1760. Input device 1720 and output device 1730 can generally correspond to those described above and can either be connectable or integrated with the computer.

Input device 1720 can be any suitable device that provides input, such as a touch screen, keyboard or keypad, mouse, gesture recognition component of a virtual/augmented reality system, or voice-recognition device. Output device 1730 can be or include any suitable device that provides output, such as a display, touch screen, haptics device, virtual/augmented reality display, or speaker.

Storage 1740 can be any suitable device that provides storage, such as an electrical, magnetic, or optical memory, including a RAM, cache, hard drive, removable storage disk, or other non-transitory computer readable medium. Communication device 1760 can include any suitable device capable of transmitting and receiving signals over a network, such as a network interface chip or device. The components of the computer can be connected in any suitable manner, such as via a physical bus or wirelessly.

Software 1750, which can be stored in storage 1740 and executed by processor 1710, can include, for example, the programming that embodies the functionality of the present disclosure (e.g., as embodied in the devices as described above). For example, software 1750 can include one or more programs for performing one or more of the steps of method 200, method 600, method 700, method 1000, method 1500, and/or method 1800.

Software 1750 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 1740, that can contain or store programming for use by or in connection with an instruction execution system, apparatus, or device.

Software 1750 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate, or transport programming for use by or in connection with an instruction execution system, apparatus, or device. The transport readable medium can include, but is not limited to, an electronic, magnetic, optical, electromagnetic, or infrared wired or wireless propagation medium.

System 1700 may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T3 lines, cable networks, DSL, or telephone lines.

System 1700 can implement any operating system suitable for operating on the network. Software 1750 can be written in any suitable programming language, such as C, C++, Java, or Python. In various aspects, application software embodying the functionality of the present disclosure can be deployed in different configurations, such as in a client/server arrangement or through a Web browser as a Web-based application or Web service, for example.

The foregoing description, for the purpose of explanation, has been described with reference to specific aspects. However, the illustrative discussions above are not intended to be exhaustive or to limit the invention to the precise forms disclosed. Many modifications and variations are possible in view of the above teachings. The aspects were chosen and described in order to best explain the principles of the techniques and their practical applications. Others skilled in the art are thereby enabled to best utilize the techniques and various aspects with various modifications as are suited to the particular use contemplated.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims. Finally, the entire disclosure of any patents and publications referred to in this application are hereby incorporated herein by reference.

## Claims

1. A system for guiding bone removal comprising one or more processors and memory storing one or more programs for execution by the one or more processors, the one or more programs including instructions that, when executed by the one or more processors, cause the system to:
receive at least one two-dimensional image of bony anatomy captured by a two-dimensional imaging system and at least one endoscopic image of the bony anatomy captured by an endoscopic imager;
register a three-dimensional model of the bony anatomy with the endoscopic imager in a common reference frame using the at least one two-dimensional image and a tracking system;
determine a position and orientation of the three-dimensional model relative to the at least one endoscopic image based on the registration of the three-dimensional model with the endoscopic imager in the common reference frame; and
display at least a portion of the three-dimensional model based on the determined position and orientation of the three-dimensional model relative to the at least one endoscopic image.

2. The system of claim 1, wherein a device tracked by the tracking system is captured in the at least one two-dimensional image of the bony anatomy and registering the three-dimensional model of the bony anatomy with the endoscopic imager in the common reference frame comprises analyzing the at least one two-dimensional image to determine a position and orientation of the tracked device with respect to the at least one two-dimensional image.

3. The system of claim 2, wherein registering the three-dimensional model of the bony anatomy with the endoscopic imager in the common reference frame comprises analyzing the at least one two-dimensional image to register the three-dimensional model relative to the at least one two-dimensional image, and transforming the registration of the three-dimensional model relative to the at least one two-dimensional image to the common reference frame based on the position and orientation of the tracked device in the at least one two-dimensional image and a position and orientation of the tracked device in the common reference frame.

4. The system of claim 2 or 3, wherein the device tracked by the tracking system is secured to bone.

5. The system of any one of claims 2-4, wherein the device tracked by the tracking system comprises a surgical instrument.

6. The system of any one of claims 1-5, wherein the tracking system comprises an electromagnetic (EM) tracking system, and a device secured to the bony anatomy comprises an EM tracking device.

7. The system of claim 6, wherein the one or more programs further include instructions that, when executed by the one or more processors, cause the system to: track movement of the bony anatomy via the EM tracking device and display the at least a portion of the three-dimensional model in an updated position and/or orientation based on the movement of the bony anatomy.

8. The system of any one of claims 1-7, wherein determining the position and orientation of the three-dimensional model relative to the at least one endoscopic image comprises analyzing the at least one endoscopic image to determine at least one value associated with a depth of the bony anatomy relative to the endoscopic imager.

9. The system of any one of claims 1-8, wherein displaying the at least a portion of the three-dimensional model comprises displaying an overlay of the at least a portion of the three-dimensional model on the at least one endoscopic image.

10. The system of claim 9, wherein the one or more programs further include instructions that, when executed by the one or more processors, cause the system to: identify a surgical instrument in the at least one endoscopic image, wherein the overlay is partially transparent and the surgical instrument in the at least one endoscopic image is visible through the overlay.

11. The system of any one of claims 1-10, wherein displaying the at least a portion of the three-dimensional model comprises displaying a simulated endoscopic image that comprises the at least a portion of the three-dimensional model.

12. The system of any one of claims 1-11, wherein the at least a portion of the three-dimensional model that is displayed comprises an indication of planned bone removal.

13. The system of any one of claims 1-12, wherein the one or more programs further include instructions that, when executed by the one or more processors, cause the system to: track a bone removal instrument using the tracking system and update the at least a portion of the three-dimensional model to reflect removal of bone by the bone removal instrument while the bone removal instrument is removing bone.

14. The system of claim 13, wherein the one or more programs further include instructions that, when executed by the one or more processors, cause the system to: display the updated three-dimensional model.

15. A method for guiding bone removal comprising:
receiving at least one two-dimensional image of bony anatomy captured by a two-dimensional imaging system and at least one endoscopic image of the bony anatomy captured by an endoscopic imager;
registering a three-dimensional model of the bony anatomy with the endoscopic imager in a common reference frame using the at least one two-dimensional image and a tracking system;
determining a position and orientation of the three-dimensional model relative to the at least one endoscopic image based on the registration of the three-dimensional model with the endoscopic imager in the common reference frame; and
displaying at least a portion of the three-dimensional model based on the determined position and orientation of the three-dimensional model relative to the at least one endoscopic image.
